# EUROPÄISCHE PATENTSCHRIFT

(11) **EP 0 865 240 B1**
(45) Veröffentlichungstag und Bekanntmachung des Hinweises auf die Patenterteilung: **04.10.2001**
(21) Anmeldenummer: 96941020.8
(22) Anmeldetag: 25.11.1996
(51) Int. Cl.: A01N 43/828, C07D 285/06, C07D 417/12

(54) **VERWENDUNG VON 1,2,3-THIADIAZOLCARBONSÄURE(THIO)ESTERN ZUR BEKÄMPFUNG VON SCHÄDLINGEN SOWIE 1,2,3-THIADIAZOLCARBONSÄURE(THIO)ESTER**
USE OF 1,2,3-THIADIAZOLE CARBOXYLIC ACID (THIO)ESTERS FOR THE CONTROL OF PESTS AND 1,2,3-THIADIAZOLE CARBOXYLIC ACID (THIO)ESTERS
UTILISATION DE (THIO)ESTERS D'ACIDE 1,2,3-THIADIAZOLCARBOXYLIQUE POUR LUTTER CONTRE LES PARASITES ET (THIO)ESTERS D'ACIDE 1,2,3-THIADIAZOLCARBOXYLIQUE

(30) Priorität: 07.12.1995 DE 19545638
(43) Veröffentlichungstag der Anmeldung: 23.09.1998
(73) Patentinhaber: BAYER AG, 51368 Leverkusen (DE)
(72) Erfinder: ALIG, Bernd, D-53639 Königswinter (DE); HÄNSSLER, Gerd, D-51381 Leverkusen (DE); STENZEL, Klaus, D-40595 Düsseldorf (DE); TURBERG, Andreas, D-40699 Erkrath (DE); LONDERSHAUSEN, Michael, D-40699 Erkrath (DE)
(86) Internationale Anmeldenummer: EP9605197
(87) Internationale Veröffentlichungsnummer: WO9720465

(56) Entgegenhaltungen:
- EP-A- 0 098 486
- EP-A- 0 338 525
- WO-A-95/29161
- WO-A-96/29871
- US-A- 3 776 907
- US-A- 4 341 551
- US-A- 4 829 072

## Beschreibung

Die vorliegende Erfindung betrifft neue 1,2,3 - Thiadiazolcarbonsäure (thio) ester und deren Verwendung zum Schutz von Pflanzen gegen Befall durch unerwünschte Mikroorganismen und tierische Schädlinge.

Es sind bereits bestimmte 1,2,3-Thiadiazolcarbonsäure-Derivate bekannt geworden (vgl. DE-A-2 728 523). Von diesen Stoffen wurden bisher allerdings nur herbizide und pflanzenwuchsregulierende Eigenschaften beschrieben.

Weiterhin sind aus der EP - A 0 098 486, der US - A 4 829 072 , der US - A 3 776 907 , der US - A 4 351 551 und der EP - A 0 338 525 zahlreiche fungizid, pharmakologisch bzw. herbizid wirksame 1,2,3 - Thiadiazolcarbonsäure (thio)-ester bekannt, in denen die Säuregruppe entweder direkt mit einem Heterocyclus oder mit einem durch Oxogruppen substituierten Carbocyclus verbunden ist. Entsprechende Stoffe, die an der Säuregruppe weder einen Heterocyclus noch einen durch Oxogruppen substituierten Carbocyclus enthalten, werden aber nicht offenbart.

Ferner ist aus der vorgängigen, aber nicht vorveröffentlichten Patentanmeldung gemäß WO 96 - 29 871 eine Gruppe von 1,2,3 - Thiadiazolcarbonsäure - Derivaten mit fungiziden Eigenschaften bekannt. Die im einzelnen aufgeführten Stoffe unterscheiden sich jedoch strukturell von den erfindungsgemäßen Verbindungen.

Schließlich betrifft die Parallelanmeldung gemäß WO 97 - 20 840 auch 1,2,3 - Thia - diazolcarbonsäureester, deren Estereinheit entweder direkt oder über ein Brückenglied mit einem Heterocyclus verknüpft ist. Analoge Stoffe, die an der betreffenden Stelle keinen Heterocyclus aufweisen, werden aber nicht beschrieben.

Es wurde nun gefunden, daß 1,2,3-Thiadiazolcarbonsäure(thio)ester der Formel 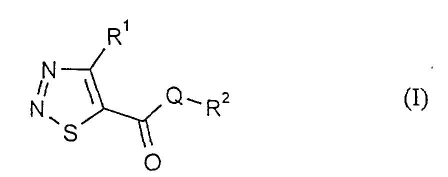 in welcher die Substituenten die nachstehend angegebenen Bedeutungen haben

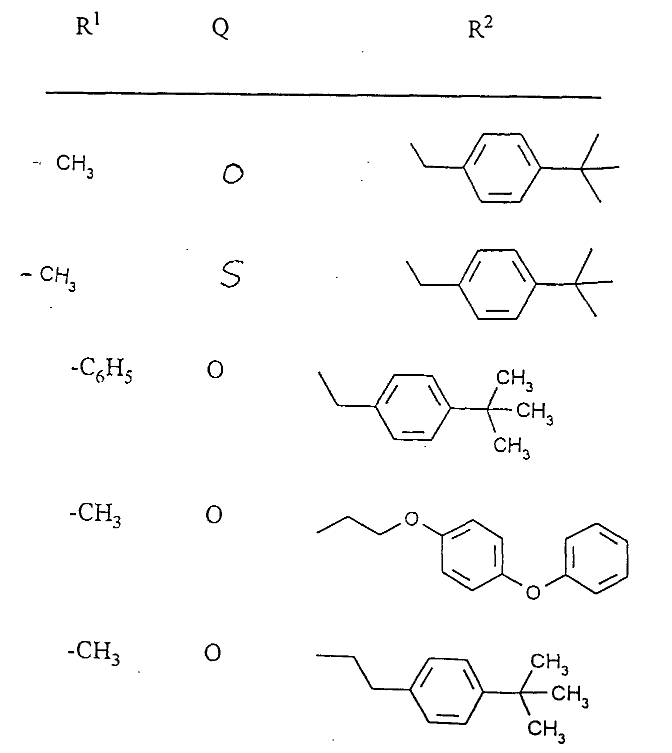

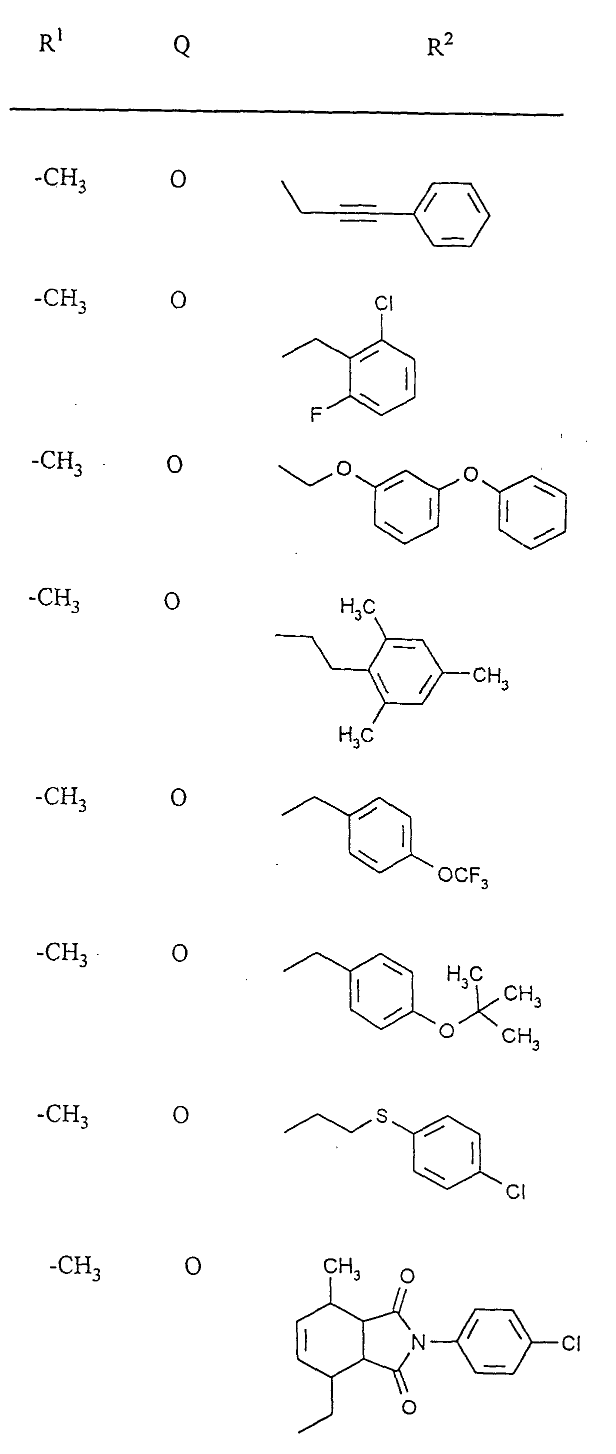

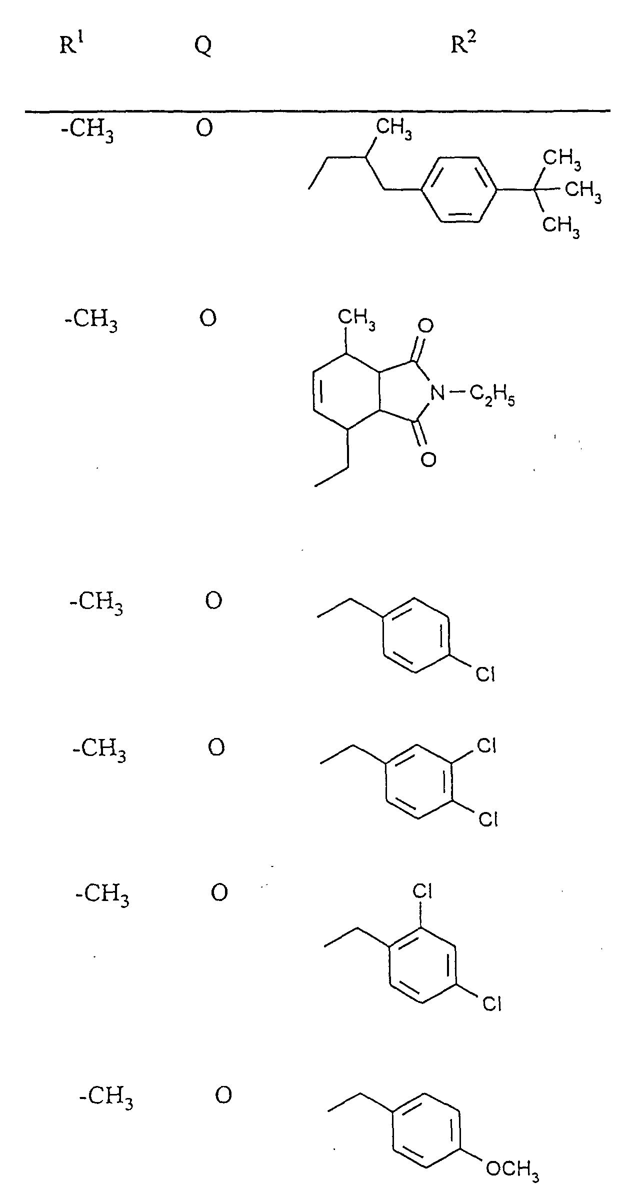

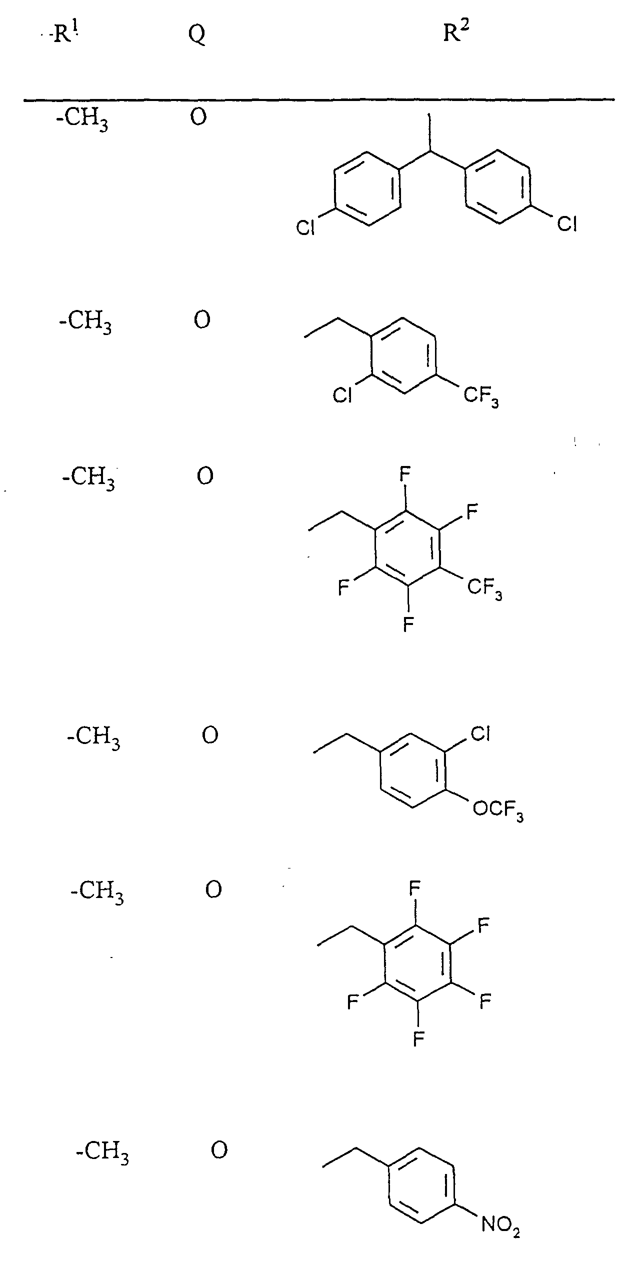

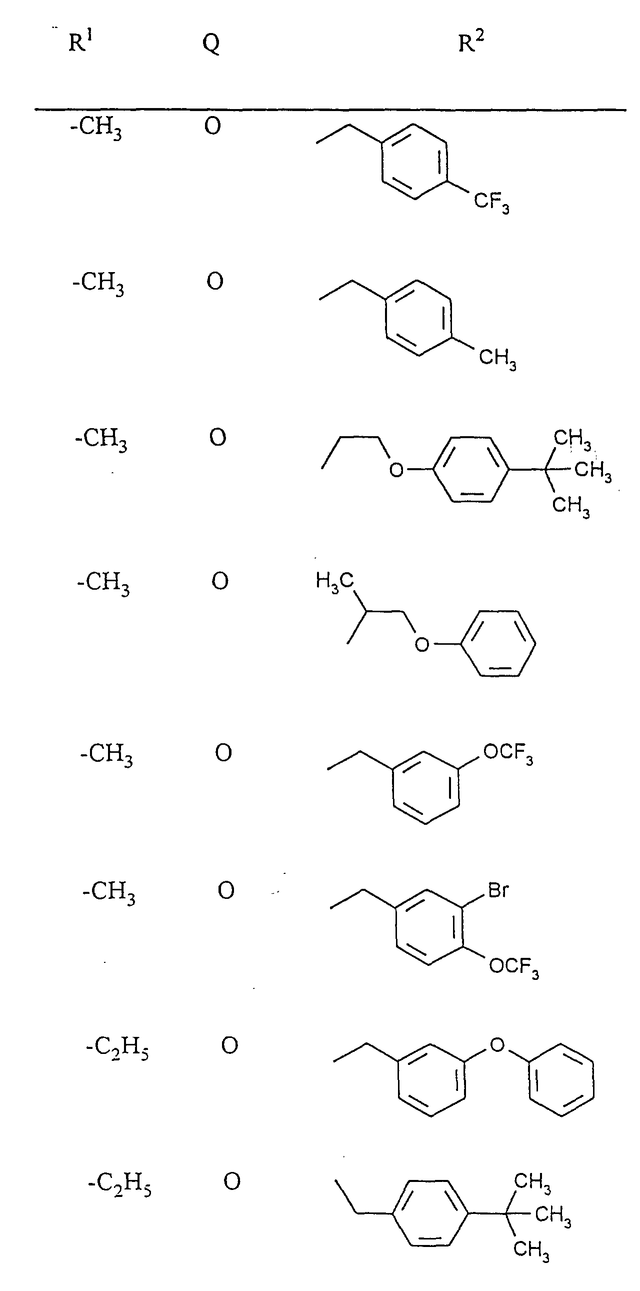

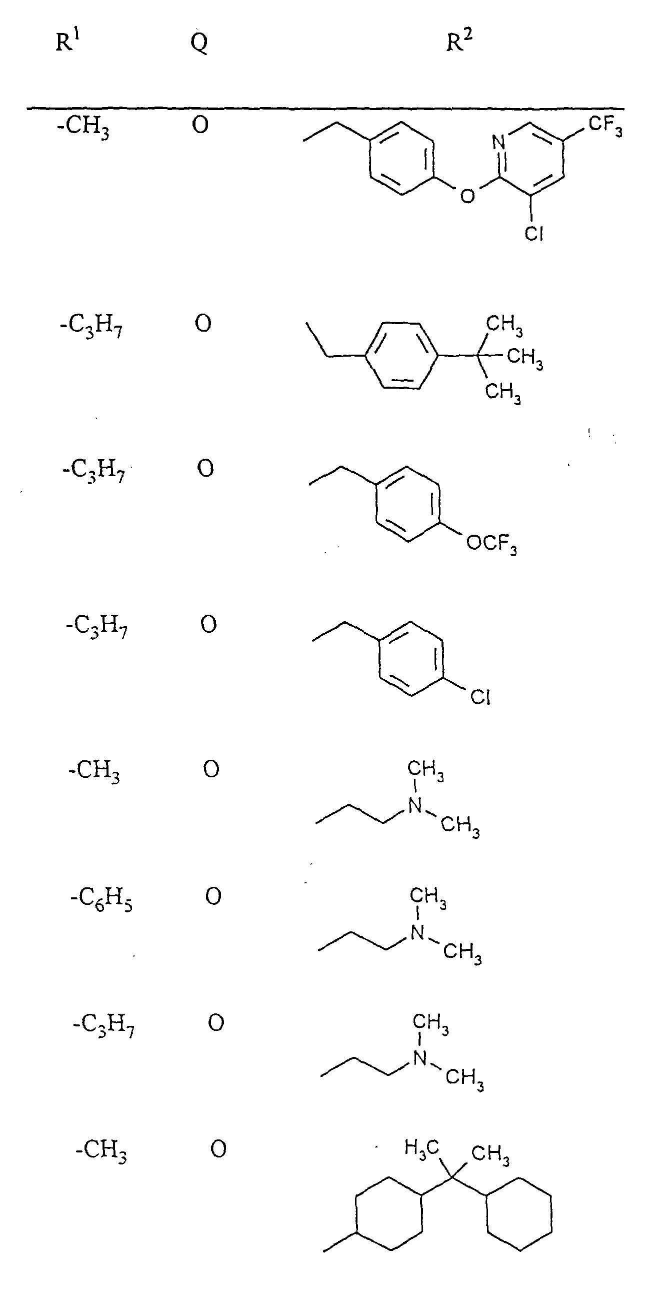

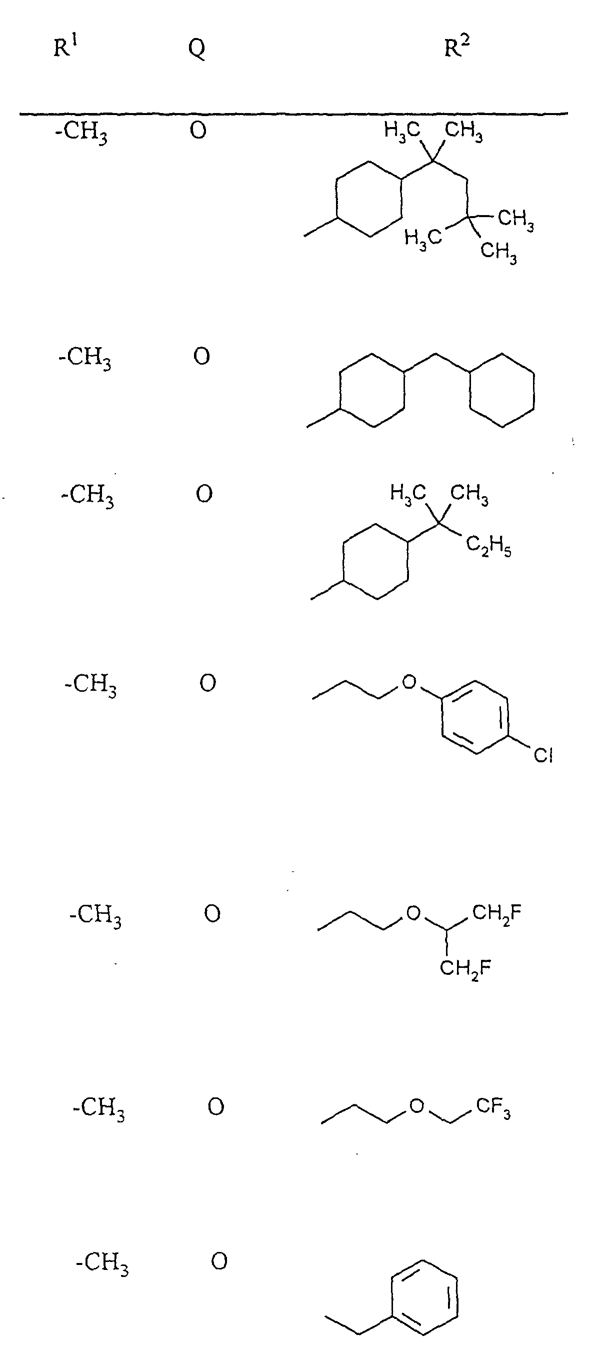

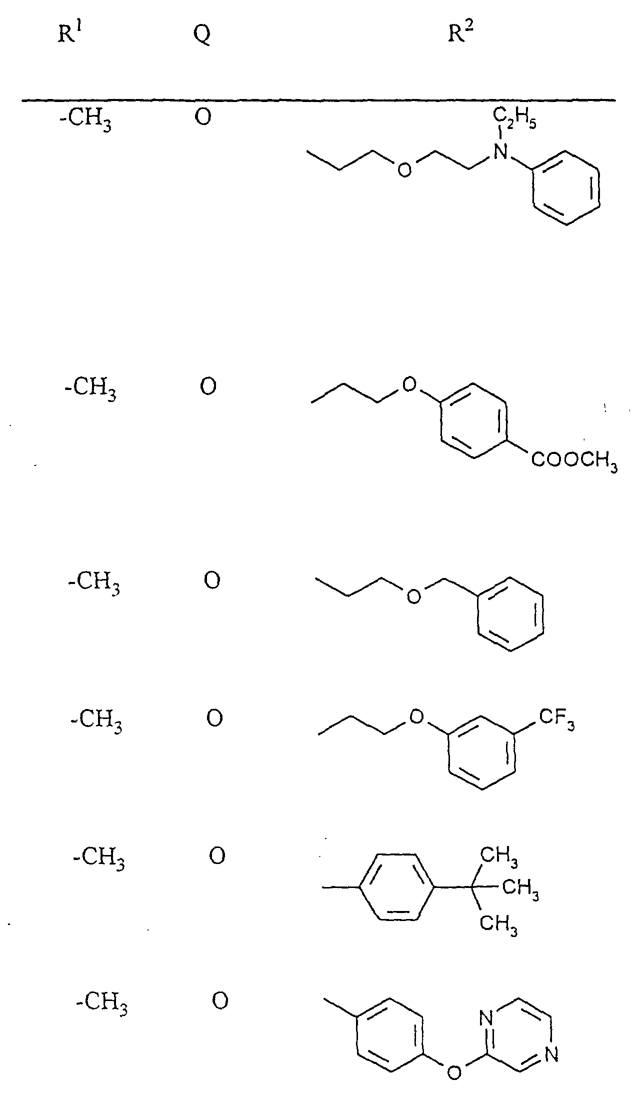

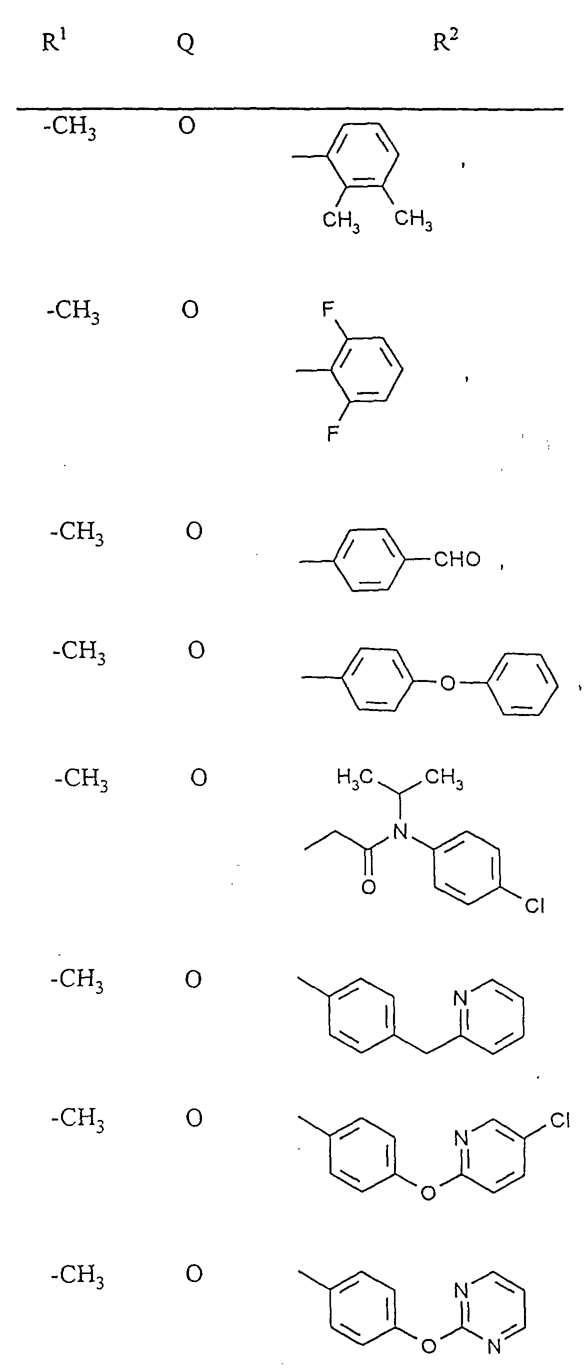

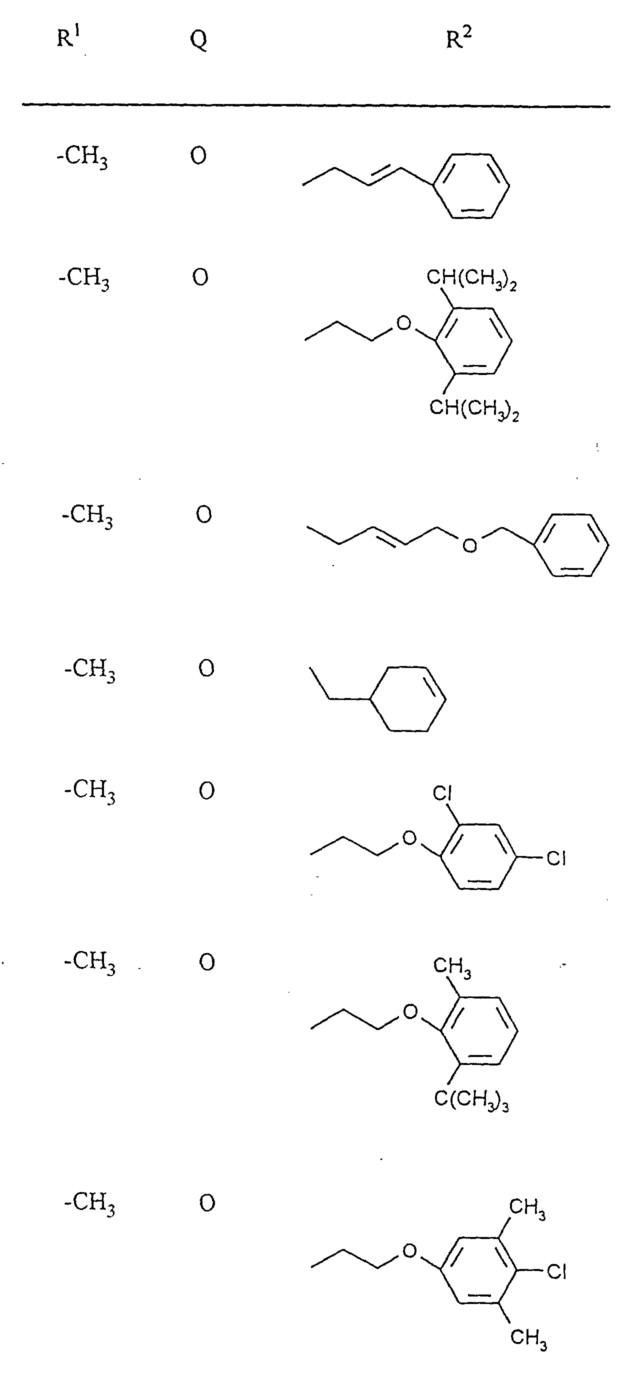

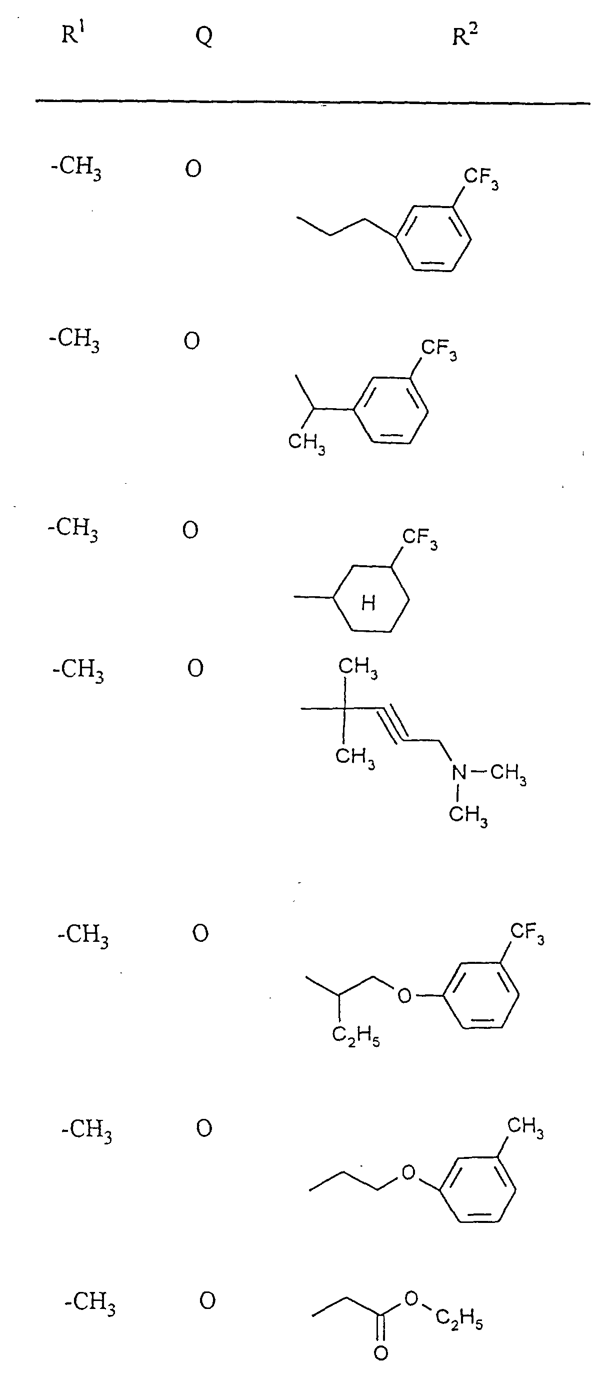

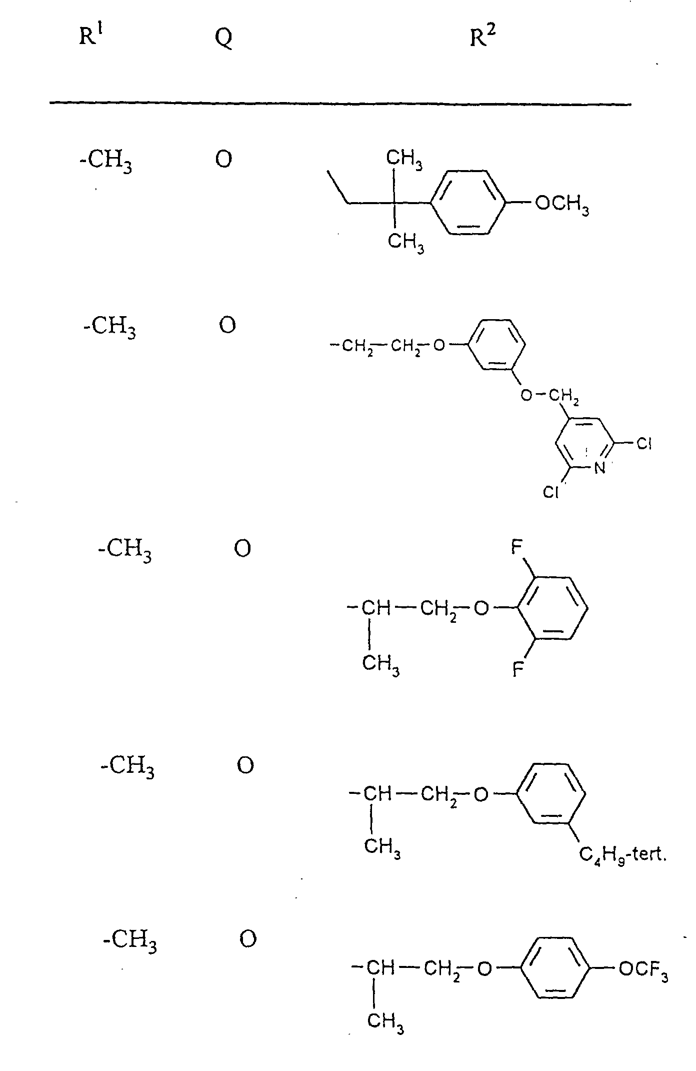

sehr gut zum Schutz von Pflanzen gegen Befall durch unerwünschte Mikrooganismen und tierische Schädlinge verwendbar sind.

Überraschenderweise eignen sich die erfindungsgemäß verwendbaren Stoffe wesentlich besser zum Schutz von Pflanzen gegen Befall durch unerwünschte Mikroorganismen und tierische Schädlinge als die konstitutionell ähnlichsten, vorbekannten Substanzen gleicher Wirkungsrichtung.

Die erfindungsgemäß verwendbaren 1,2,3-Thiadiazolcarbonsäure (thio) ester der oben angegebenen Formel (I) sind neu. Sie lassen sich herstellen, indem _ man 1,2,3 - Thiadiazolcarbonsäure - Derivate der Formel 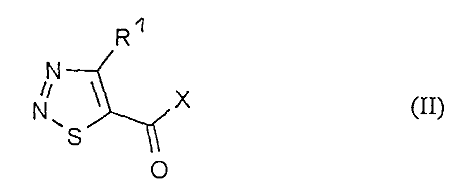 in welcher
- R¹: die oben angegebene Bedeutung hat und
- X: für Halogen steht,
mit (Thio)Alkoholen der Formel

H-Q-R²

in welcher
- Q und R²: die oben angegebenen Bedeutungen haben,
gegebenenfalls in Gegenwart eines Säurebindemittels und gegebenenfalls in Gegenwart eines Verdünnungsmittels umsetzt.

Die erfindungsgemäß verwendbaren 1,2,3-Thiadiazolcarbonsäure(thio)ester der Formel (I) können gegebenenfalls als Mischungen von verschiedenen möglichen isomeren Formen, insbesondere in Form von Stereoisomeren, anfallen. Die Erfindung betrifft sowohl die reinen Isomeren als auch beliebige Mischungen davon

Verwendet man 4-Methyl-1,2,3-thiadiazolcarbonsäurechlorid und 4-Trifluormethylbenzylalkohol als Ausgangsstoffe, so läßt sich der Verlauf des erfindungsgemäßen Verfahrens durch das folgende Formelschema veranschaulichen:

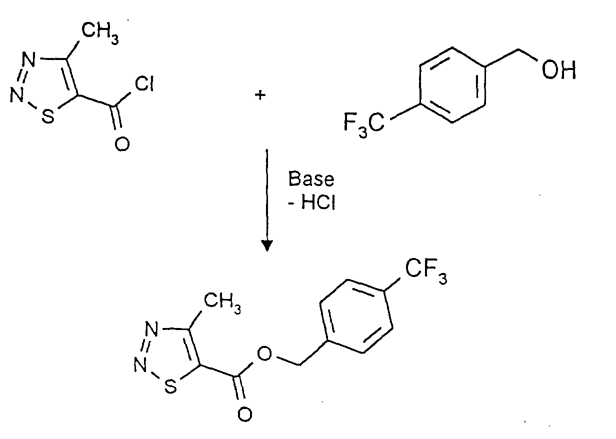

Die zur Durchführung des erfindungsgemäßen Verfahrens als Ausgangsstoffe benötigten 1,2,3-Thiadiazolcarbonsäurederivate sind durch die Formel (II) allgemein definiert. In dieser Formel (II) hat R¹ diejenigen Bedeutungen, die bereits im Zusammenhang mit der Beschreibung der erfindungsgemäßen Verbindungen der Formel (I.) für R¹ angegeben wurden. X steht vorzugsweise für Chlor.

Die 1,2,3-Thiadiazolcarbonsäure-Derivate der Formel (II) sind bekannt oder können nach an sich bekannten Verfahren hergestellt werden (vgl. DE-A-2 728 523 oder J. Heterocyclic Chem. 1976, 301).

Die zur Durchführung des erfindungsgemäßen Verfahrens weiterhin als Ausgangsstoffe benötigten (Thio)Alkohole sind durch die Formel (III) allgemein definiert. In dieser Formel (III) haben Q und R² diejenigen Bedeutungen, die bereits im Zusammenhang mit der Beschreibung der erfindungsgemäßen Verbindungen der Formel (I) für Q und R² angegeben wurden.

Die (Thio)Alkohole der Formel (III) sind bekannte Reagenzien der Organischen Chemie.

Als Säurebindemittel kommen bei der Durchführung des erfindungsgemäßen Verfahrens alle üblichen anorganischen und organischen Basen in Frage. Vorzugsweise verwendbar sind Erdalkalimetall- oder Alkalimetall-hydride, -hydroxide, -amide, -alkoholate, -acetate, -carbonate oder -hydrogencarbonate, wie beispielsweise Natriumhydrid, Natriumamid, Natrium-methylat, Natrium-ethylat, Kalium- tert.-butylat, Natriumhydroxid, Kaliumhydroxid, Natriumacetat, Calciumacetat, Natriumcarbonat, Kaliumcarbonat, Kaliumhydrogencarbonat, Natriumhydrogencarbonat, ferner basische Ammonium-Verbindungen, wie Ammoniumhydroxid, Ammoniumacetat oder Ammoniumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Als Verdünnungsmittel kommen bei der Durchführung des erfindungsgemäßen Verfahrens alle üblichen inerten, organischen Solventien in Betracht. Vorzugsweise verwendbar sind aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid; Sulfone, wie Sulfolan.

Die Reaktionstemperaturen können bei der Durchführung des erfindungsgemäßen Verfahrens in einem größeren Bereich variiert werden. Im allgemeinen arbeitet man bei Temperaturen zwischen -20°C und +180°C, vorzugsweise bei Temperaturen zwischen 20°C und 130°C.

Das erfindungsgemäße Verfahren wird üblicherweise unter Normaldruck durchgeführt. Es ist jedoch auch möglich, unter erhöhtem oder vermindertem Druck zu arbeiten.

Bei der Durchführung des erfindungsgemäßen Verfahrens setzt man auf 1 Mol an 1,2,3-Thiadiazolcarbonsäure-Derivat der Formel (II) im allgemeinen 0,5 bis 10 Mol, vorzugsweise 0,8 bis 3 Mol an (Thio)Alkohol der Formel (III) ein. Die Durchführung der Reaktion und die Aufarbeitung erfolgen nach üblichen Methoden (vgl. Herstellungsbeispiele).

Die erfindungsgemäß verwendbaren Wirkstoffe weisen eine starke resistenz-induzierende Wirkung in Pflanzen auf. Sie eignen sich daher zur Erzeugung von Resistenz in Pflanzen gegen Befall durch unerwünschte Mikroorganismen.

Unter resistenzinduzierenden Stoffen sind im vorliegenden Zusammenhang solche Substanzen zu verstehen, die in der Lage sind, das Abwehrsystem von Pflanzen so zu stimulieren, daß die behandelten Pflanzen bei nachfolgender Inokulation mit unerwünschten Mikroorganismen weitgehende Resistenz gegen diese Mikroorganismen entfalten.

Unter unerwünschten Mikroorganismen sind im vorliegenden Fall phytopathogene Pilze, Bakterien und Viren zu verstehen. Die erfindungsgemäßen Stoffe können also eingesetzt werden, um in Pflanzen innerhalb eines gewissen Zeitraumes nach der Behandlung Resistenz gegen den Befall durch die genannten Schaderreger zu erzeugen. Der Zeitraum, innerhalb dessen Resistenz herbeigeführt wird, erstreckt sich im allgemeinen von 1 bis 10 Tage, vorzugsweise 1 bis 7 Tage nach der Behandlung der Pflanzen mit den Wirkstoffen.

Die erfindungsgemäß verwendbaren Wirkstoffe weisen neben der resistenzinduzierenden Wirkung auch eine starke mikrobizide Wirkung auf und werden auch zur direkten Bekämpfung von unerwünschten Mikroorganismen praktisch eingesetzt. Die Wirkstoffe sind für den Gebrauch als Pflanzenschutzmittel, insbesondere als Fungizide geeignet.

Zu den unerwünschten Mikroorganismen im Pflanzenschutz gehören Pilze aus den Klassen Plasmodiophoromycetes, Oomycetes, Chytridiomycetes, Zygomycetes, Ascomycetes, Basidiomycetes, Deuteromycetes.

Bakterizide Mittel werden im Pflanzenschutz zur Bekämpfung von Pseudomonadaceae, Rhizobiaceae, Enterobacteriaceae, Corynebacteriaceae und Streptomycetaceae eingesetzt.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei werden die erfindungsgemäß verwendbaren Wirkstoffe mit besonders gutem Erfolg zur Bekämpfung von Reiskrankheiten, wie Pyricularia-Arten, oder von Krankheiten im Wein-, Obst- und Gemüseanbau, wie beispielsweise Uncinula-Arten, sowie mit gutem Erfolg auch gegen Getreidekrankheiten, wie echten Mehltau, eingesetzt.

Die Wirkstoffe können in Abhängigkeit von ihren jeweiligen physikalischen und/oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäß verwendbaren Wirkstoffe können als solche oder in ihren Formulierungen auch in Mischung mit bekannten Fungiziden, Bakteriziden, Akariziden, Nematiziden oder Insektiziden eingesetzt werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen in Betracht:
**Fungizide:**
   2-Aminobutan; 2-Anilino-4-methyl-6-cyclopropyl-pyrimidin; 2',6'-Dibromo-2-methyl-4'-trifluoromethoxy-4'-trifluoro-methyl-1,3-thiazol-5-carboxanilid; 2,6-Dichloro-N-(4-trifluoromethylbenzyl)-benzamid; (E)-2-Methoxyimino-N-methyl-2-(2-phenoxyphenyl)-acetamid; 8-Hydroxychinolinsulfat; Methyl-(E)-2-{2-[6-(2-cyanophenoxy)-pyrimidin-4-yloxy]-phenyl)-3-methoxyacrylat; Methyl-(E)-methoximino [alpha-(o-tolyloxy)-o-tolyl] acetat; 2-Phenylphenol (OPP), Aldimorph, Ampropylfos, Anilazin, Azaconazol,
   Benalaxyl, Benodanil, Benomyl, Binapacryl, Biphenyl, Bitertanol, Blasticidin-S, Bromuconazole, Bupirimate, Buthiobate,
   Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
   Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodine, Drazoxolon,
   Edifenphos, Epoxyconazole, Ethirimol, Etridiazol,
   Fenarimol, Fenbuconazole, Fenfuram, Fenitropan, Fenpiclonil, Fenpropidin, Fenpropimorph, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
   Guazatine,
   Hexachlorobenzol, Hexaconazol, Hymexazol,
   Imazalil, Imibenconazol, Iminoctadin, Iprobenfos (IBP), Iprodion, Isoprothiolan,
   Kasugamycin, Kupfer-Zubereitungen, wie: Kupferhydroxid, Kupfernaphthenat, Küpferoxychlorid, Kupfersulfat, Kupferoxid, Oxin-Kupfer und Bordeaux-Mischung,
   Mancopper, Mancozeb, Maneb, Mepanipyrim, Mepronil, Metalaxyl, Metconazol, Methasulfocarb, Methfuroxam, Metiram, Metsulfovax, Myclobutanil,
   Nickel-dimethyldithiocarbamat, Nitrothal-isopropyl, Nuarimol,
   Ofurace, Oxadixyl, Oxamocarb, Oxycarboxin,
   Pefurazoat, Penconazol, Pencycuron, Phosdiphen, Pimaricin, Piperalin, Polyoxin, Probenazol, Prochloraz, Procymidon, Propamocarb, Propiconazole, Propineb, Pyrazophos, Pyrifenox, Pyrimethanil, Pyroquilon,
   Quintozen (PCNB),
   Schwefel und Schwefel-Zubereitungen,
   Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
   Validamycin A, Vinclozolin,
   Zineb, Ziram.
**Bakterizide:**
   Bronopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.
**Insektizide / Akarizide / Nematizide:**
   Abamectin, Acephat, Acrinathrin, Alanycarb, Aldicarb, Alphamethrin, Amitraz, Avermectin, AZ 60541, Azadirachtin, Azinphos A, Azinphos M, Azocyclotin,
   Bacillus thuringiensis, 4-Bromo-2-(4-chlorphenyl)-1-(ethoxymethyl)-5-(trifluoromethyl)-1H-pyrrole-3-carbonitrile, Bendiocarb, Benfuracarb, Bensultap, Betacyfluthrin, Bifenthrin, BPMC, Brofenprox, Bromophos A, Bufencarb, Buprofezin, Butocarboxim, Butylpyridaben,
   Cadusafos, Carbaryl, Carbofuran, Carbophenothion, Carbosulfan, Cartap, Chloethocarb, Chlorethoxyfos, Chlorfenvinphos, Chlorfluazuron, Chlormephos, N-[(6-Chloro-3-pyridinyl)-methyl]-N'-cyano-N-methyl-ethanimidamide, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
   Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat,
   Dimethylvinphos, Dioxathion, Disulfoton,
   Edifenphos, Emamectin, Esfenvalerat, Ethiofencarb, Ethion, Ethofenprox, Ethoprophos, Etrimphos,
   Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Fluazuron ,Flucycloxuron, Flucythrinat, Flufenoxuron, Flufenprox, Fluvalinate, Fonophos, Formothion, Fosthiazat, Fubfenprox, Furathiocarb,
   HCH, Heptenophos, Hexaflumuron, Hexythiazox,
   Imidacloprid, Iprobenfos, Isazophos, Isofenphos, Isoprocarb, Isoxathion, Ivermectin, Lambda-cyhalothrin, Lufenuron,
   Malathion, Mecarbam, Mevinphos, Mesulfenphos, Metaldehyd, Methacrifos, Methamidophos, Methidathion, Methiocarb, Methomyl, Metolcarb, Milbemectin, Monocrotophos, Moxidectin,
   Naled, NC 184, Nitenpyram,
   Omethoat, Oxamyl, Oxydemethon M, Oxydeprofos,
   Parathion A, Parathion M, Permethrin, Phenthoat, Phorat, Phosalon, Phosmet, Phosphamidon, Phoxim, Pirimicarb, Pirimiphos M, Pirimiphos A, Profenophos, Promecarb, Propaphos, Propoxur, Prothiofos, Prothiophos, Prothoat, Pymetrozin, Pyraclophos, Pyridaphenthion, Pyresmethrin, Pyrethrum, Pyridaben, Pyrimidifen, Pyriproxifen,
   Quinalphos,
   Salithion, Sebufos, Silafluofen, Sulfotep, Sulprofos,
   Tebufenozide, Tebufenpyrad, Tebupirimiphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
   Vamidothion, XMC, Xylylcarb, Zetamethrin.

Auch eine Mischung mit anderen bekannten Wirkstoffen, wie Herbiziden oder mit Düngemitteln und Wachstumsregulatoren ist möglich.

Die erfindungsgemäß verwendbaren Wirkstoffe können als solche, in Form ihren handelsüblichen Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verschäumen, Bestreichen usw. Gegebenenfalls werden die Wirkstoffe nach dem Ultra-Low-Volume-Verfahren ausgebracht oder die Wirkstoffzubereitung oder der Wirkstoff selbst wird in den Boden injiziert. Gegebenenfalls wird auch das Saatgut der Pflanzen behandelt.

Bei der Behandlung von Pflanzenteilen können die Wirkstoffkonzentrationen in den Anwendungsformen in einem größeren Bereich variiert werden: Sie liegen im allgemeinen zwischen 1 und 0,0001 Gew.-%, vorzugsweise zwischen 0,5 und 0,001 Gew.-%.

Bei der Saatgutbehandlung werden im allgemeinen Wirkstoffmengen von 0,001 bis 50 g je Kilogramm Saatgut, vorzugsweise 0,01 bis 10 g benötigt.

Bei der Behandlung des Bodens sind Wirkstoffkonzentrationen von 0,00001 bis 0,1 Gew.-%, vorzugsweise von 0,0001 bis 0,02 Gew.-% am Wirkungsort erforderlich,

Die erfindungsgemäßen Wirkstoffe wirken außerdem gegen Pflanzen-, Hygiene- und Vorratsschädlinge und auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:

Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..

Aus der Ordnung der Siphonapterida z.B. Pulex spp., Ctenocephalides spp., Xenopsylla spp., Ceratophyllus spp..

Aus der Ordnung der Heteropterida z.B. Cimex spp., Triatoma spp., Rhodnius spp., Panstrongylus spp..
Aus der Ordnung der Blattarida z.B. Blatta orientalis, Periplaneta americana, Blattela germanica, Supella spp..
Aus der Unterklasse der Acaria (Acarida) und den Ordnungen der Meta- sowie Mesostigmata z.B. Argas spp., Ornithodorus spp., Otabius spp., Ixodes spp., Amblyomma spp., Boophilus spp., Dermacentor spp., Haemaphysalis spp., Hyalomma spp., Rhipicephalus spp., Dermanyssus spp., Raillietia spp., Pneumonyssus spp., Sternostoma spp., Varroa spp..

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Beispielsweise zeigen sie eine hervorragende Wirksamkeit gegen Musca domestica und Lucilia cuprina-Larven.

Die erfindungsgemäß verwendbaren Wirkstoffe eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäß verwendbaren Wirkstoffe eine wirtschaftlichere und einfachere Tierhaltung möglich ist.

Der Einsatz der erfindungsgemäß verwendbaren Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Die Herstellung und die Verwendung der erfindungsgemäß verwendbaren Wirkstoffe gehen aus den nachfolgenden Beispielen hervor.

### Herstellungsbeispiele

### Beispiel 1:

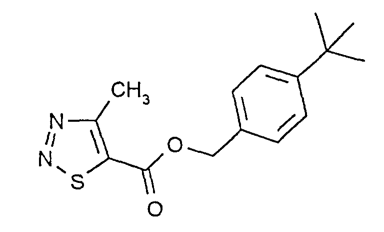

Eine Lösung von 6,5 g (39,6 mMol) 4-t-Butylbenzylalkohol und 15 ml Triethylamin in 100 ml Toluol wird mit 5,5 g (33,8 mMol) 4-Methyl-1,2,3-thiadiazol-5-carbonsäurechlorid versetzt und 18 Stunden bei 100°C gerührt. Nach dem Abkühlen wird die Reaktionsmischung mit Wasser versetzt. Die organische Phase wird abgetrennt, über Magnesiumsulfat getrocknet und eingedampft. Das zurückbleibende Öl wird einer Kugelrohr-Destillation unterworfen. Man erhält 6,7 g (68% der Theorie) 4-Methyl-1,2,3-thiadiazol-5-carbonsäure-4-t-butylbenzylester als Öl vom Siedepunkt 200°C bei 0,1 mbar.

### Beispiel 2:

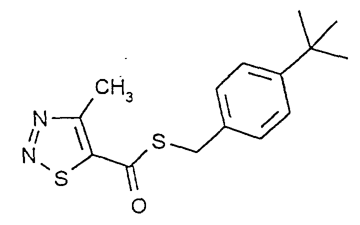

Eine Lösung von 4 g (22,2 mMol) 4-t-Butylbenzylthiol und 10 ml Triethylamin in 60 ml Toluol wird mit 3,5 g (21,5 mMol) 4-Methyl-1,2,3-thiadiazol-5-carbonsäurechlorid versetzt und 18 Stunden bei 100°C gerührt. Nach dem Abkühlen wird die Reaktionsmischung mit Wasser versetzt. Die organische Phase wird abgetrennt, über Magnesiumsulfat getrocknet und eingedampft. Das zurückbleibende Öl wird einer Kugelrohr-Destillation unterworfen. Man erhält 4,0 g (61% der Theorie) 4-Methyl-1,2,3-thiadiazol-5-carbonsäure-4-t-butylbenzylthiolester als gelbes Öl vom Siedepunkt 210°C bei 0,1 mbar.

Analog den Beispielen 1 und 2 sowie entsprechend den Angaben in der Beschreibung werden die in der nachstehenden Tabelle 1 genannten Verbindungen erhalten.

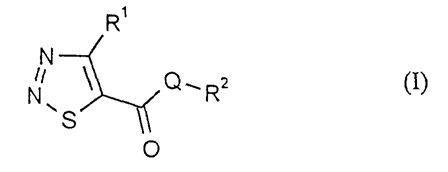

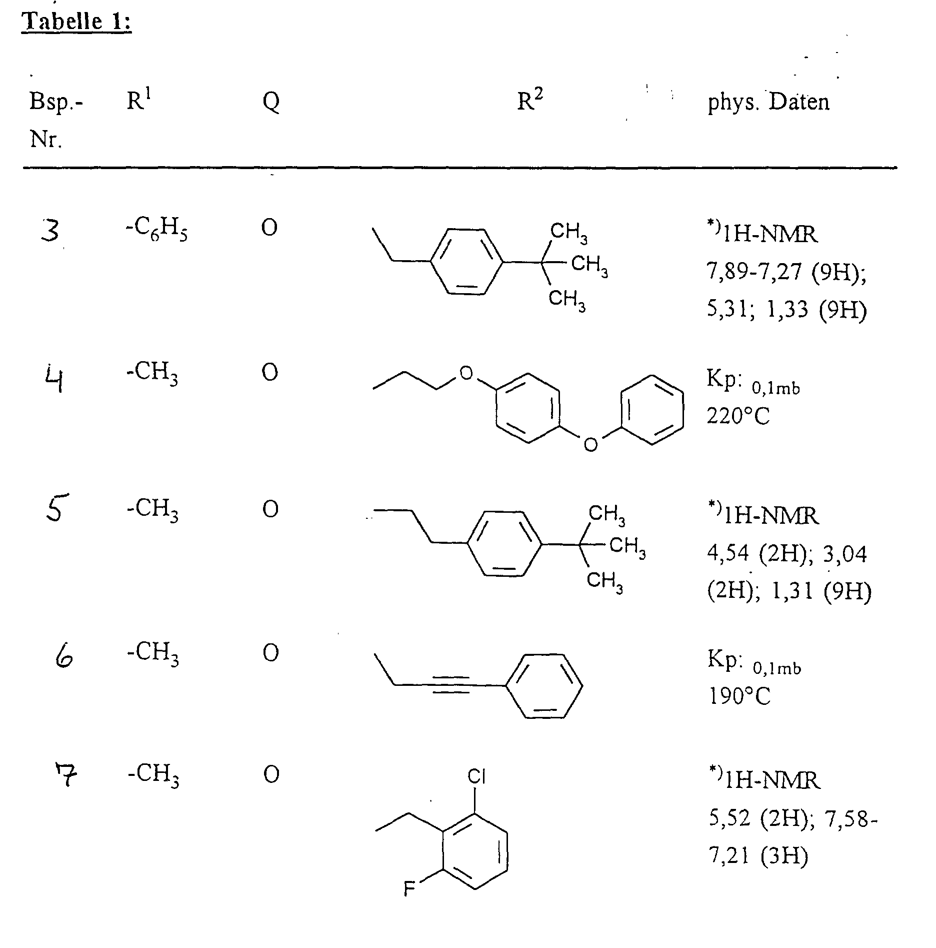

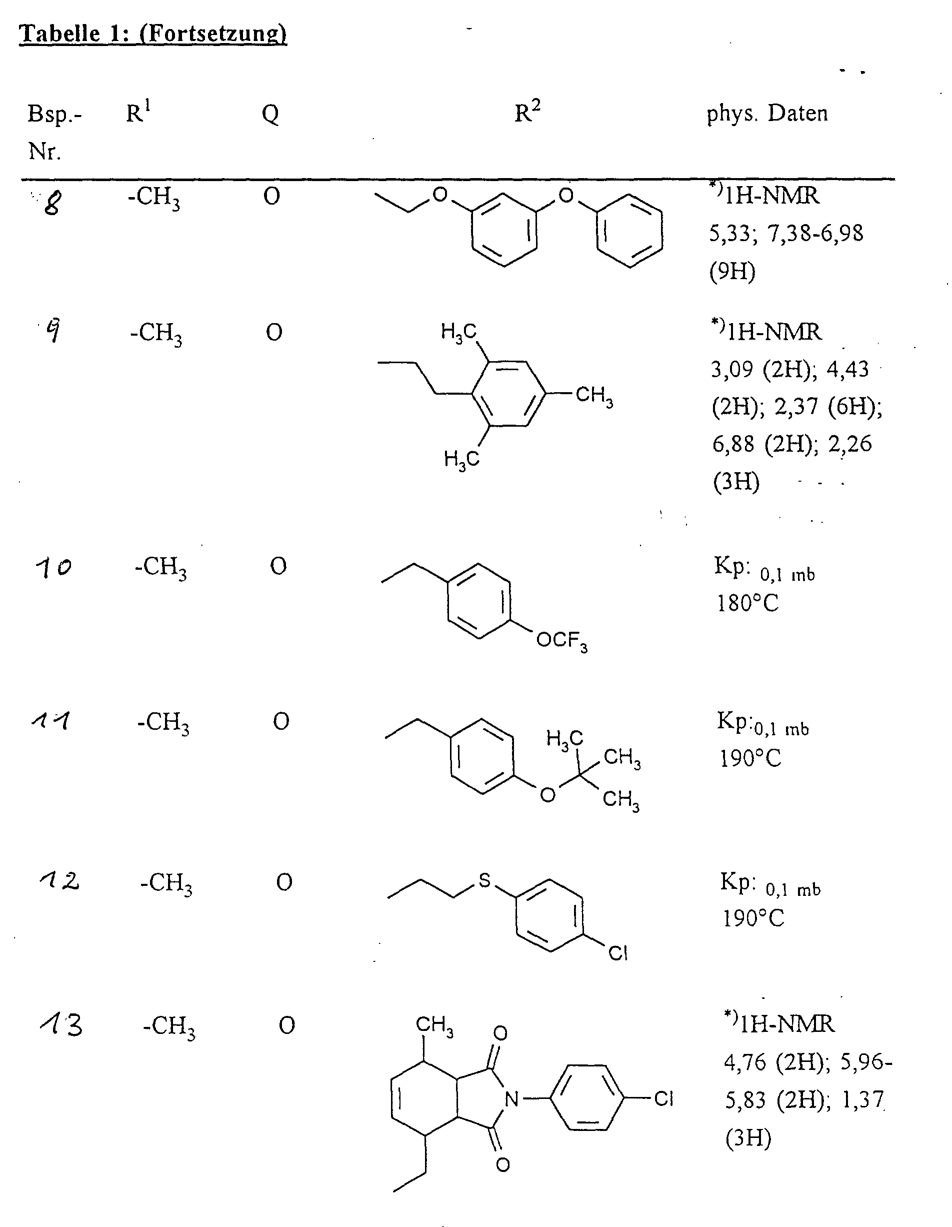

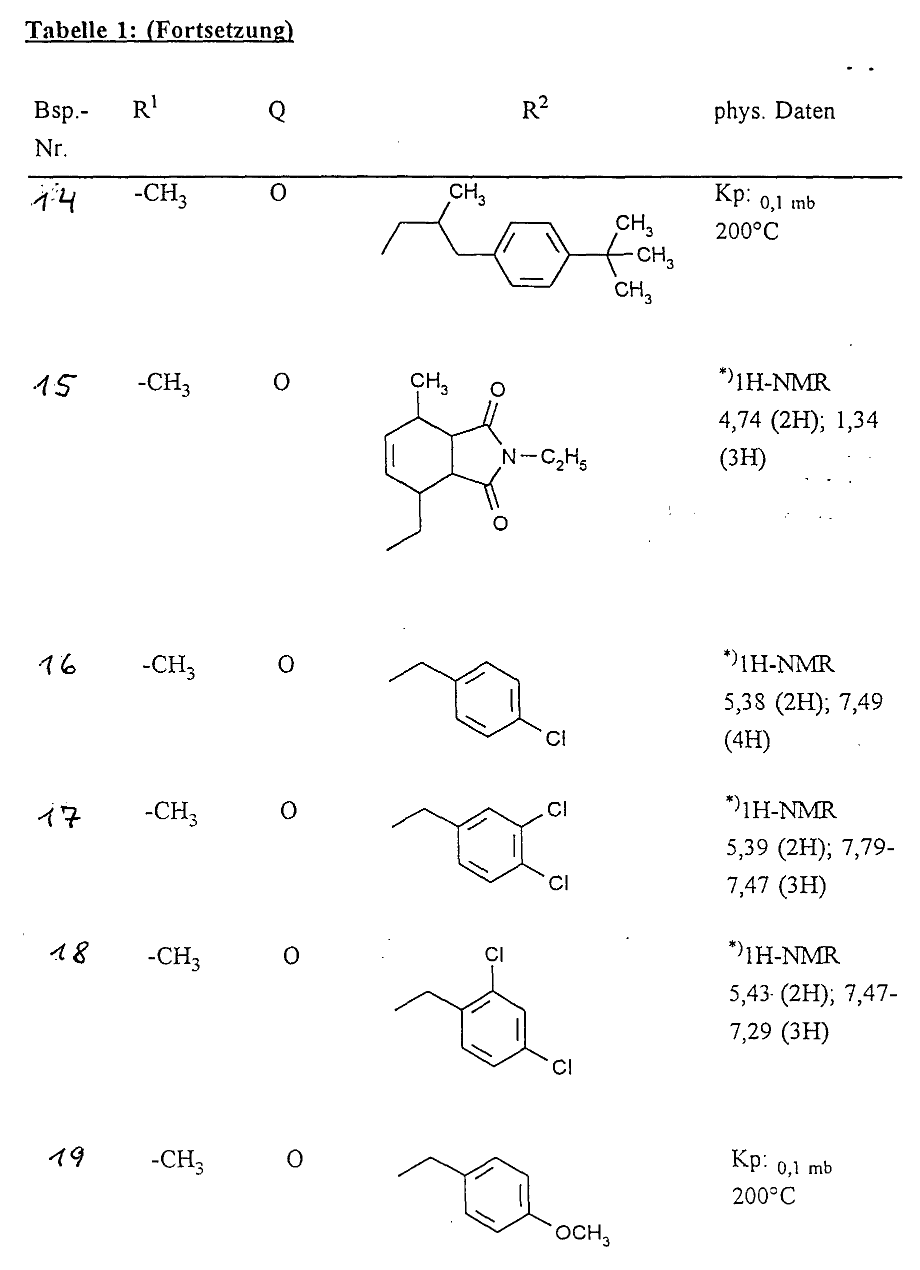

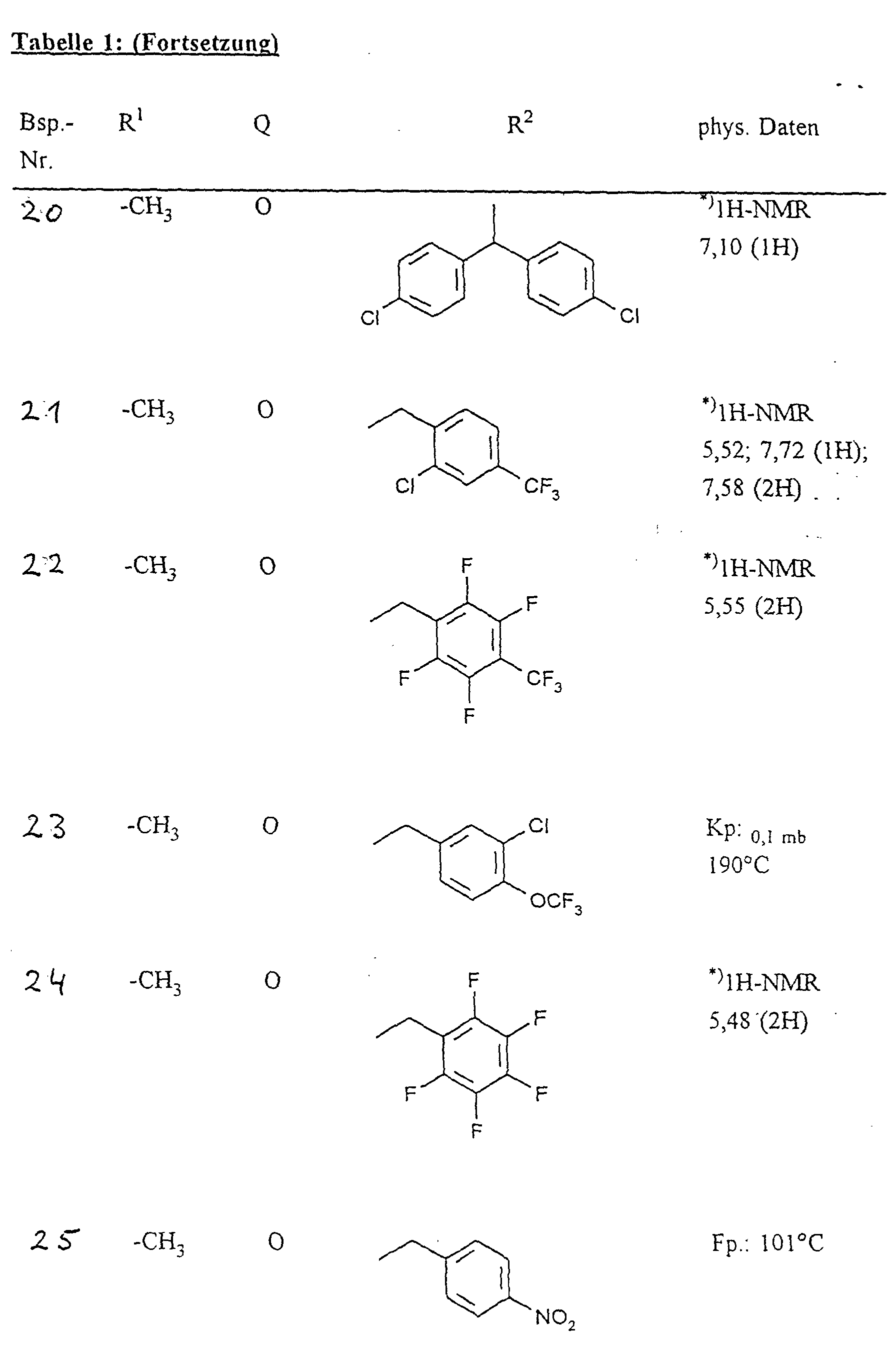

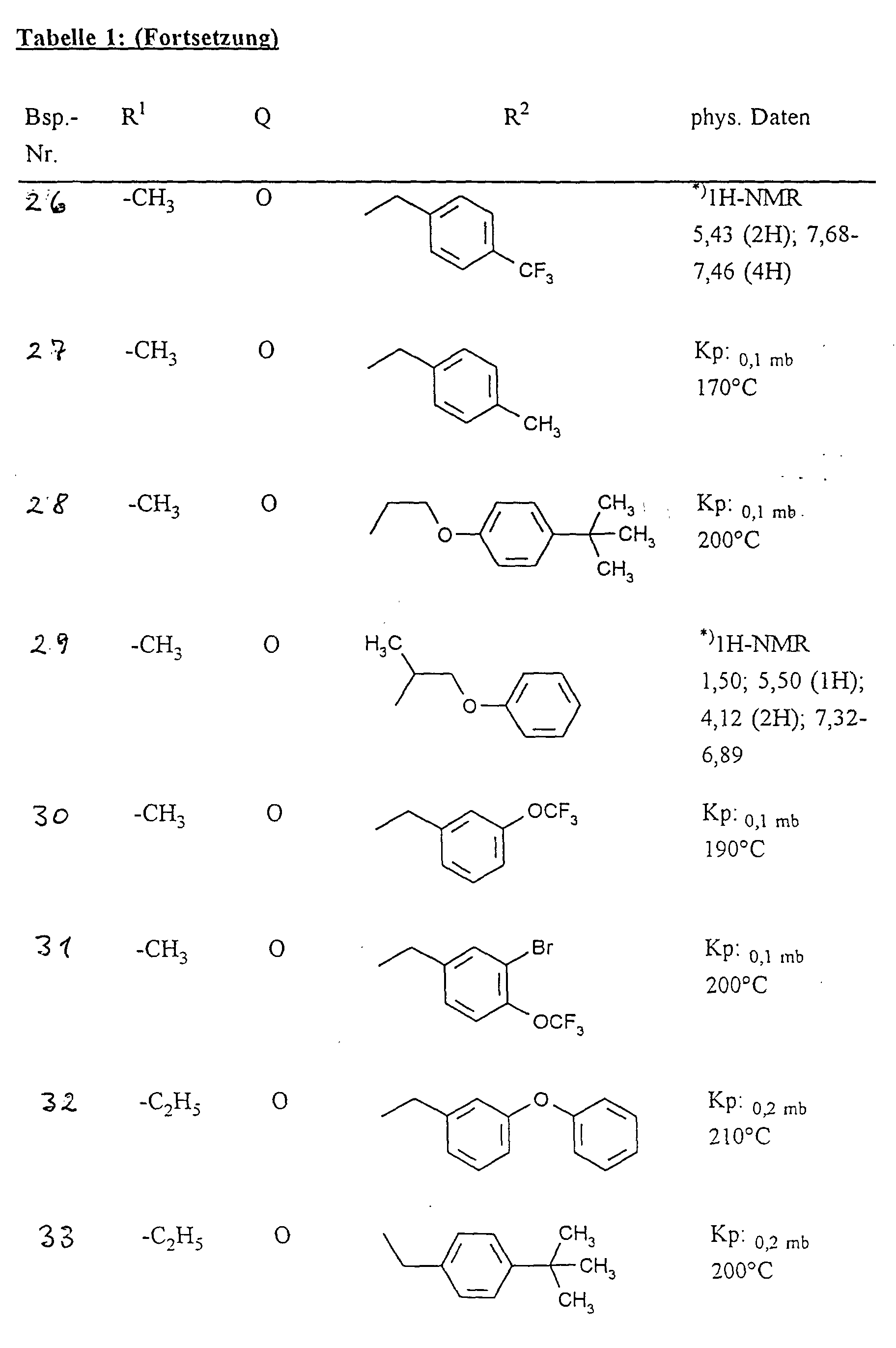

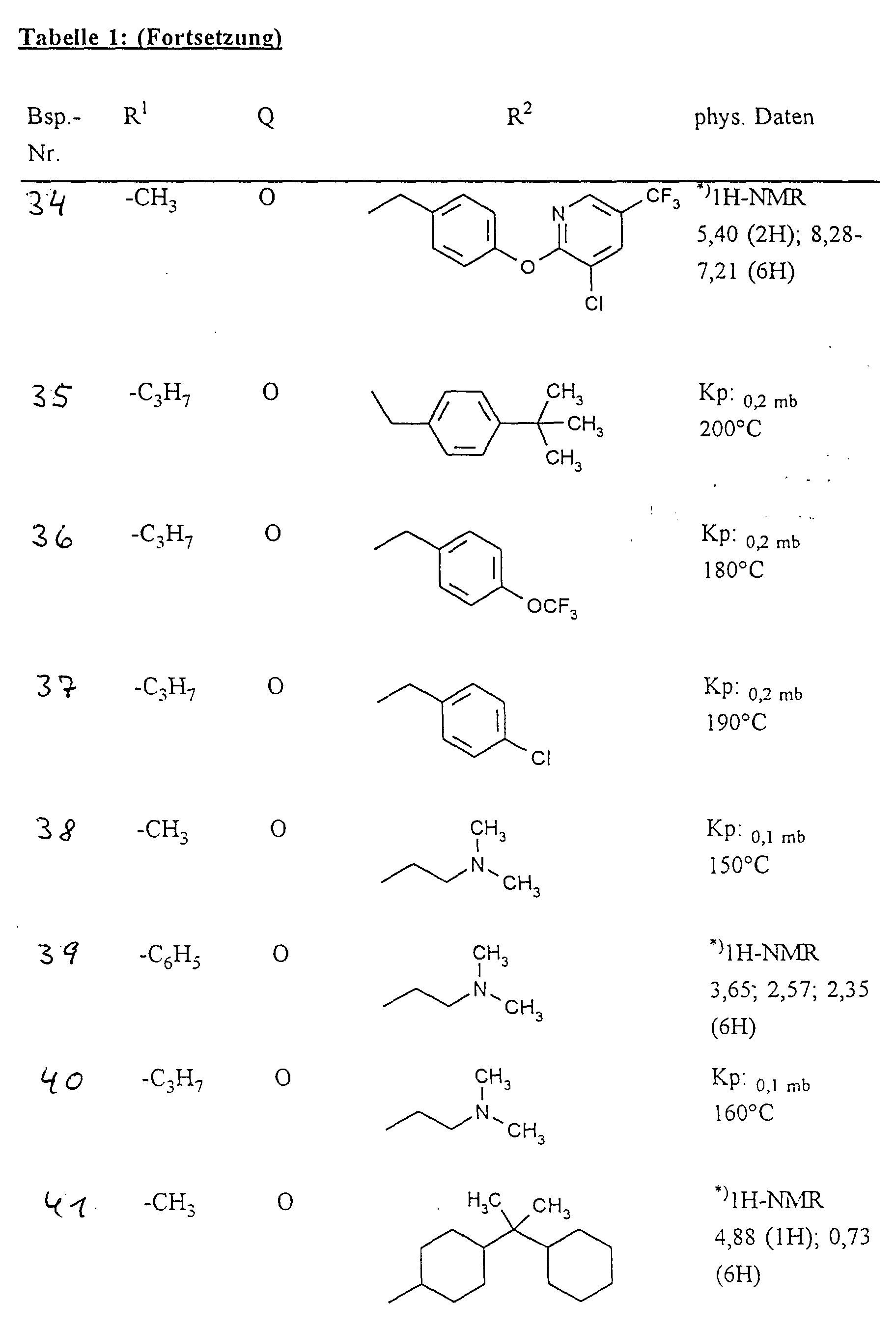

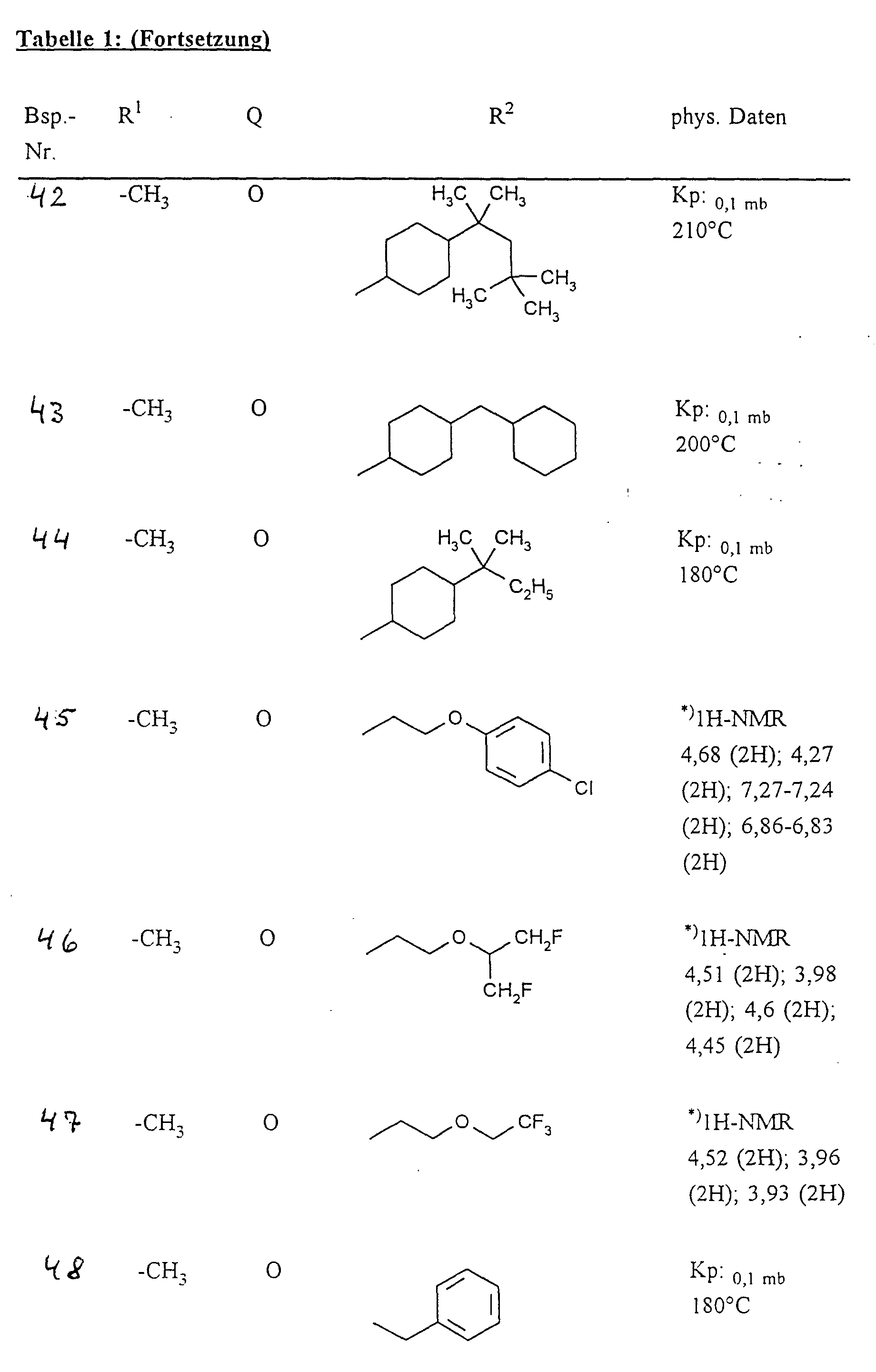

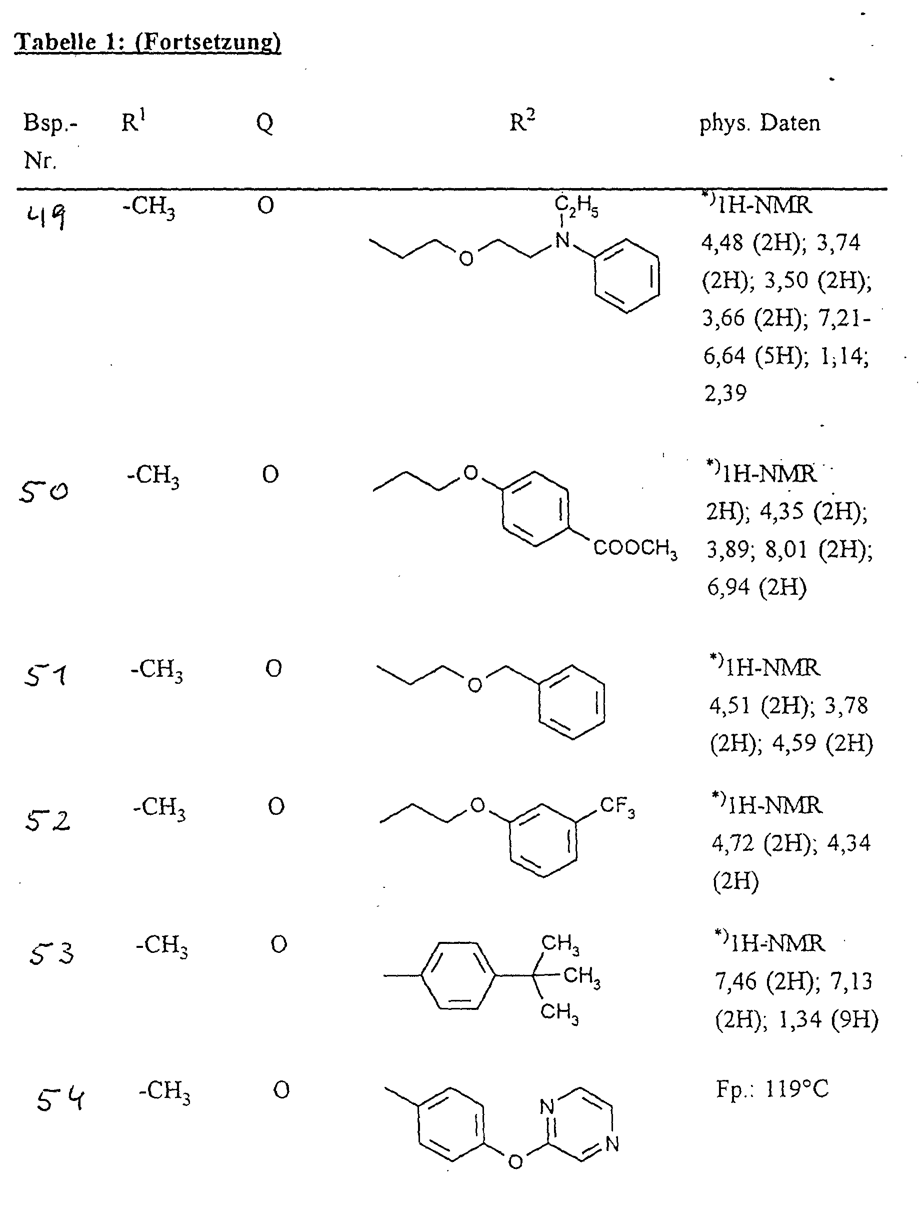

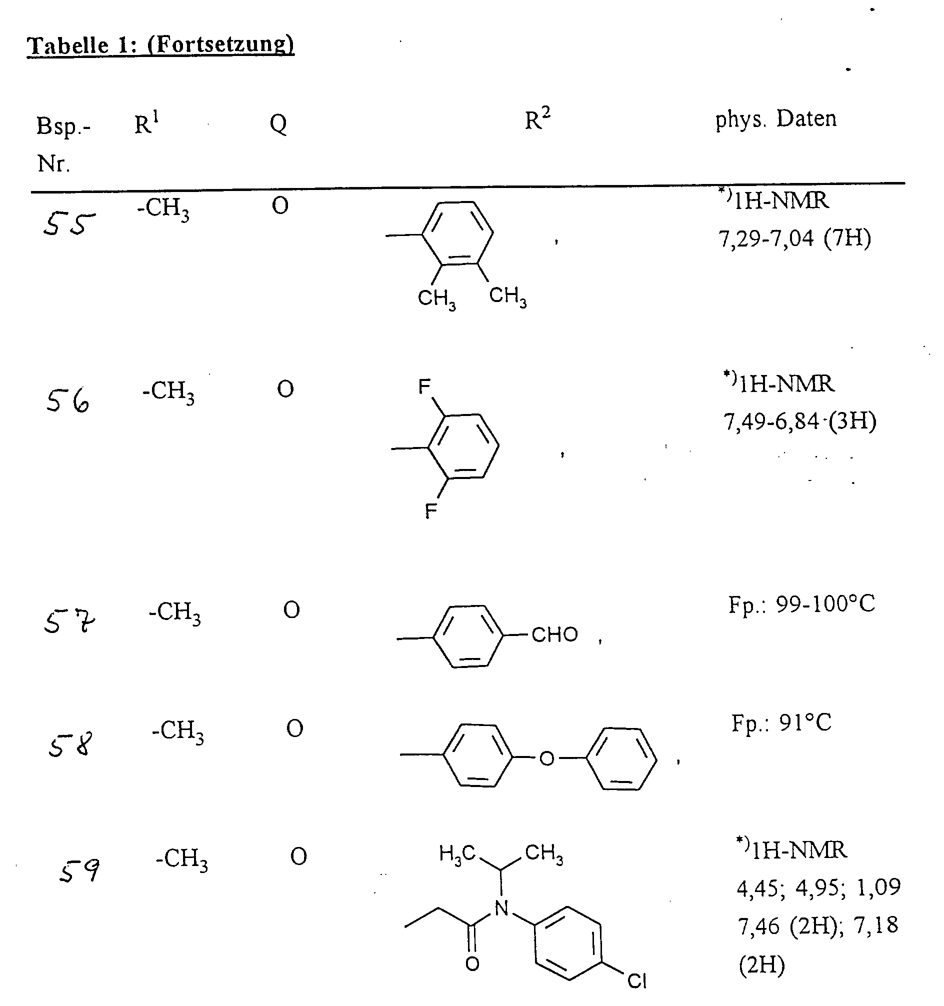

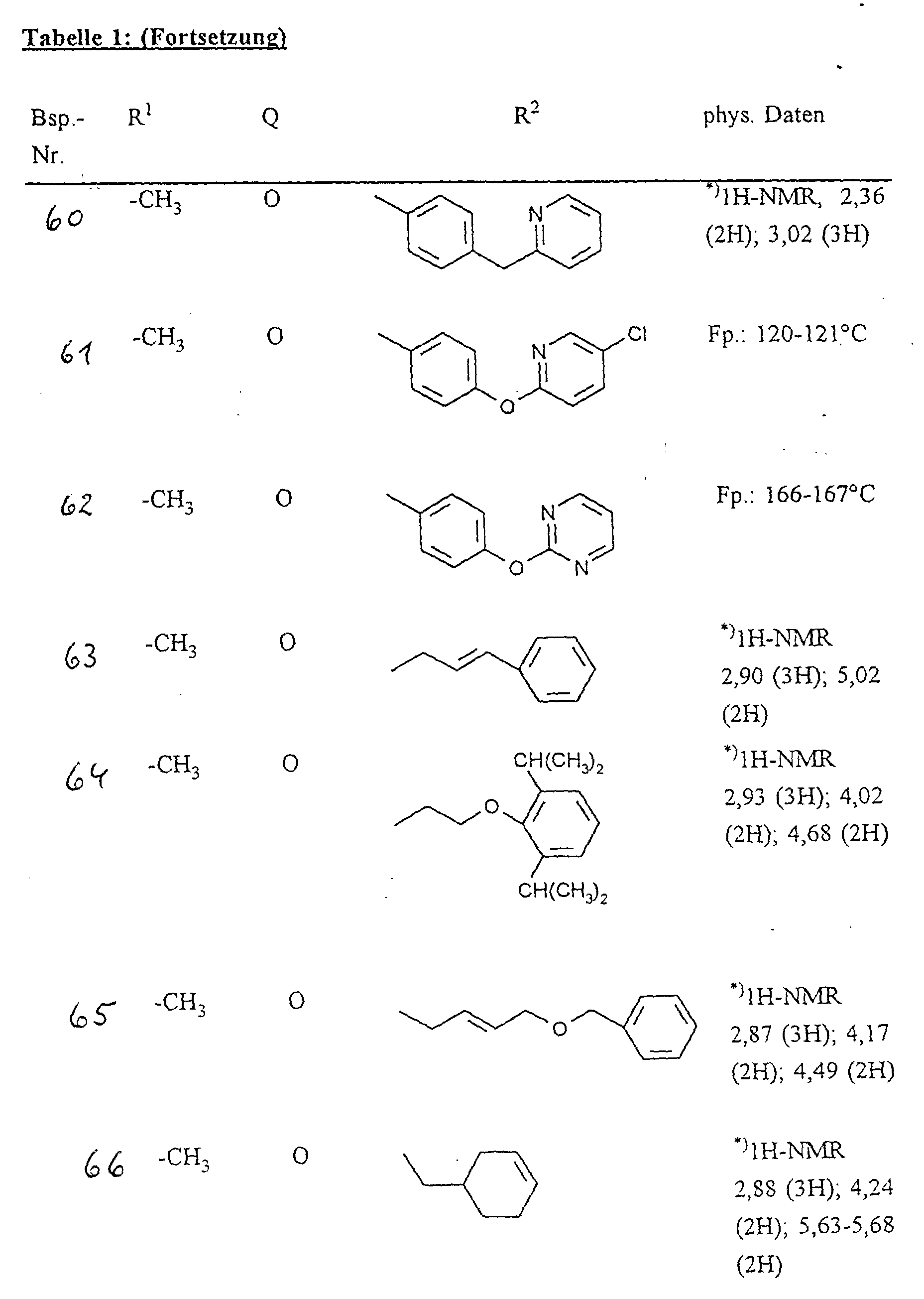

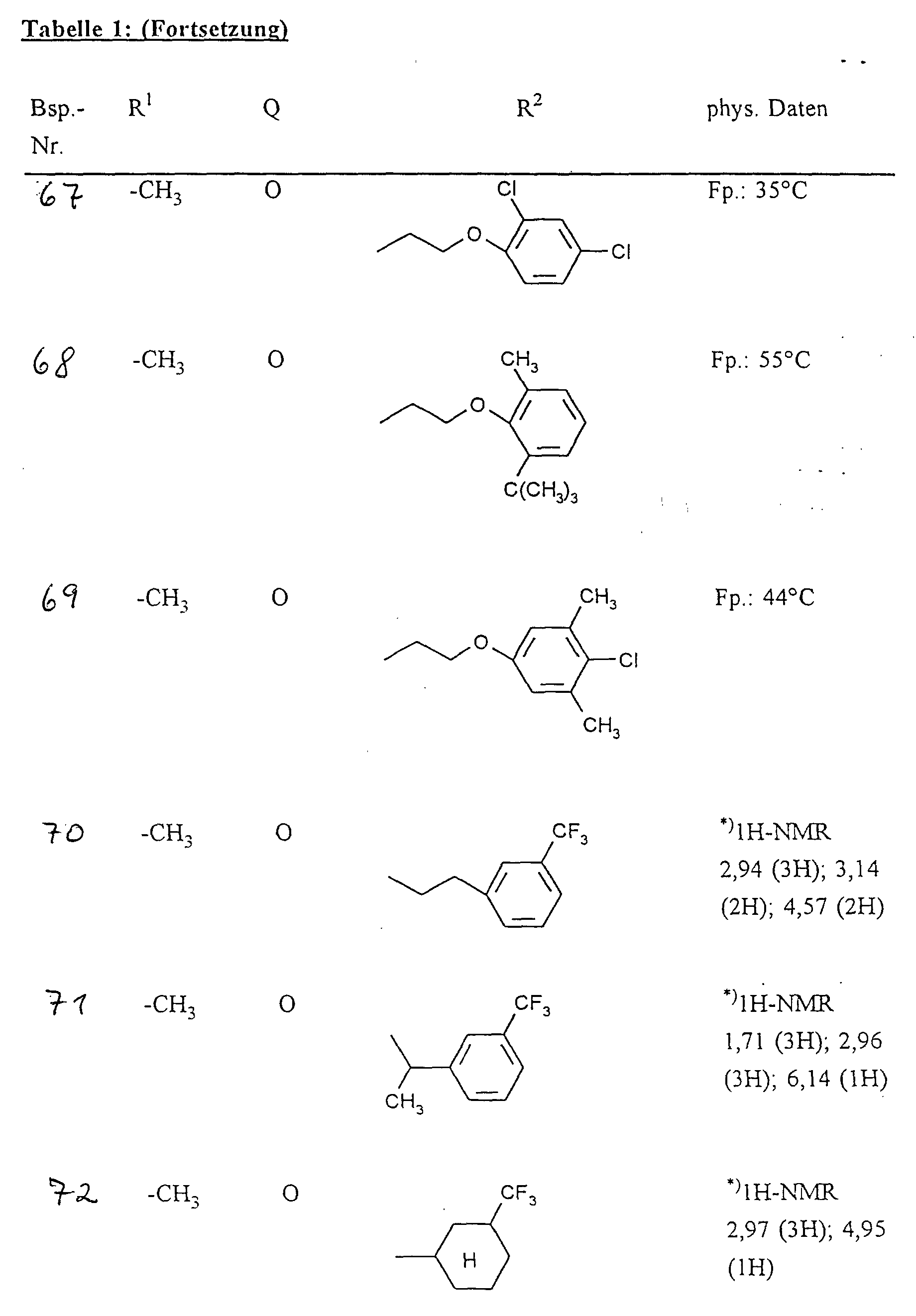

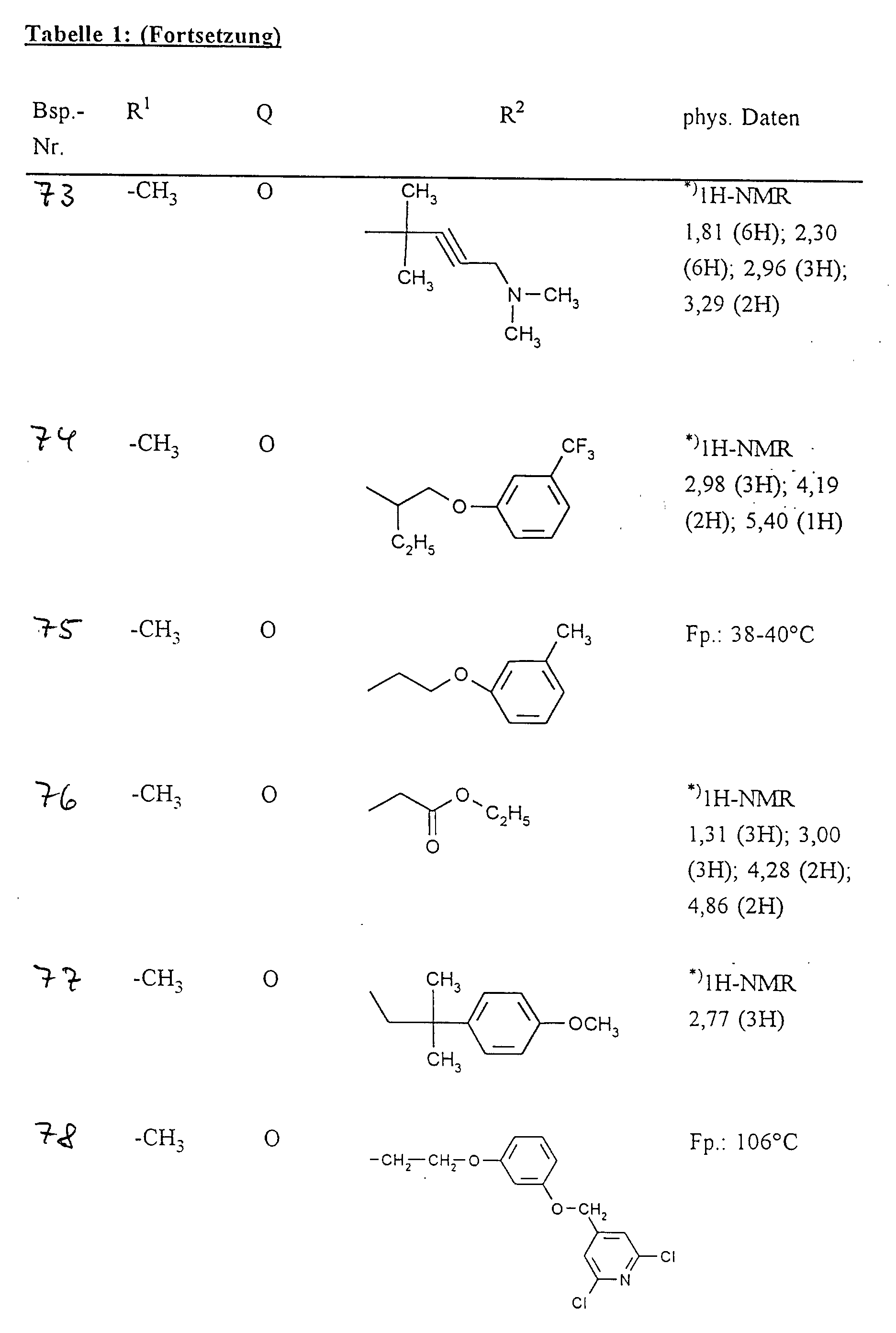

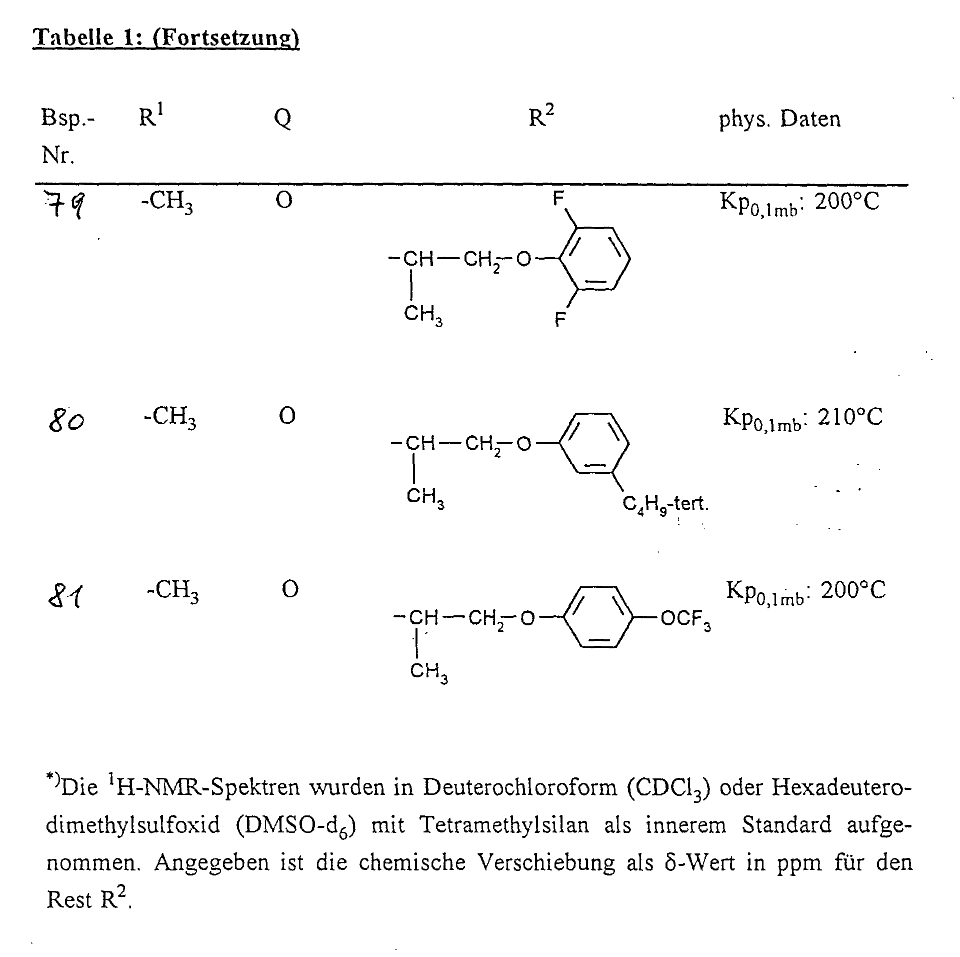

### Verwendungsbeispiele:

### Beispiel A

### Pyricularia-Test (Reis) / protektiv

- Lösungsmittel:: 12,5 Gewichtsteile Aceton
- Emulgator:: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. 5 Tage nach der Behandlung werden die Pflanzen mit einer wäßrigen Sporensuspension von Pyricularia oryzae inokuliert. Anschließend werden die Pflanzen in einem Gewächshaus bei 100% rel. Luftfeuchtigkeit und 25°C aufgestellt.

4 Tage nach der Inokulation erfolgt die Auswertung des Krankheitsbefalls. Dabei bedeutet 0 % ein Wirkungsgrad, der demjenigen der Kontrolle entspricht, während ein Wirkungsgrad von 100 % bedeutet, daß kein Befall beobachtet wird.

In diesem Test zeigen die in den Beispielen 8, 16-18, 20-24, 28, 29, 31, 33 und 34 aufgeführten Stoffe bei einer Wirkstoffkonzentration von 0,025 Gew.-% in der Spritzflüssigkeit einen Wirkungsgrad von mindestens 70 %.

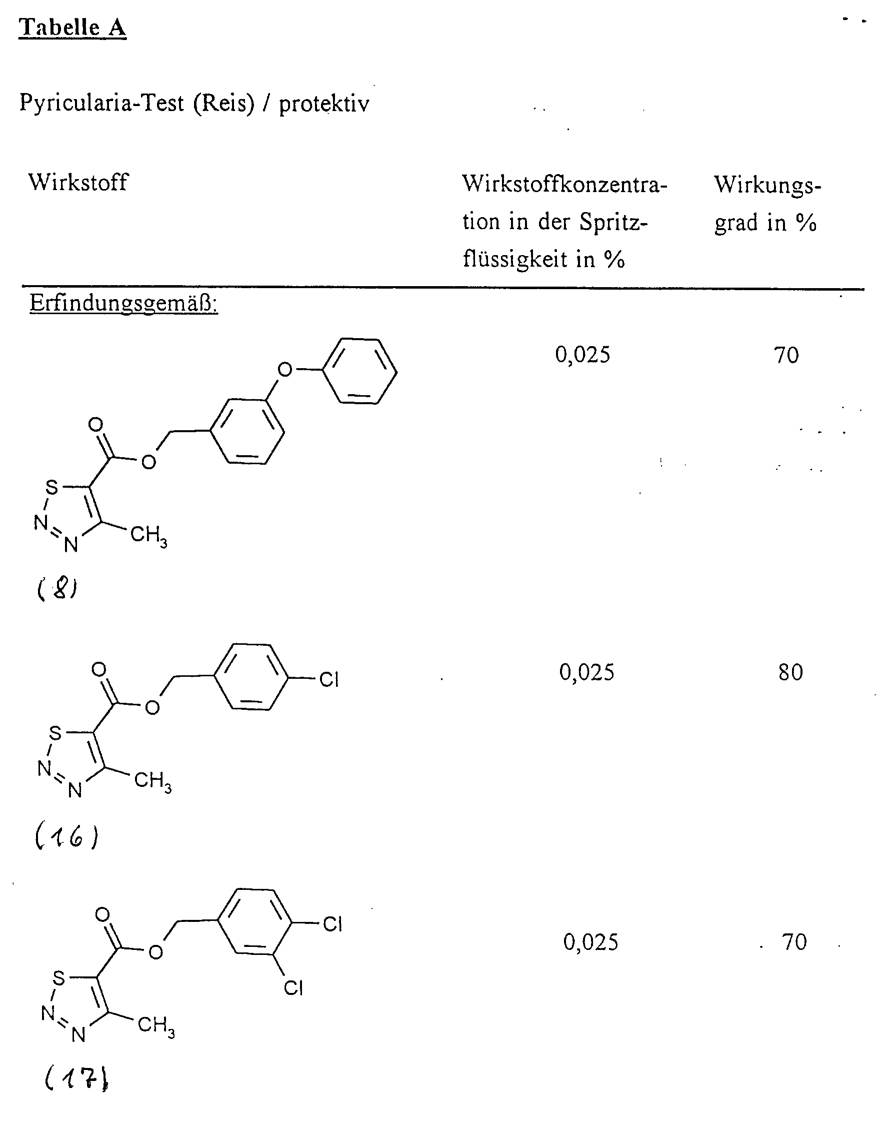

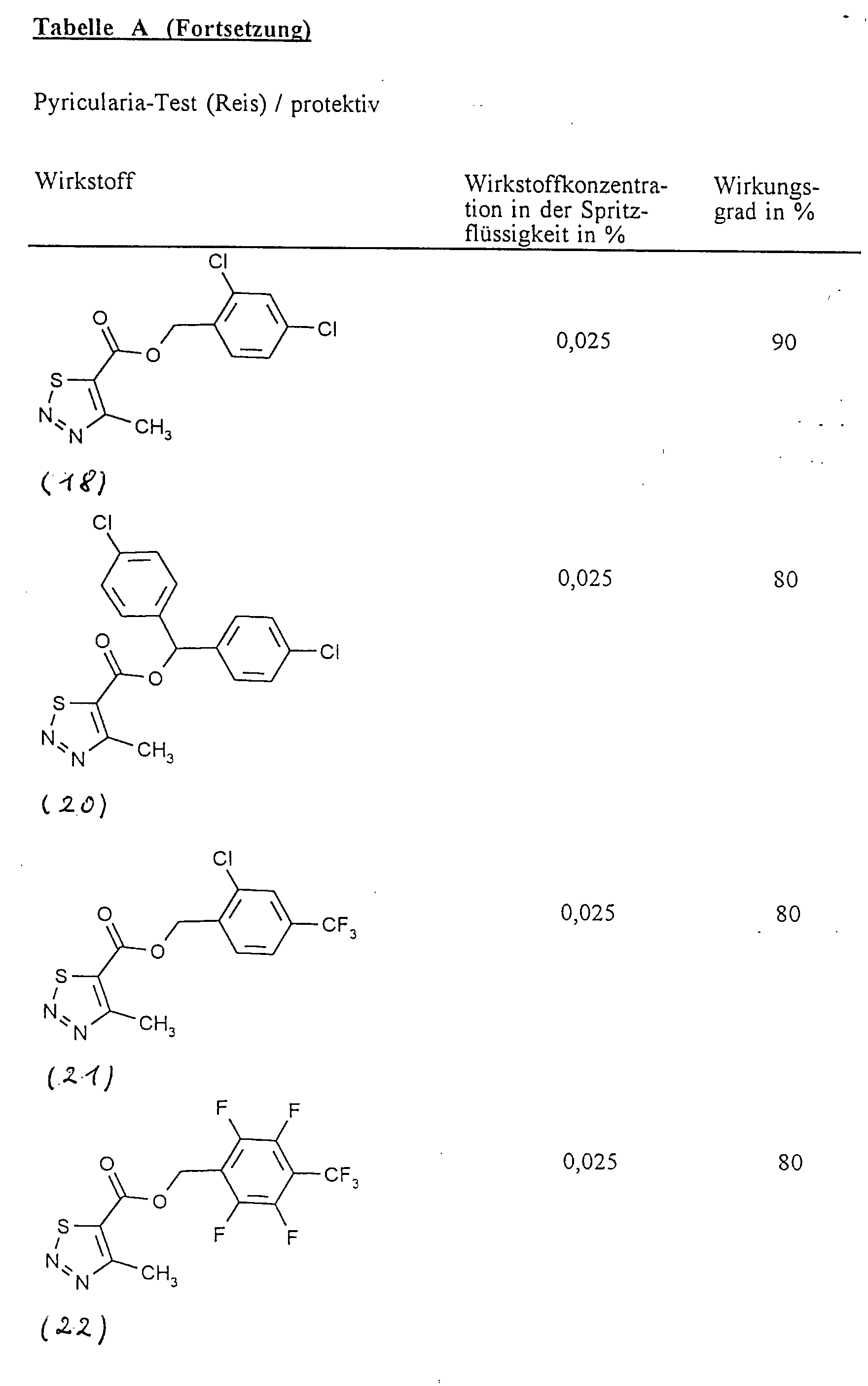

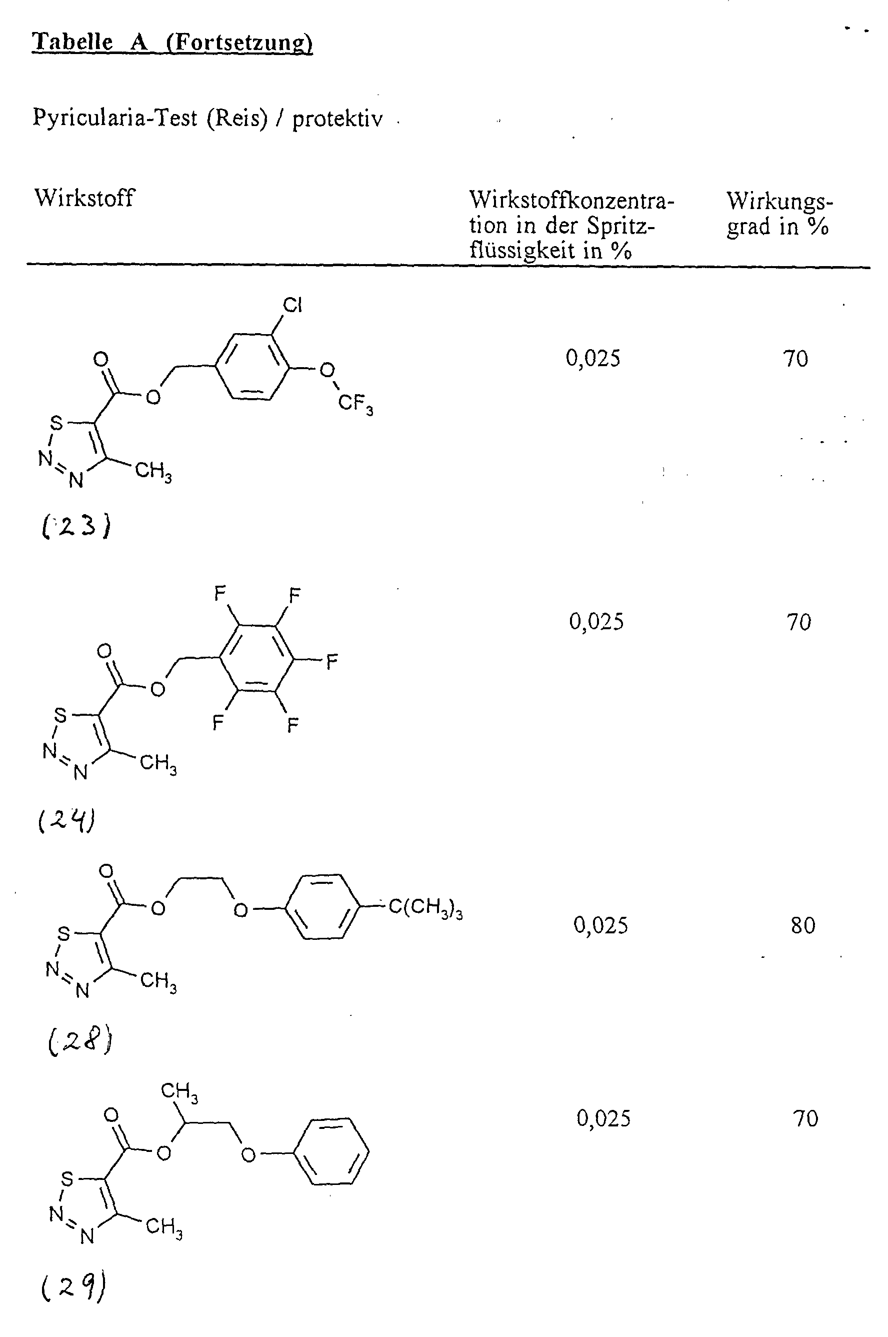

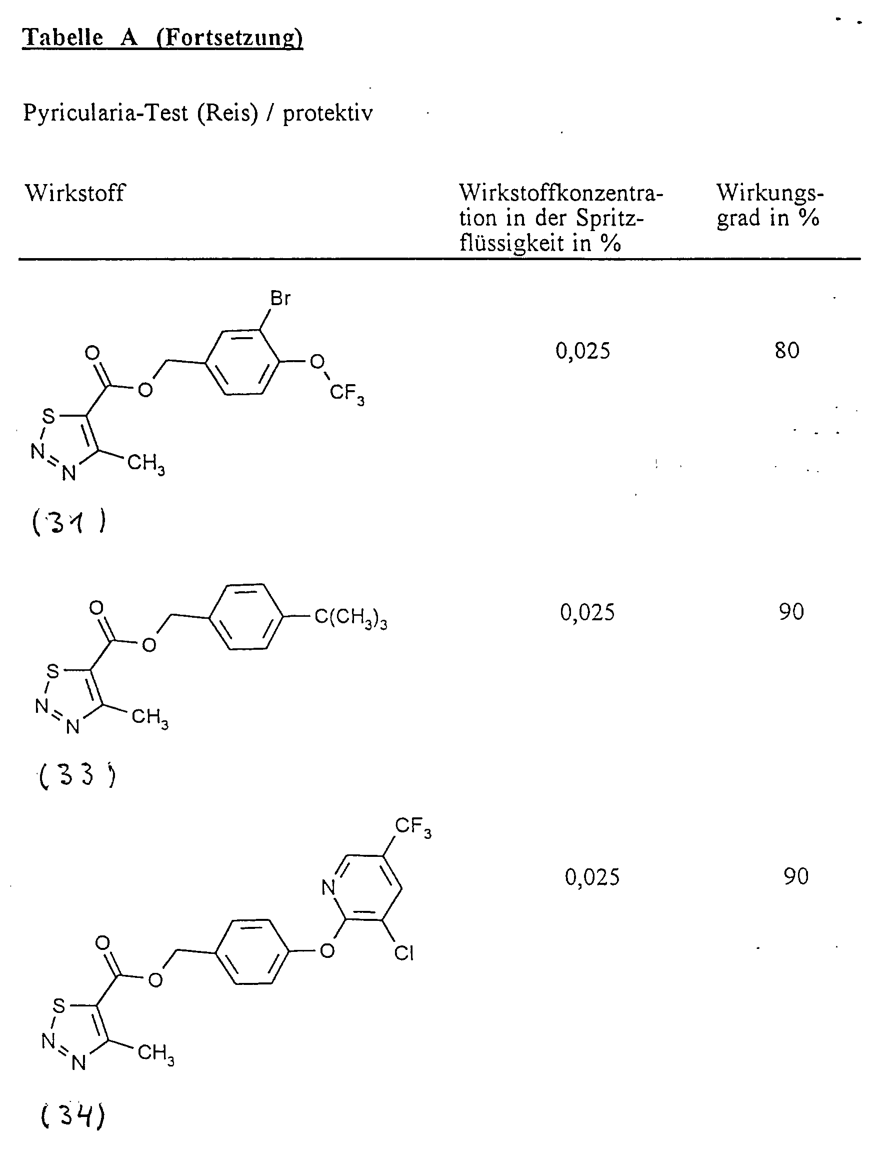

### Beispiel B

### Pyricularia-Test (Reis) / systemisch

- Lösungsmittel:: 12,5 Gewichtsteile Aceton
- Emulgator:: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wäßrigen Sporensuspension von Pyricularia oryzae inokuliert. Danach verbleiben die Pflanzen in einem Gewächshaus bei einer Temperatur von 25°C und einer rel. Luftfeuchtigkeit von 100% bis zur Auswertung.

4 Tage nach der Inokulation erfolgt die Auswertung des Krankheitsbefalls. Dabei bedeutet ein Wirkungsgrad von 0 %, daß der Befall demjenigen der Kontrolle entspricht, und ein Wirkungsgrad von 100 %, daß kein Befall beobachtet wird.

In diesem Test zeigen die in den Beispielen 8, 16, 17, 21-24, 28, 29, 31 und 34 aufgeführten Stoffe bei einer Konzentration von 100 mg Wirkstoff pro 100 cm² einen Wirkungsgrad von über 65 %.

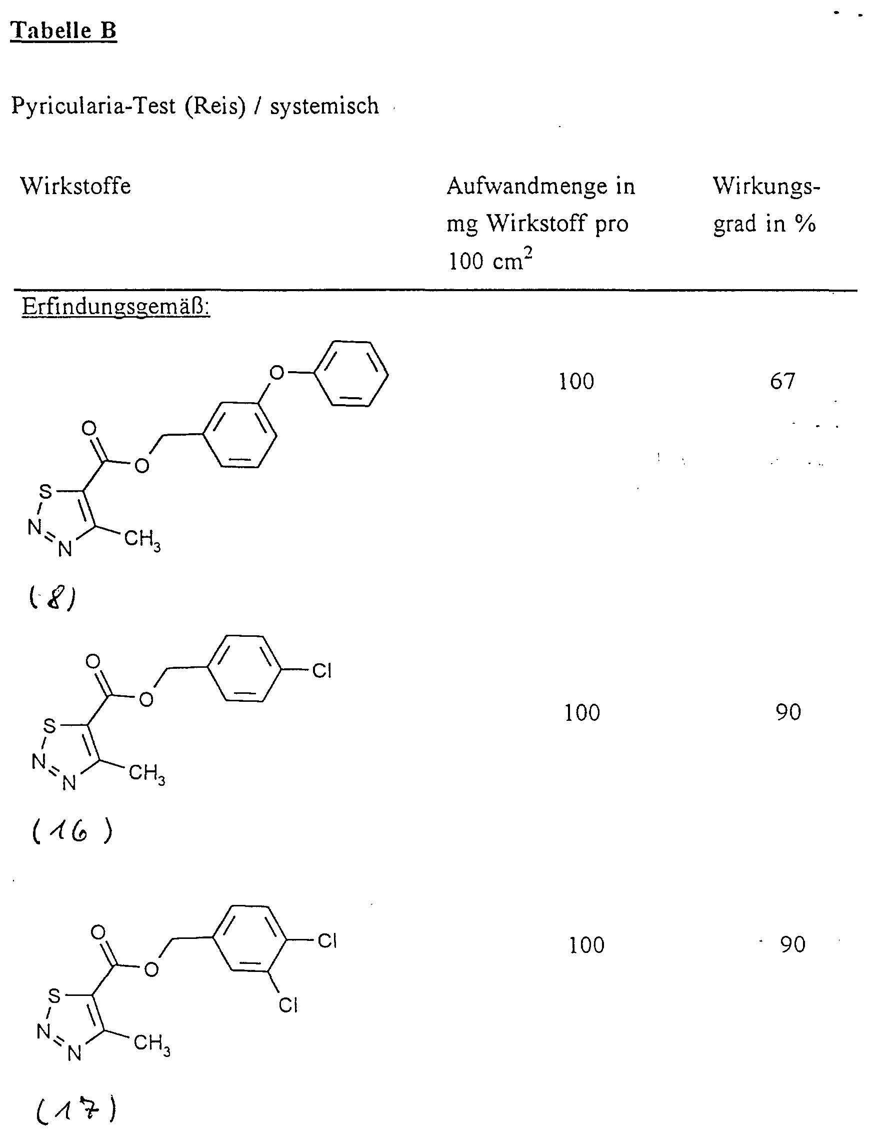

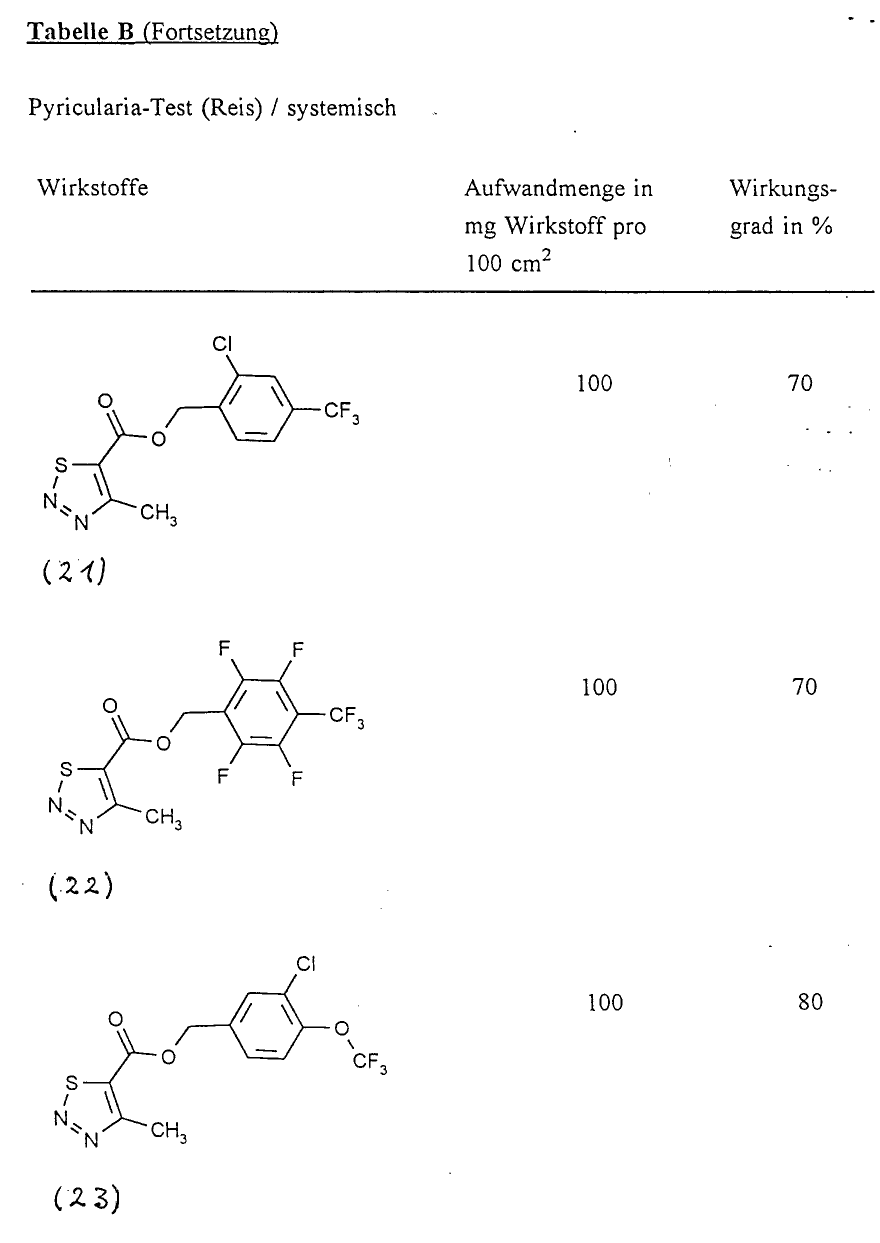

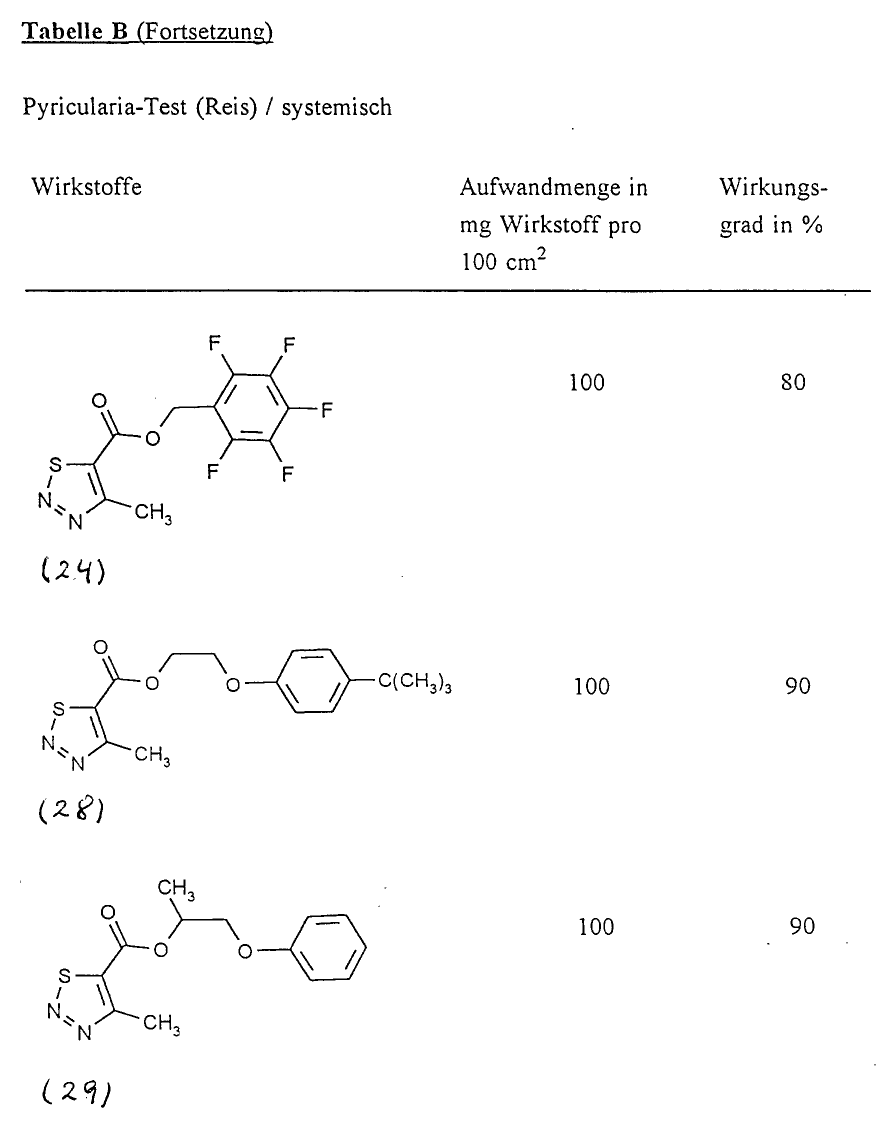

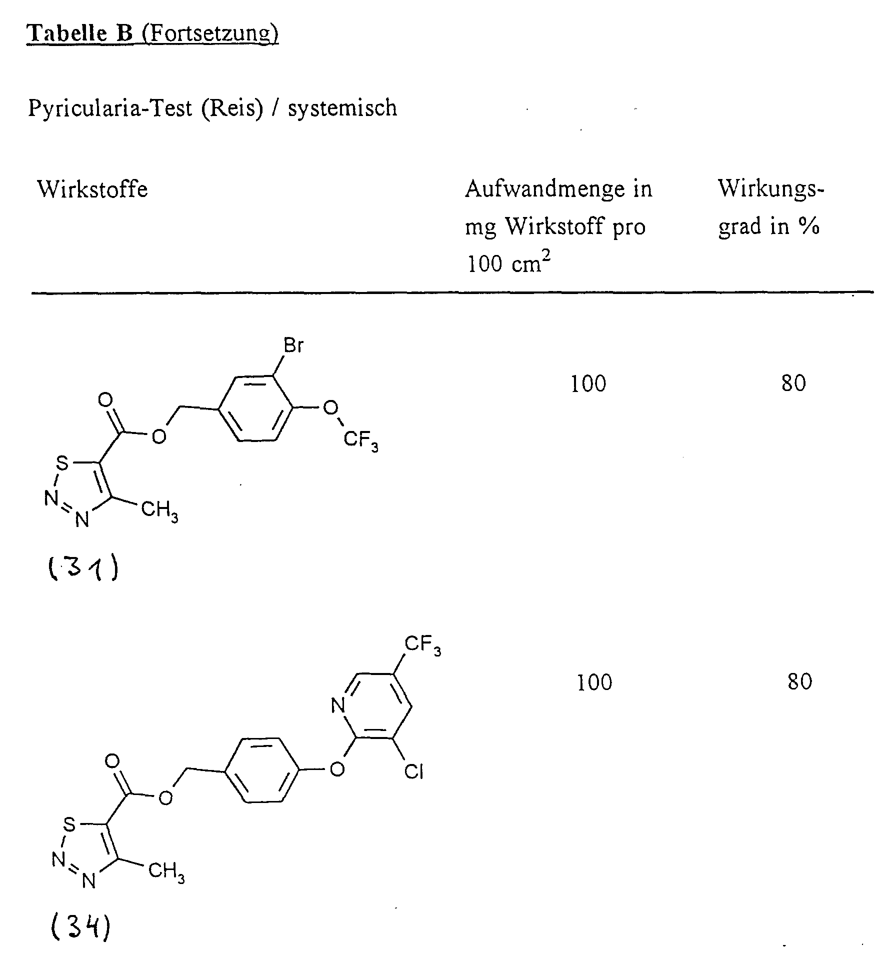

### Beispiel C

### Uncinula-Test (Rebe) / protektiv

- Lösungsmittel:: 47 Gewichtsteile Aceton
- Emulgator:: 3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit Konidien des Pilzes Uncinula necator bestäubt. Die Pflanzen werden anschließend bei 23 bis 24°C und bei einer relativen Luftfeuchtigkeit von ca 75% im Gewächshaus aufgestellt.

14 Tage nach der Inokulation erfolgt die Auswertung. Dabei bedeutet ein Wirkungsgrad von 0 %, daß der Befall demjenigen der Kontrolle entspricht, und ein Wirkungsgrad von 100 %, daß kein Befall beobachtet wird.

In diesem Test zeigt der im Beispiel 10 aufgeführte Wirkstoff bei einer Wirkstoffkonzentration von 25 ppm in der Spritzflüssigkeit einen Wirkungsgrad von über 80 %.

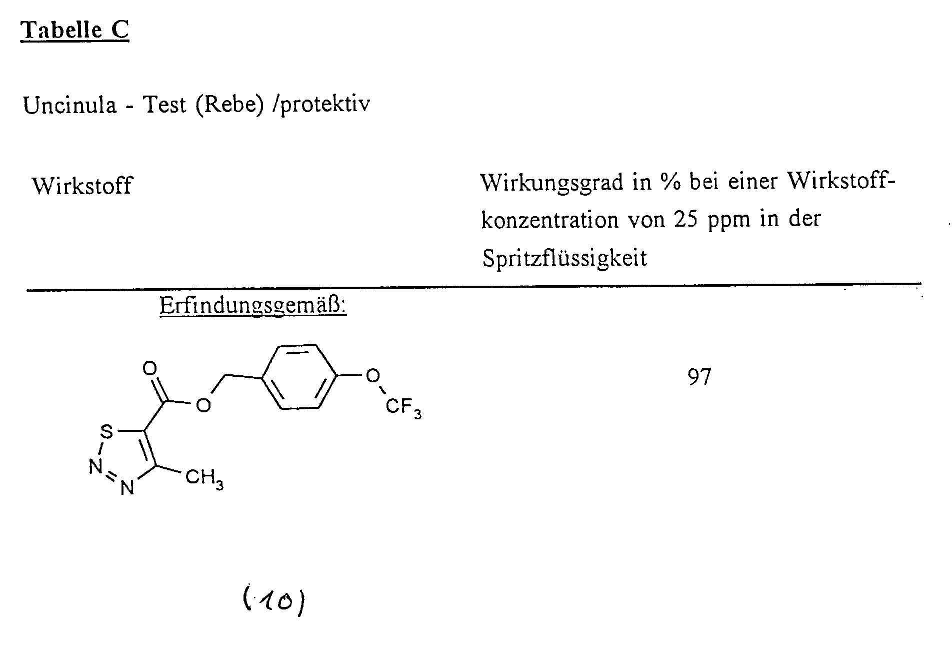

### Beispiel D

### Test mit Fliegen (Musca domestica)

- Testtiere:: Musca domestica, Stamm Reichswald (OP, SP, Carbamatresistent)
- Lösungsmittel:: 35 Gewichtsteile Ethylenglykolmonomethylether
- Emulgator:: 35 Gewichtsteile Nonylphenolpolyglykolether

Zur Herstellung einer geeigneten Formulierung vermischt man drei Gewichtsteile Wirkstoff mit sieben Teilen des oben angegebenen Lösungsmittel-Emulgator-Gemisches und verdünnt das so erhaltene Emulsionskonzentrat mit Wasser auf die jeweils gewünschte Konzentration.

2 ml dieser Wirkstoffzubereitung werden auf Filterpapierschalen (Ø 9,5 cm) pipettiert, die sich in Petrischalen entsprechender Größe befinden. Nach Trocknung der Filterscheiben werden 25 Testtiere in die Petrischale überführt und abgedeckt.

Nach 24 Stunden wird die Wirksamkeit der Wirkstoffzubereitung ermittelt. Dabei bedeutet 100%, daß alle Fliegen abgetötet wurden; 0% bedeutet, daß keine Fliegen abgetötet wurden.

In diesem Test zeigen die in den Beispielen 23, 24 und 31 aufgeführten Wirkstoffe bei einer Konzentration von 1000 ppm in der Wirkstoffzubereitung einen Wirkungsgrad von 100 %.

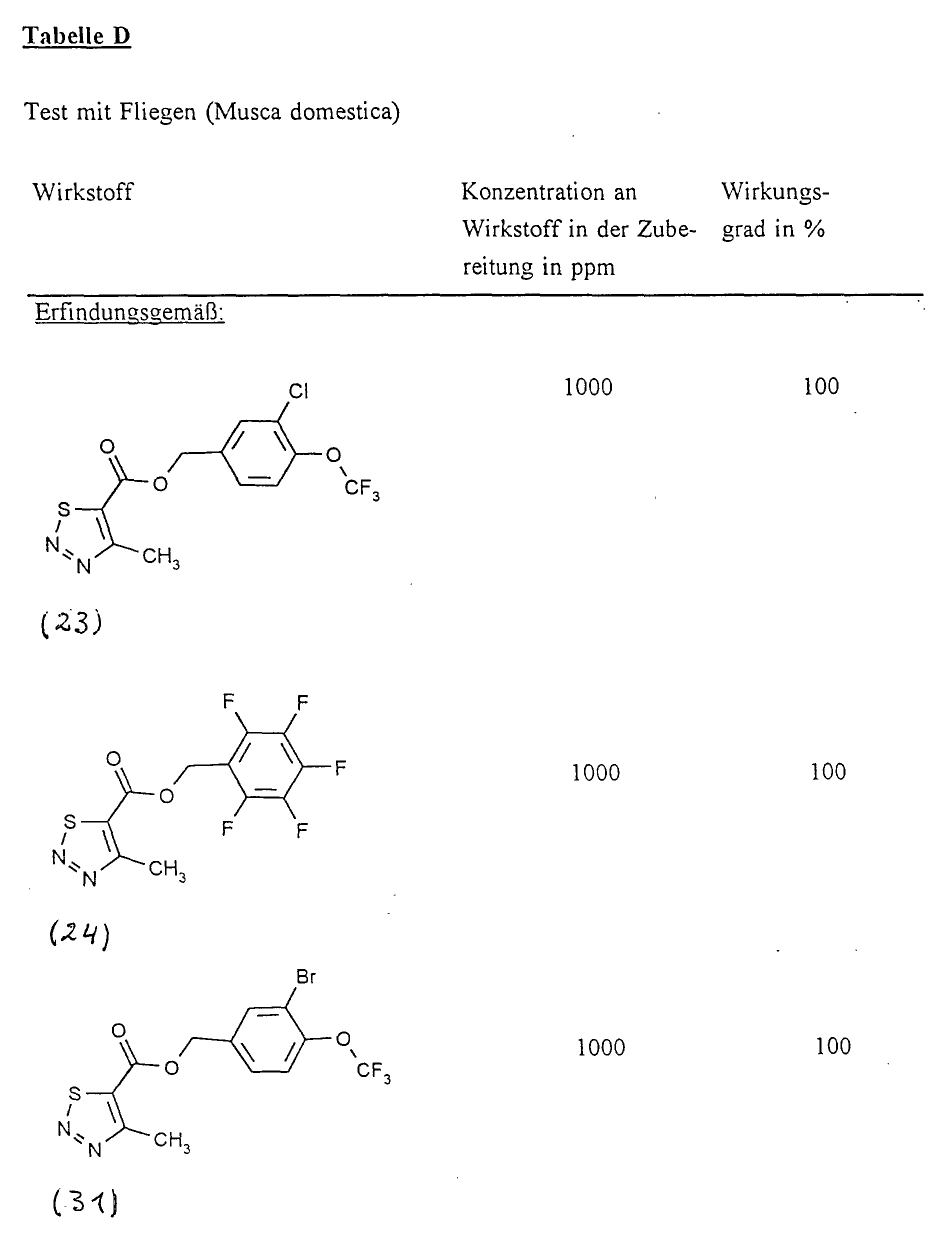

### Beispiel E

### Blowfly-Larven-Test/Entwicklungshemmende Wirkung

- Testtiere:: Lucilia cuprina-Larven
- Lösungsmittel:: 35 Gewichtsteile Ethylenglykolmonomethylether
- Emulgator:: 35 Gewichtsteile Nonylphenolpolyglykolether

Zur Herstellung einer zweckmäßigen Wirkstoffzubereitung vermischt man drei Gewichtsteile Wirkstoff mit sieben Gewichtsteilen des oben angegebenen Gemisches und verdünnnt das so erhaltene Emulsionskonzentrat mit Wasser auf die jeweils gewünschte Konzentration.

Etwa 20 Lucilia cuprina res.-Larven werden in ein Teströhrchen gebracht, welches ca. 1 cm³ Pferdefleisch und 0,5 ml der Wirkstoffzubereitung enthält. Nach 48 Stunden wird die Wirksamkeit der Wirkstoffzubereitung ermittelt. Die Teströhrchen werden im Becher mit Sand-bedecktem Boden überführt. Nach weiteren 2 Tagen werden die Teströhrchen entfernt und die Puppen gezählt.

Die Wirkung der Wirkstoffzubereitung wird nach der Zahl der geschlüpften Fliegen nach 1,5-facher Entwicklungsdauer einer unbehandelten Kontrolle beurteilt. Dabei bedeutet 100%, daß keine Fliegen geschlüpft sind; 0% bedeutet, daß alle Fliegen normal geschlüpft sind.

In diesem Test zeigt der im Beispiel 31 aufgeführte Wirkstoff bei einer Konzentration von 1000 ppm in der Wirkstoffzubereitung einen Wirkungsgrad von 100 %.

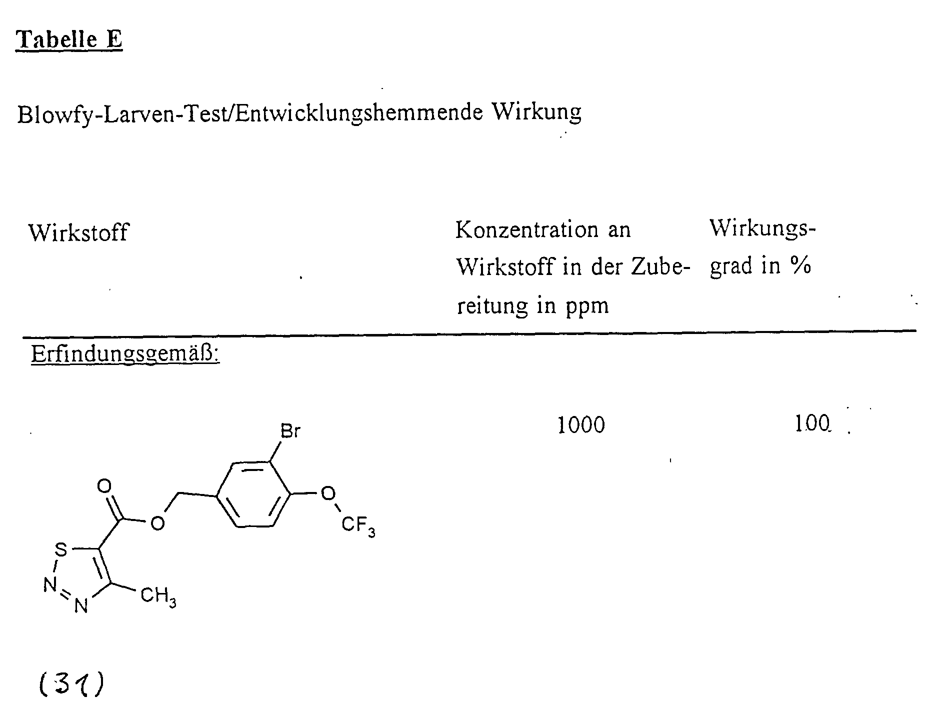

## Patentansprüche

1. Verwendung von 1,2,3-Thiadiazolcarbonsäure(thio)estern der Formel 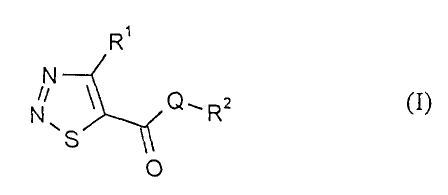 in welcher die Substituenten die nachstehend angegebenen Bedeutungen haben 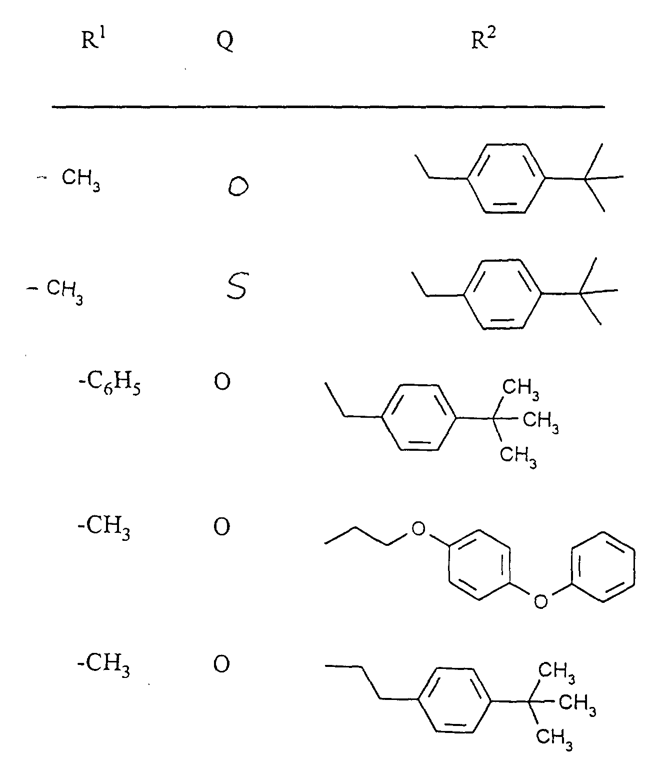 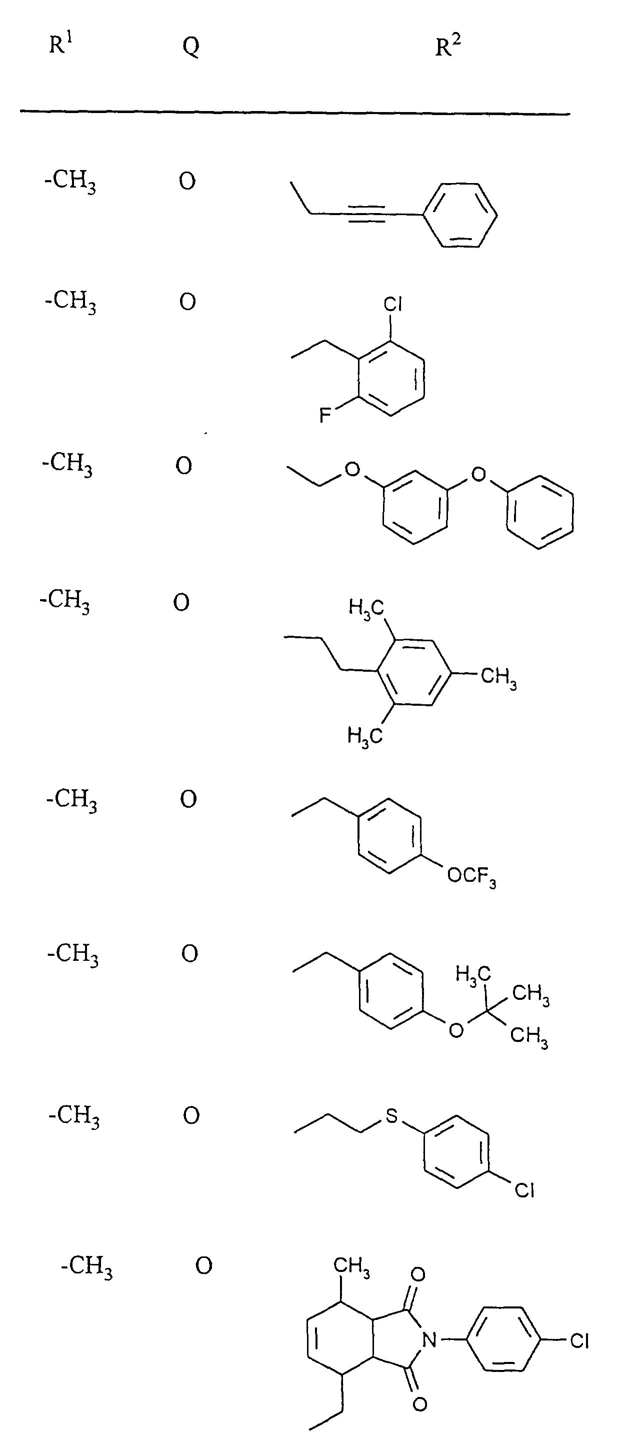 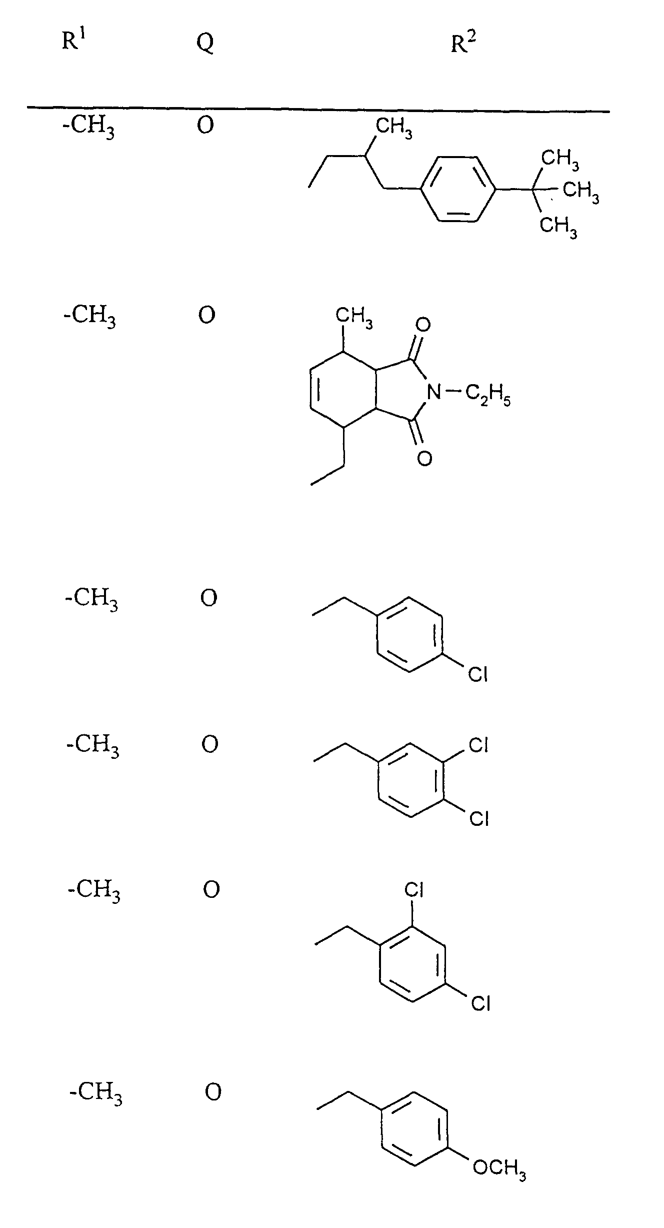 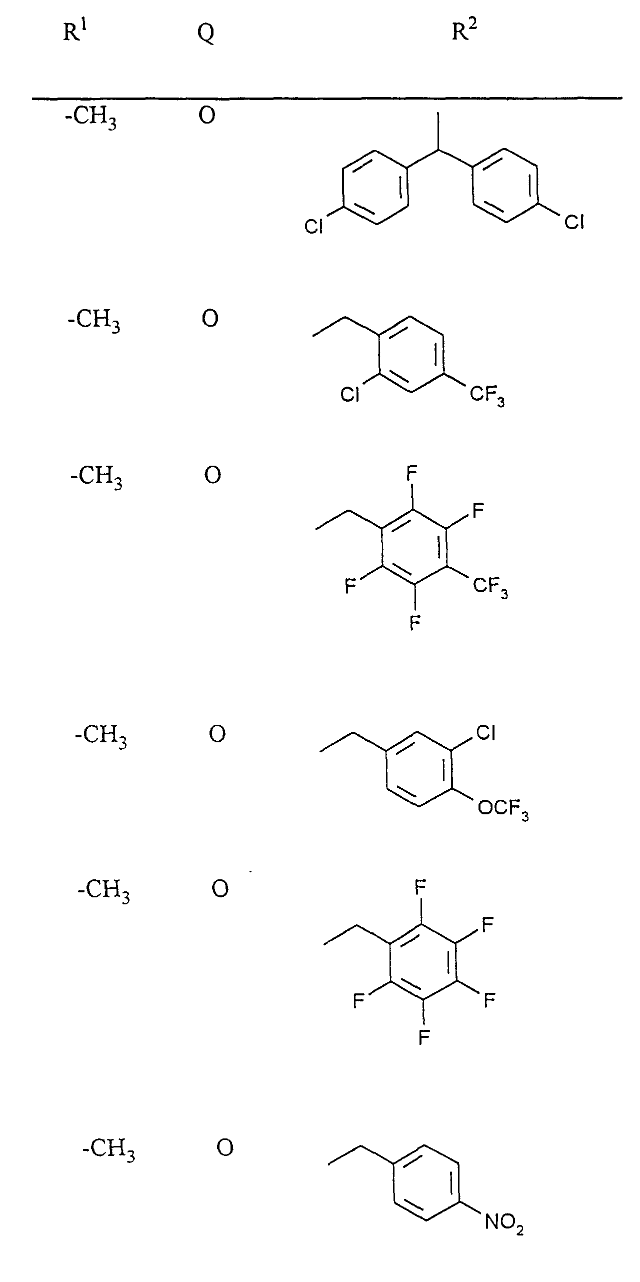 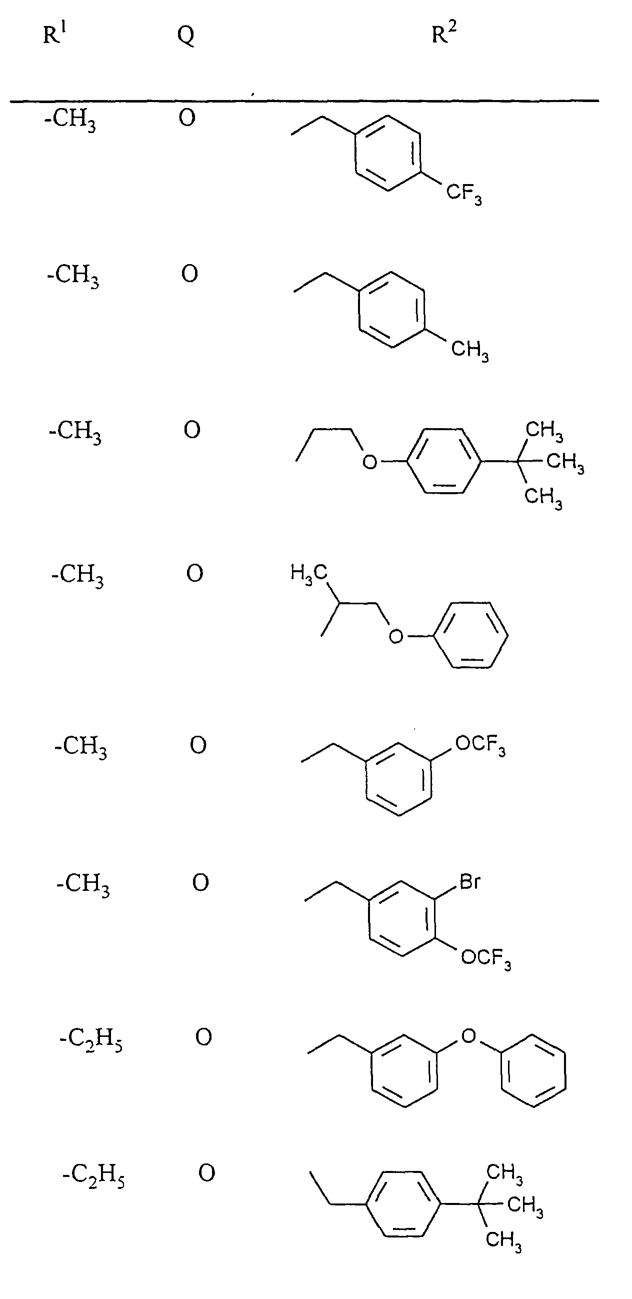 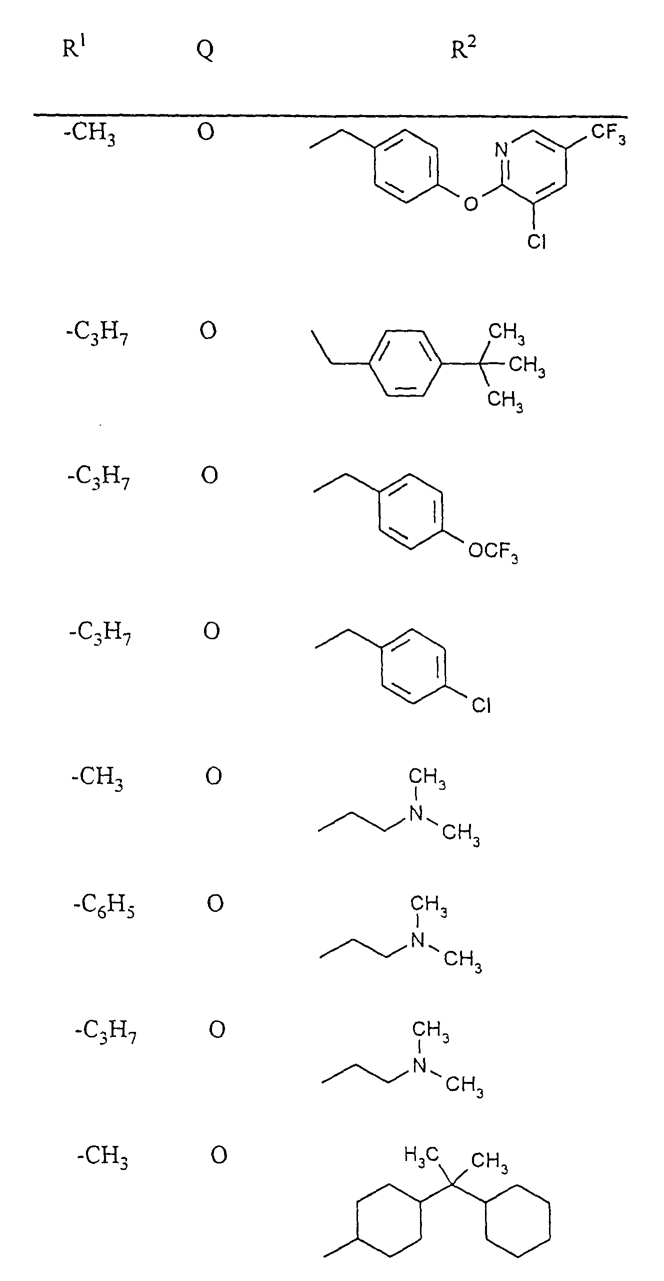 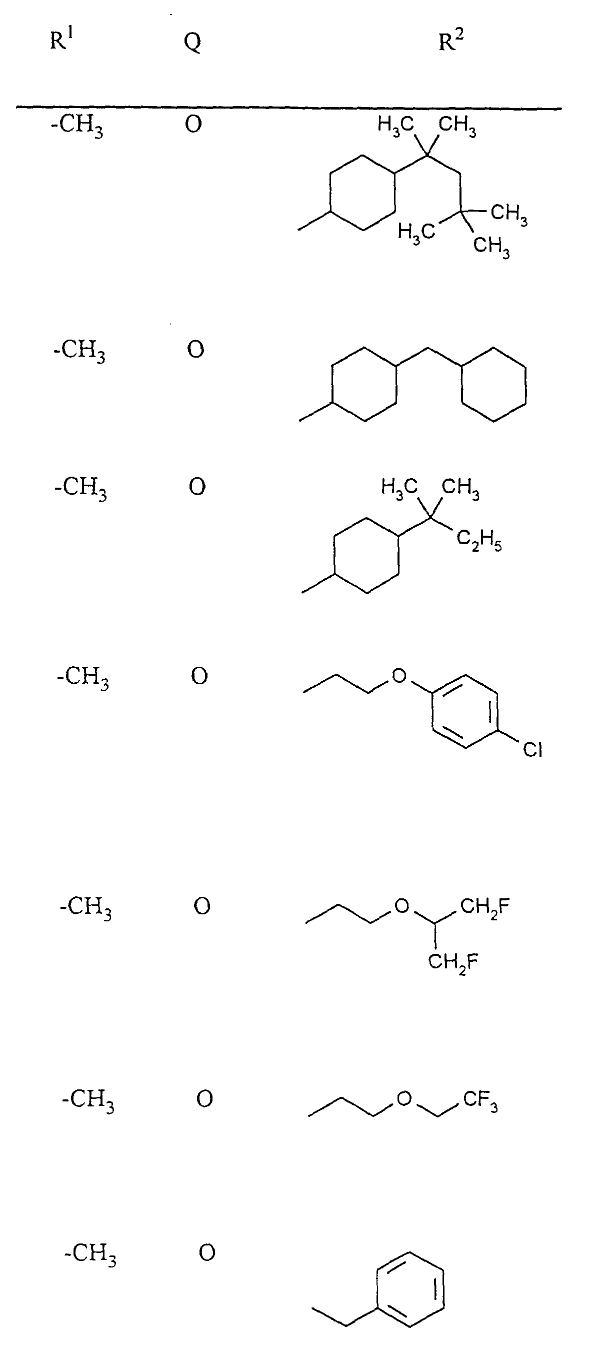 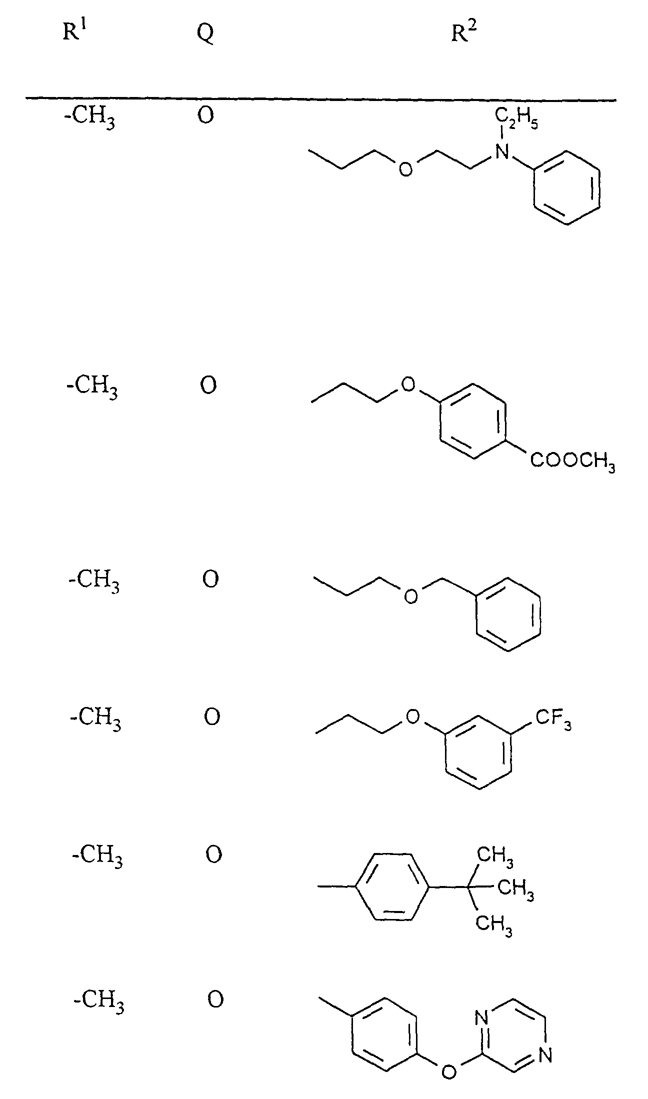 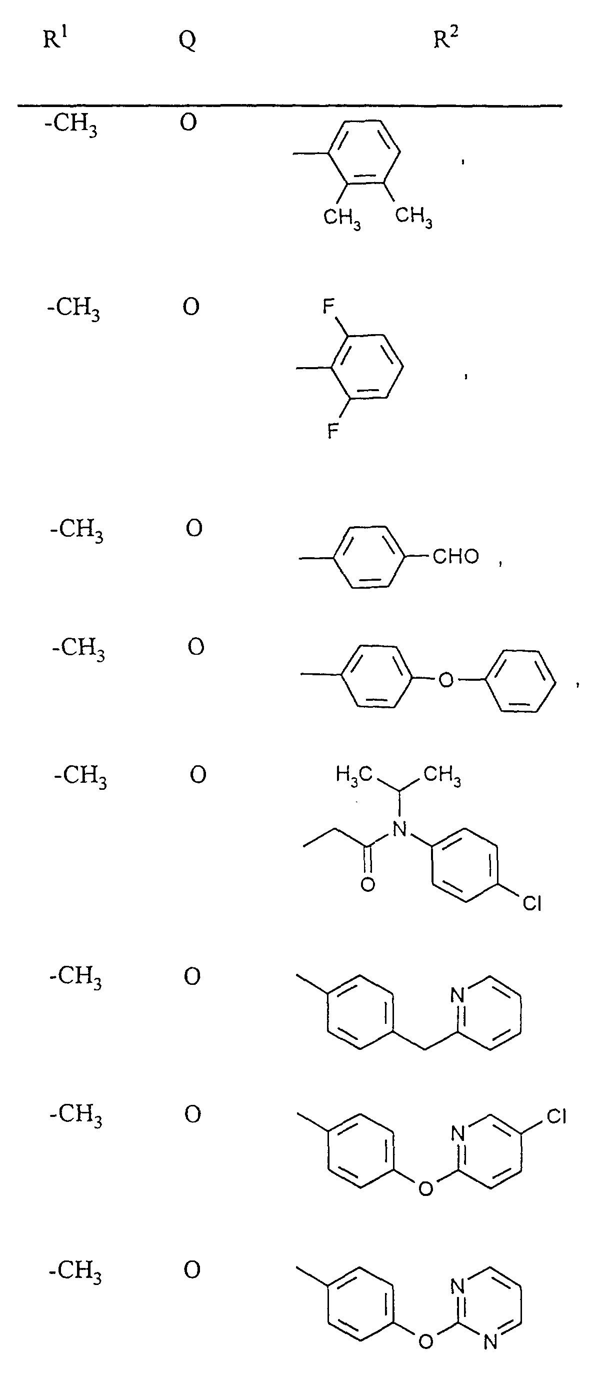 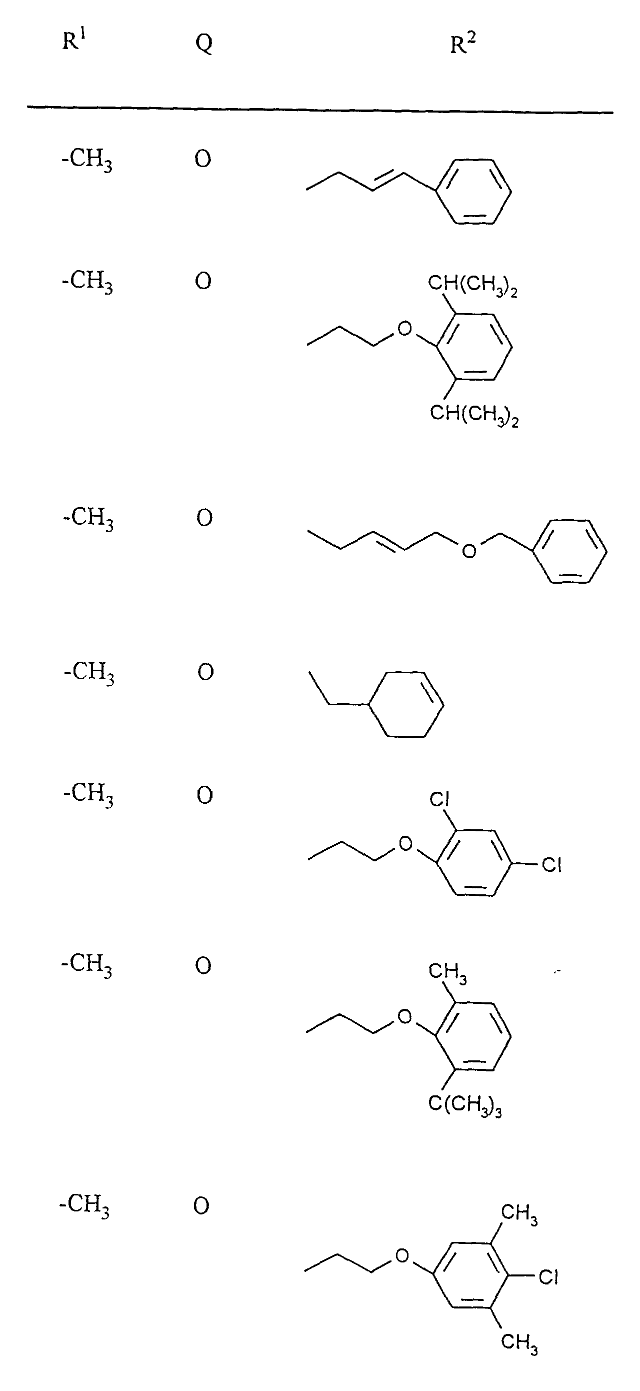 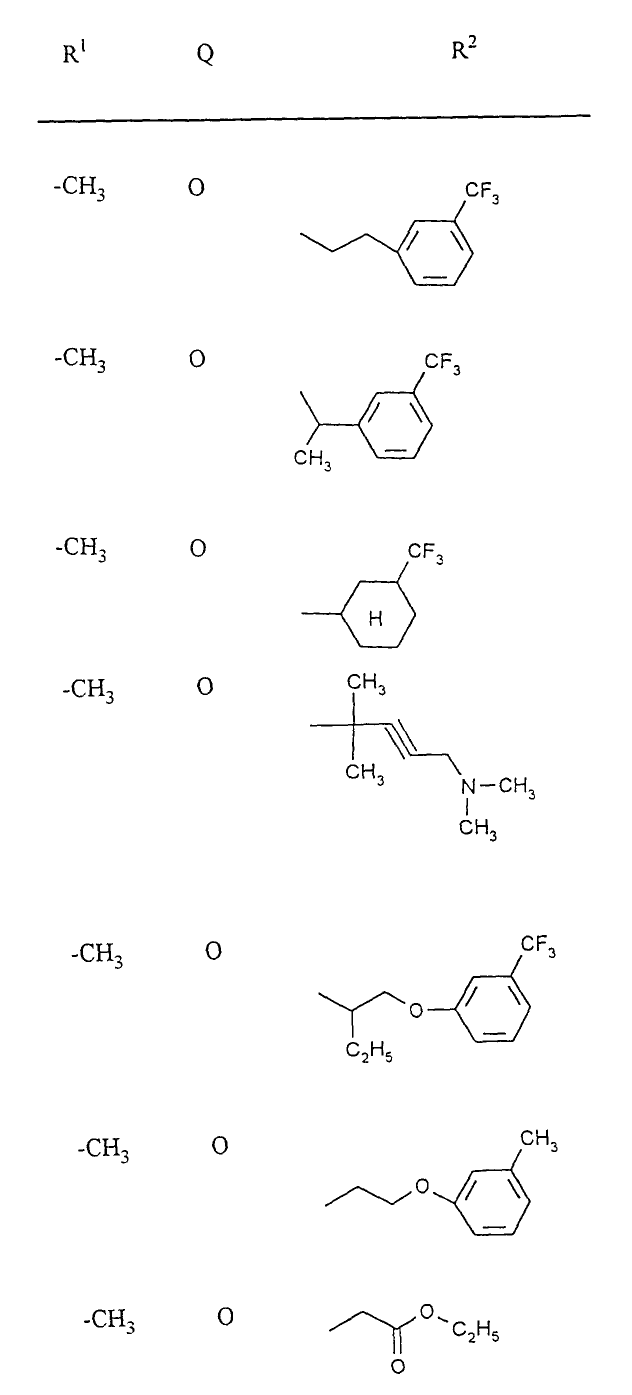 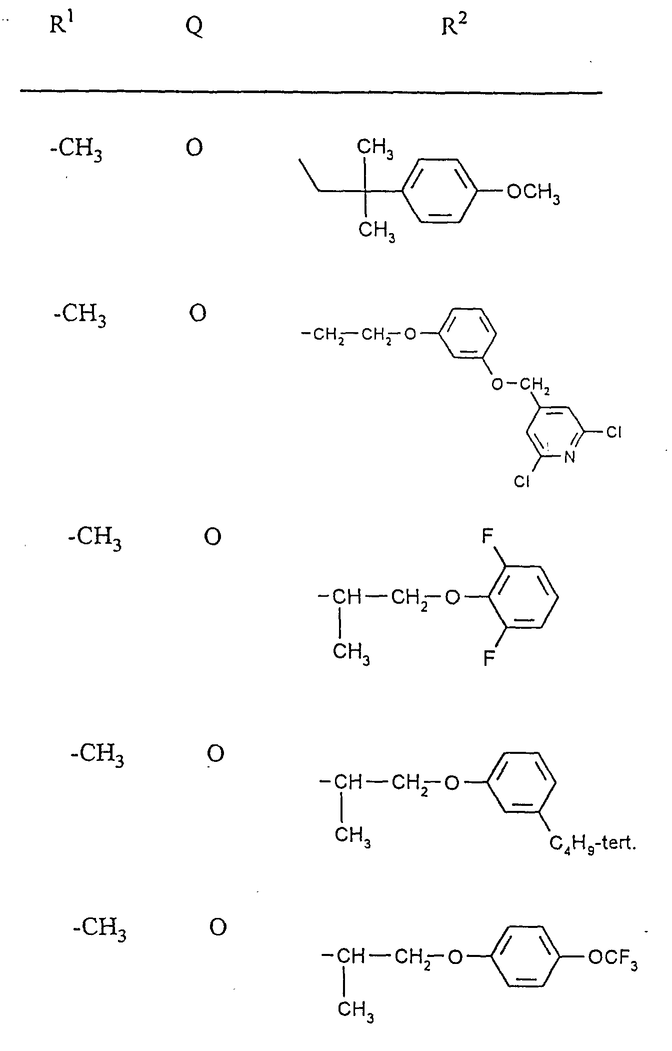 zum Schutz von Pflanzen gegen Befall durch unerwünschte Mikroorganismen und tierische Schädlinge .

2. Verfahren zum Schutz von Pflanzen gegen Befall durch unerwünschte Mikroorganismen und tierische Schädlinge , **dadurch gekennzeichnet, daß** man 1,2,3 - Thiadiazolcarbonsäure (thio )-ester der Formel (I) gemäß Anspruch 1 auf die Mikroorganismen oder die tierischen Schädlinge und / oder deren Lebensraum aufbringt.

3. 1,2,3 - Thiadiazolcarbonsäure (thio) ester der Formel (I) gemäß Anspruch 1

4. Verfahren zur Herstellung von 1,2,3 - Thiadiazolcarbonsäure (thio) estern der Formel (I) gemäß Anspruch 1, **dadurch gekennzeichnet, daß** man 1,2,3 - Thiadiazolcarbonsäure - Derivate der Formel 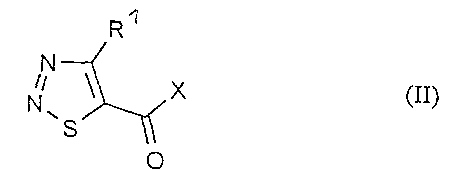 in welcher
R¹ die oben angegebene Bedeutung hat und
X für Halogen steht,
mit (Thio)Alkoholen der Formel
H-Q-R²
in welcher
Q und R² die oben angegebenen Bedeutungen haben,
gegebenenfalls in Gegenwart eines Säurebindemittels und gegebenenfalls in Gegenwart eines Verdünnungsmittels umsetzt.

5. Mittel zum Schutz von Pflanzen gegen Befall durch unerwünschte Mikroorganismen und tierische Schädlinge, **gekennzeichnet durch** einen Gehalt an mindestens einem 1,2,4-Thiadiazolcarbonsäure-(thio)ester der Formel (I ) gemäß Anspruch 1.

6. Verfahren zur Herstellung von Mitteln zum Schutz von Pflanzen gegen Befall durch unerwünschte Mikroorganismen und tierische Schädlinge, **dadurch gekennzeichnet, daß** man 1,2,3-Thiadiazolcarbonsäure(thio)ester der Formel (Ia) gemäß Anspruch 1 mit Streckmitteln und/oder oberflächenaktiven Stoffen vermischt.

## Claims

1. Use of 1,2,3-thiadiazolecarboxylic (thio)esters of the formula 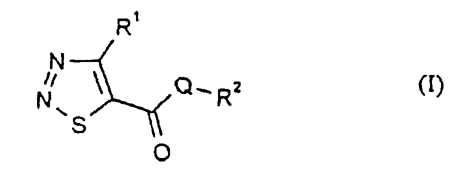 in which the substituents are as defined below 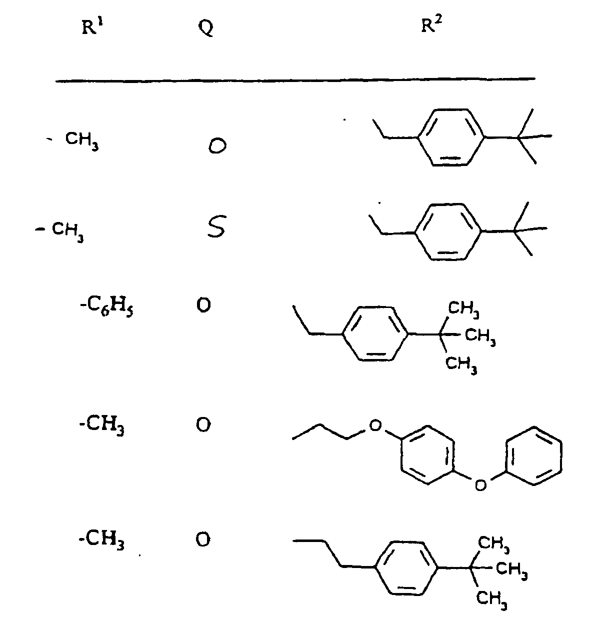 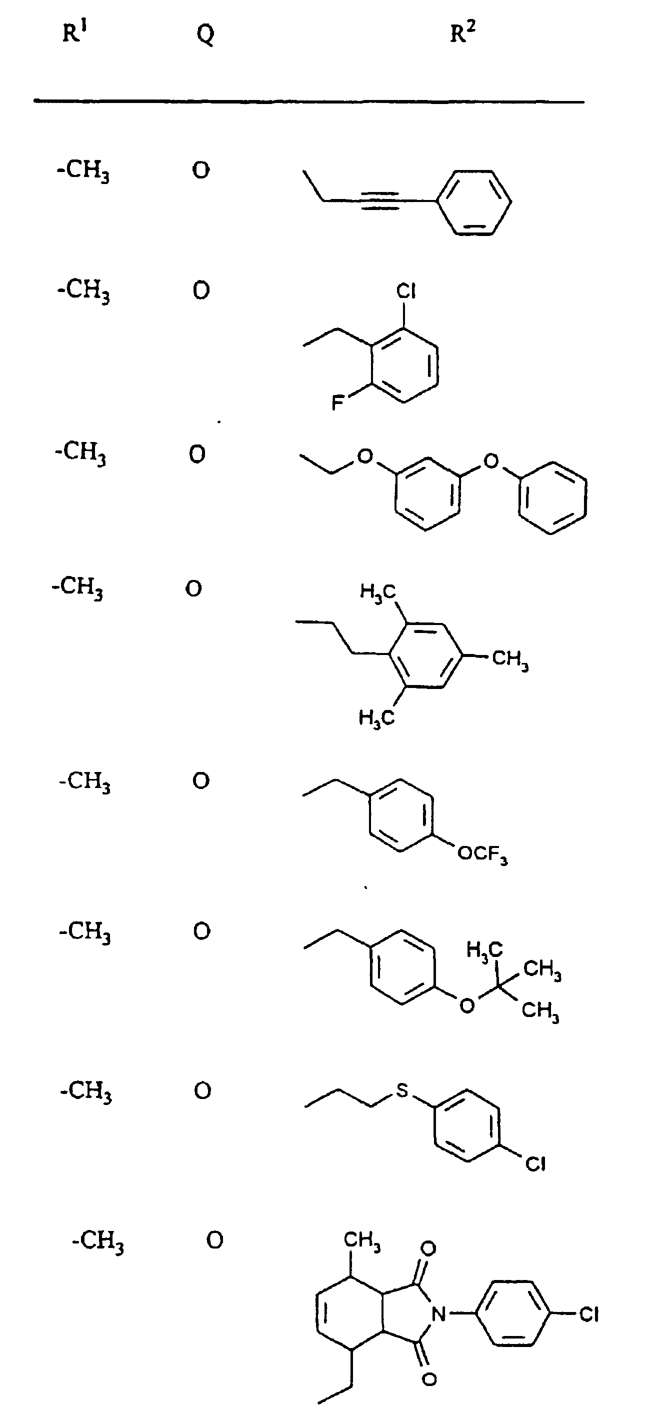 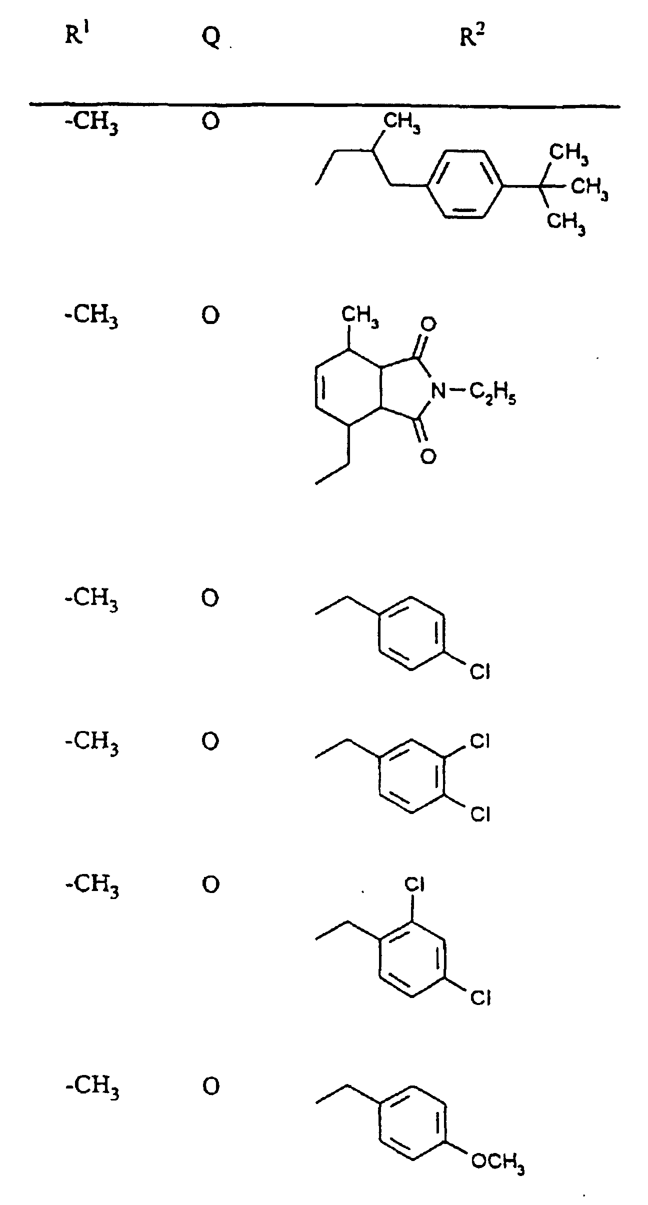 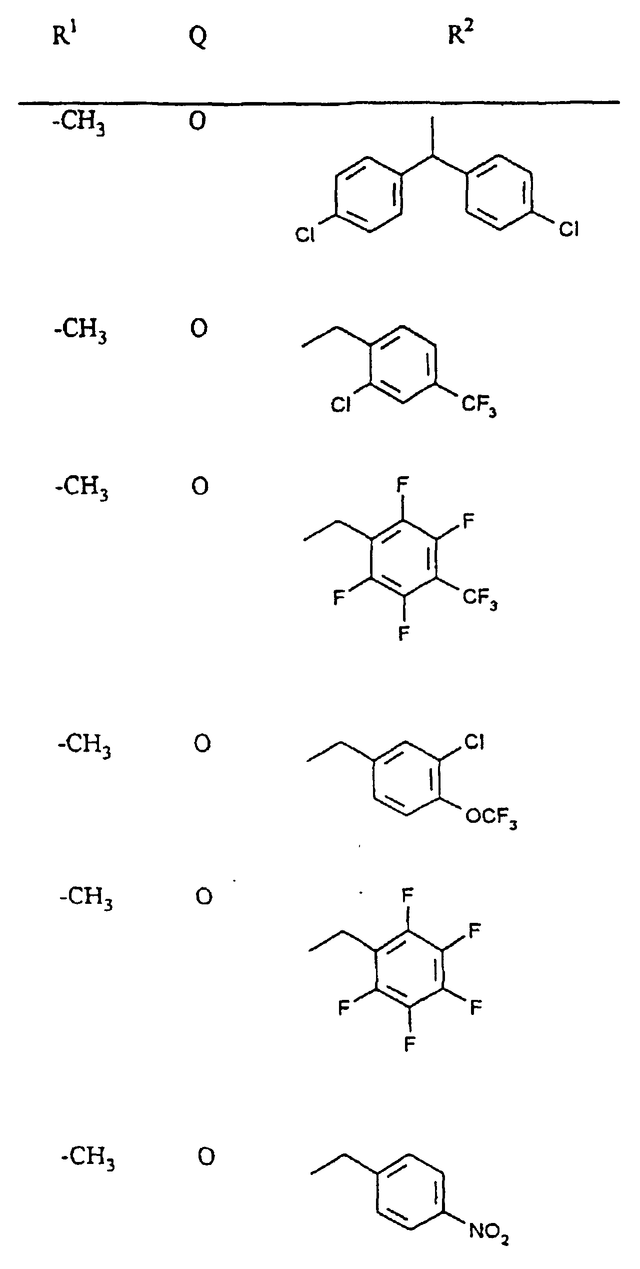 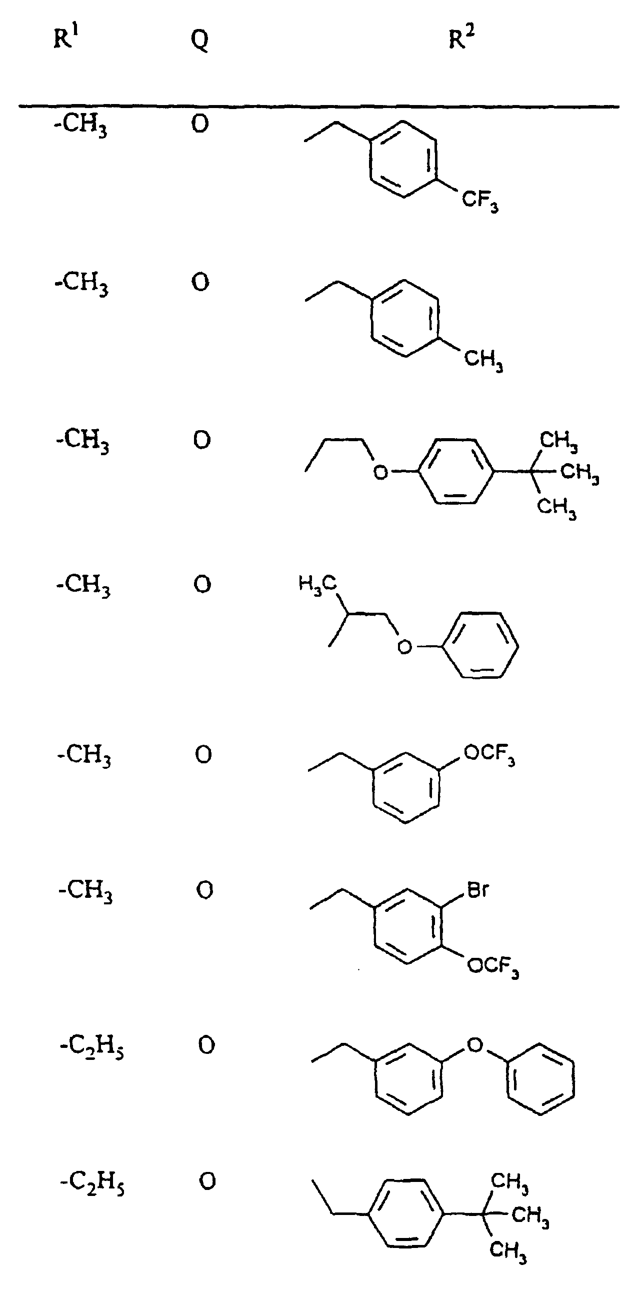 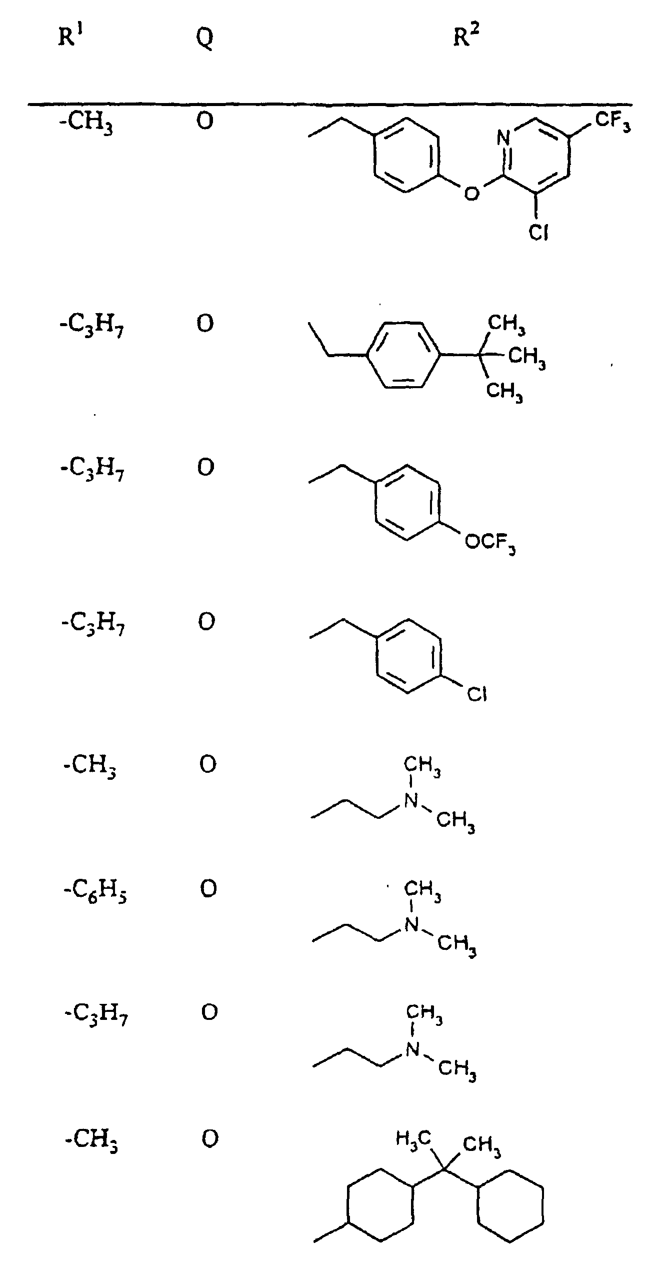 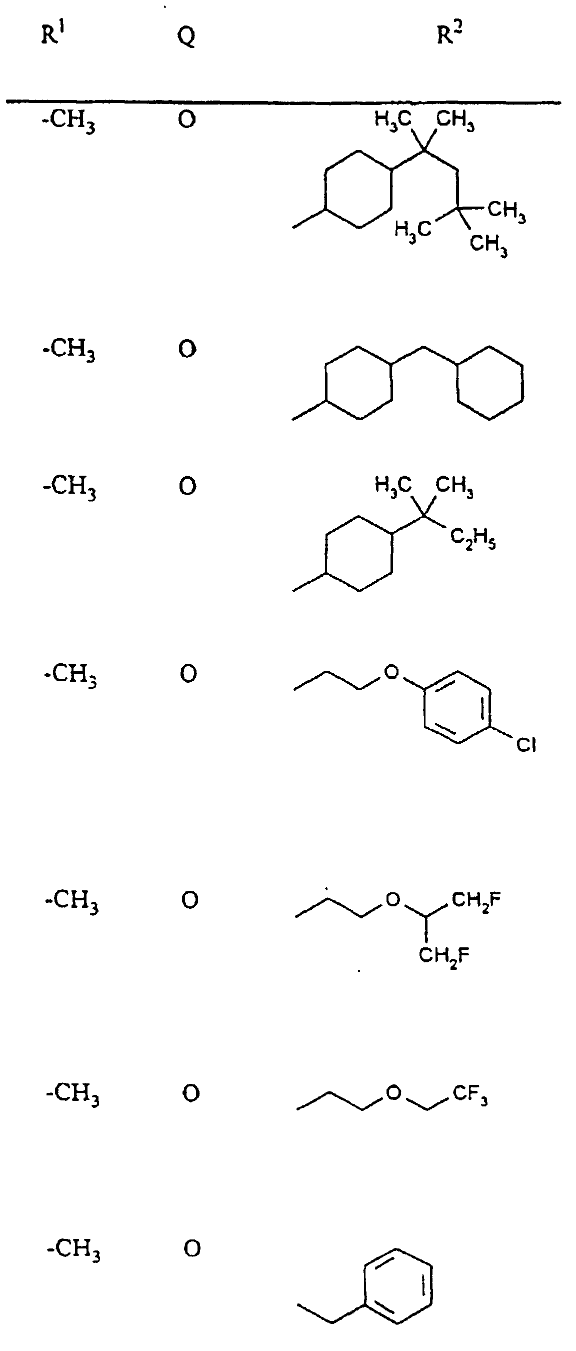 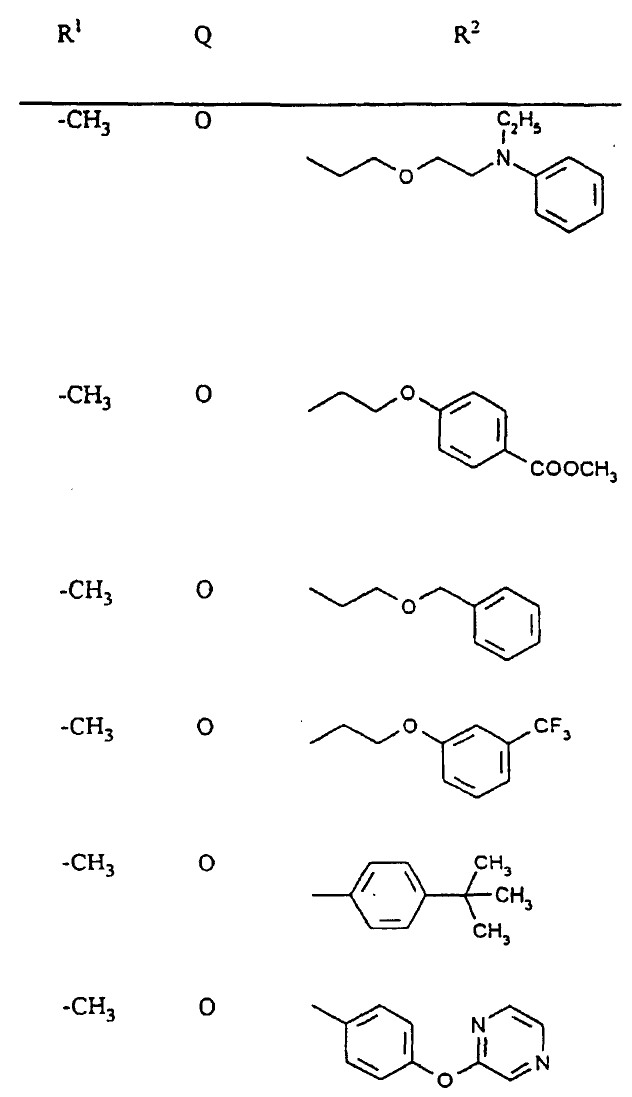 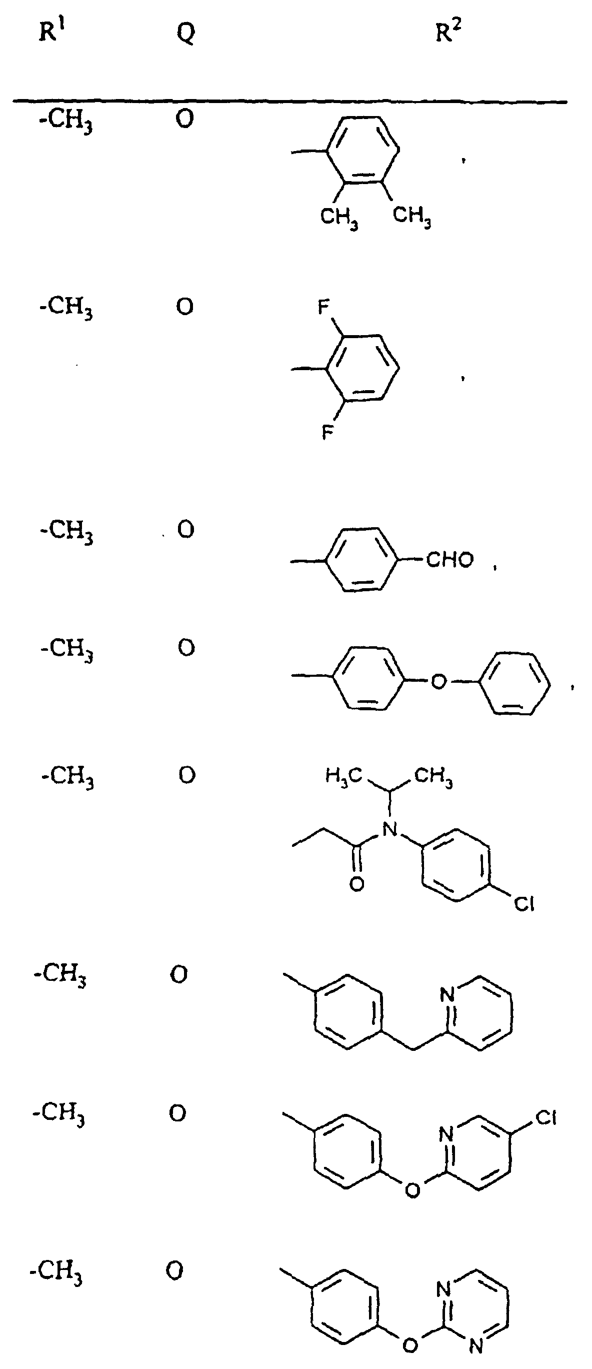 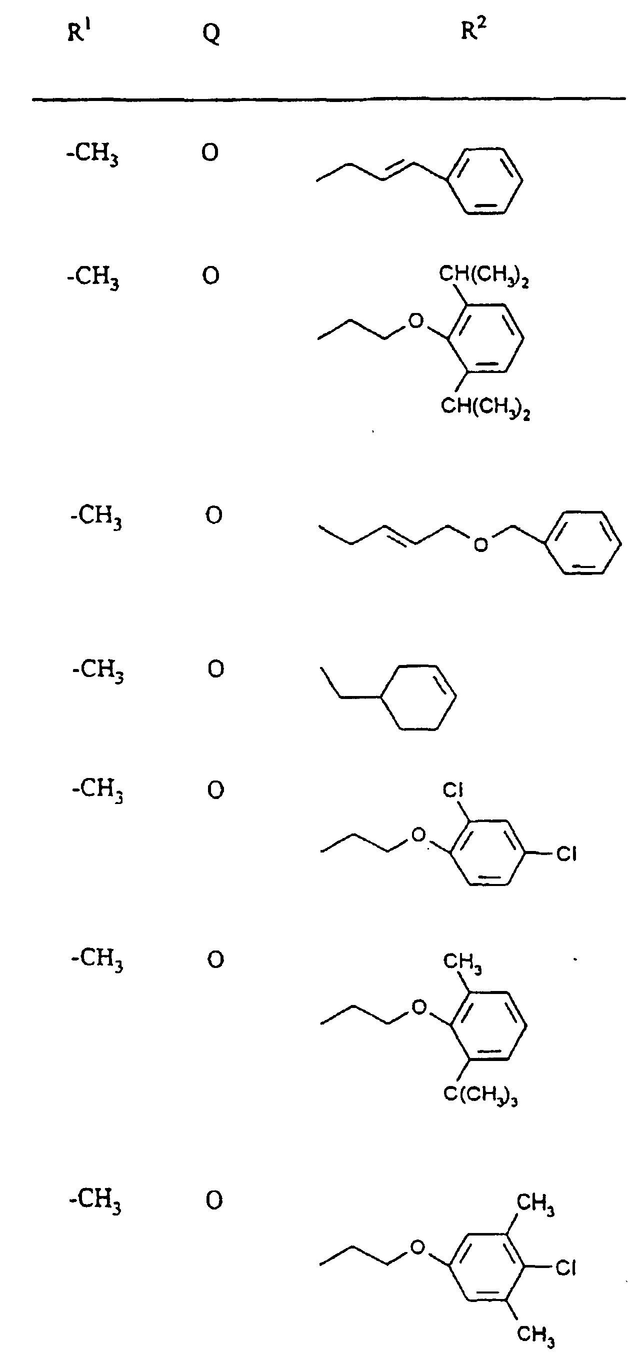 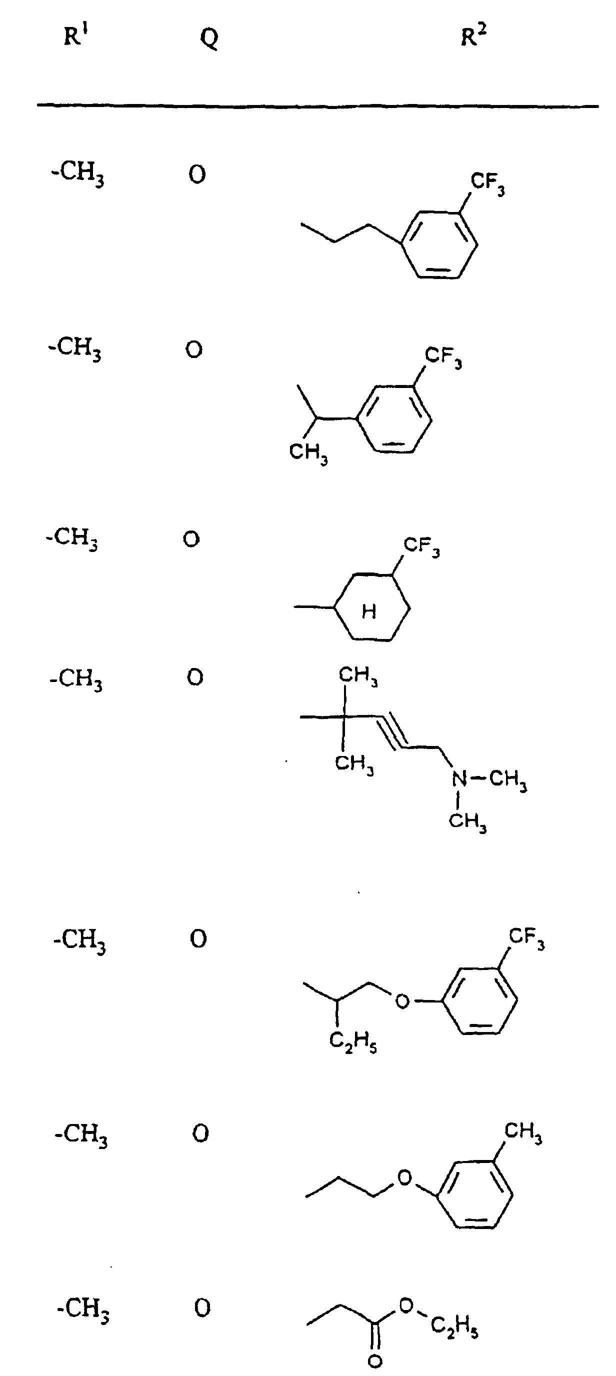 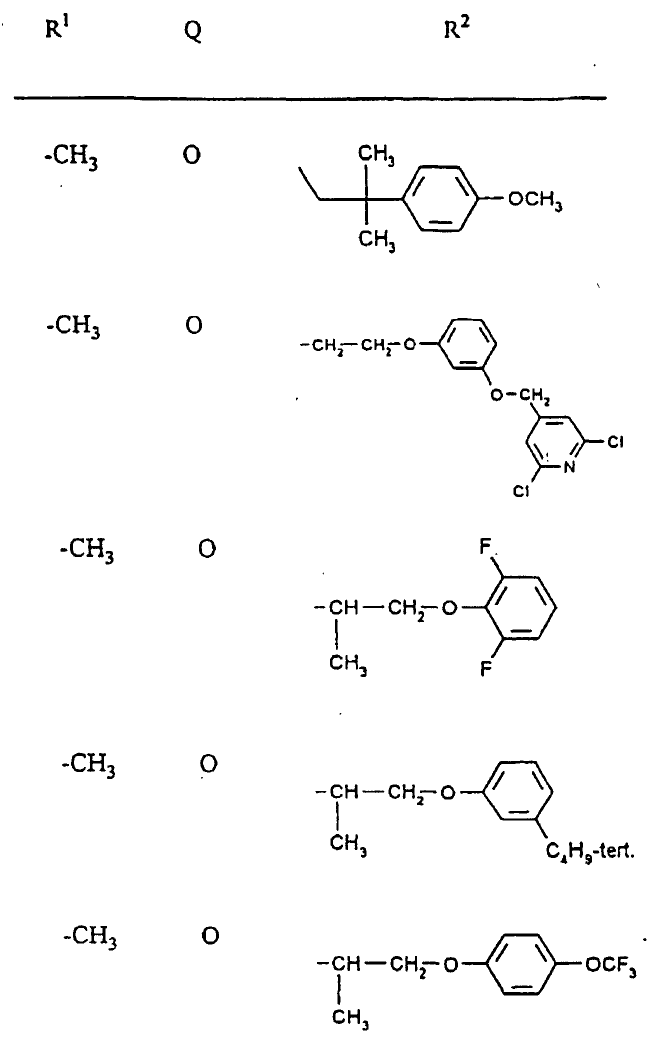 for protecting plants against attack by unwanted micro-organisms and animal pests.

2. Method for protecting plants against attack by unwanted micro-organisms and animal pests, **characterized in that** 1,2,3-thiadiazolecarboxylic (thio)esters of the formula (I) according to Claim 1 are applied to the micro-organisms or to the animal pests and/or to their habitat.

3. 1,2,3-thiadiazolecarboxylic (thio)esters of the formula (I) according to Claim 1.

4. Process for preparing 1,2,3-thiadiazolecarboxylic (thio)esters of the formula (I) according to Claim 1, **characterized in that** 1,2,3-thiadiazolecarboxylic acid derivatives of the formula 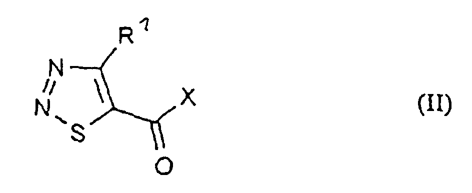 in which
R¹ is as defined above and
X represents halogen
are reacted with (thio)alcohols of the formula
H-Q-R²
in which
Q and R² are each as defined above,
if appropriate in the presence of an acid binder and
if appropriate in the presence of a diluent.

5. Compositions for protecting plants against attack by unwanted micro-organisms and animal pests, **characterized in that** they comprise at least one 1,2,4-thiadiazolecarboxylic (thio)ester of the formula (I) according to Claim 1.

6. Process for preparing compositions for protecting plants against attack by unwanted micro-organisms and animal pests, **characterized in that** 1,2,3-thiadiazolecarboxylic (thio)esters of the formula (I) according to Claim 1 are mixed with extenders and/or surfactants.

## Revendications

1. Utilisation de (thio)esters 1,2,3-thiadiazole-carboxyliques répondant à la formule 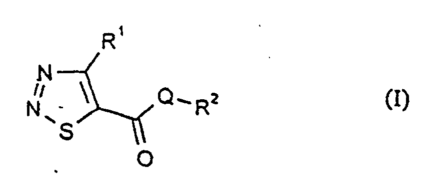 dans laquelle les substituants ont les significations indiquées ci-après 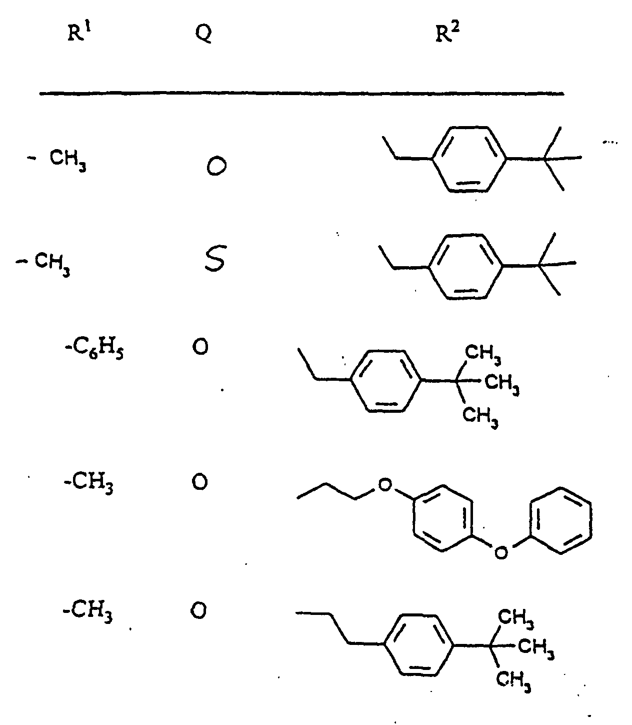 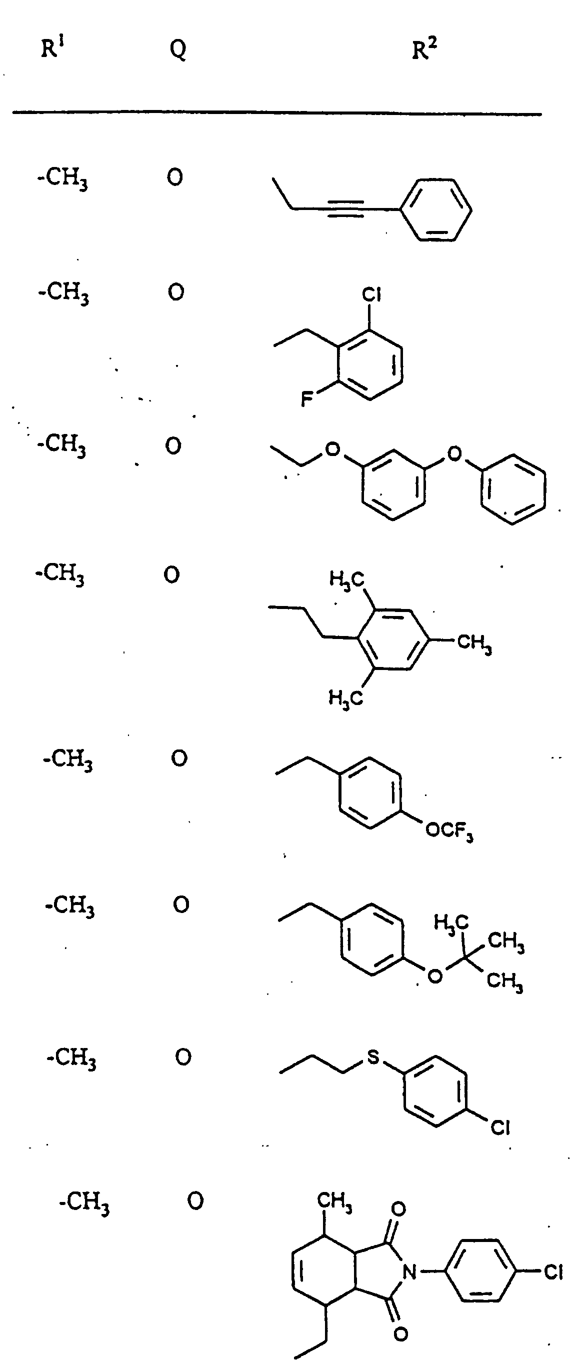 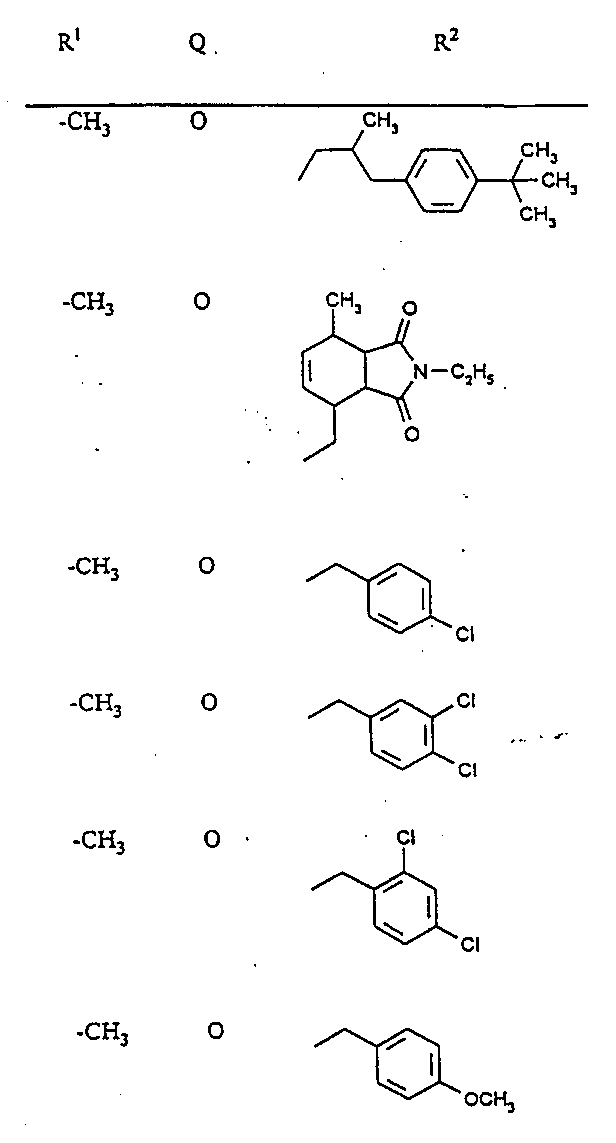 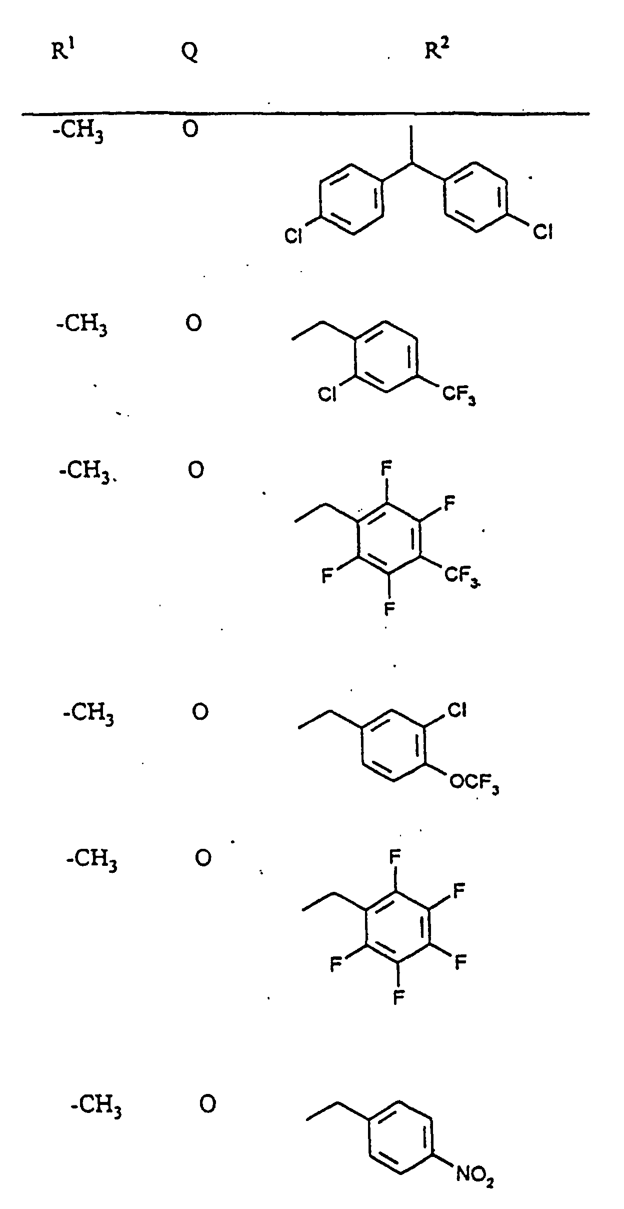 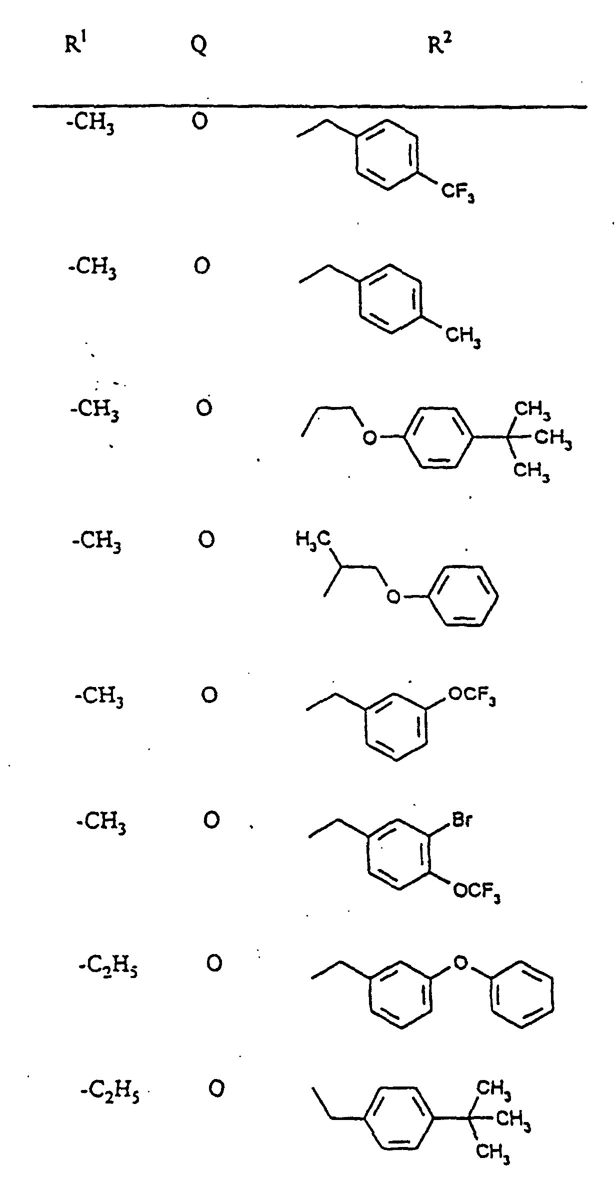 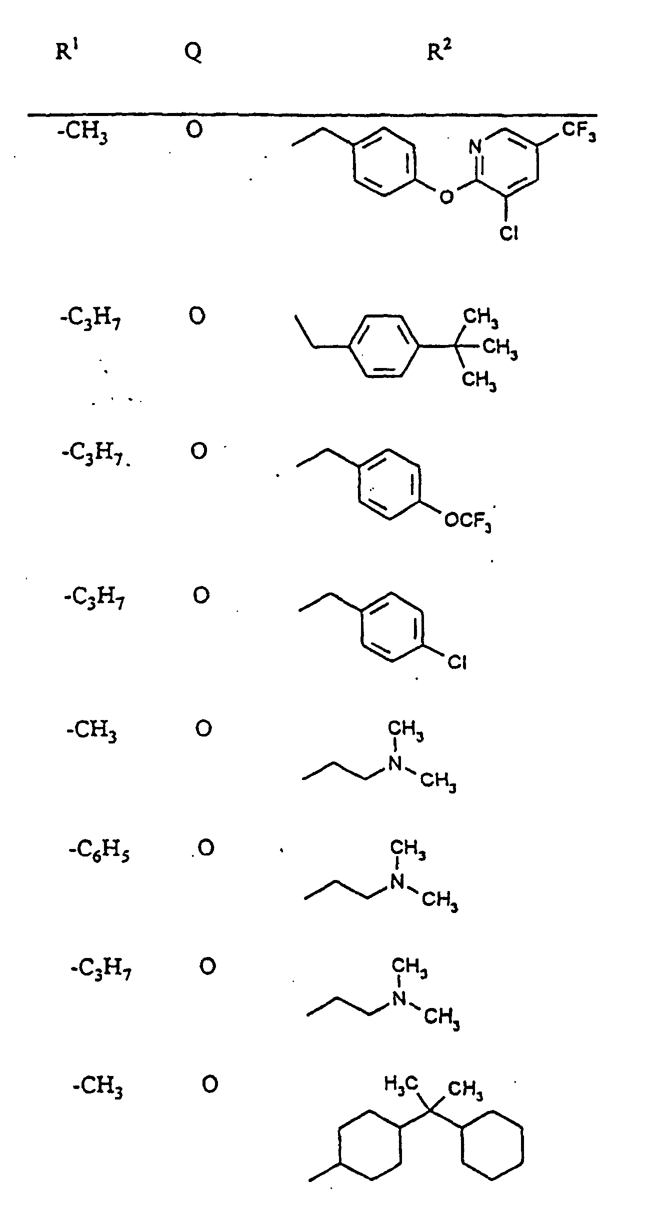 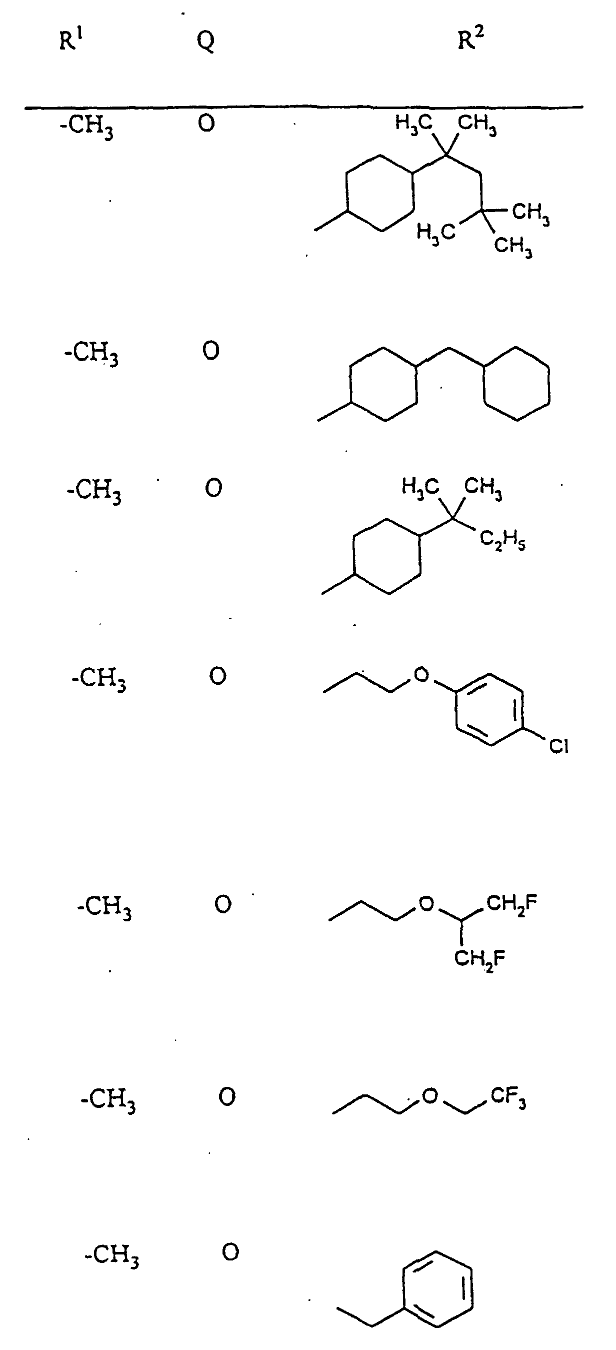 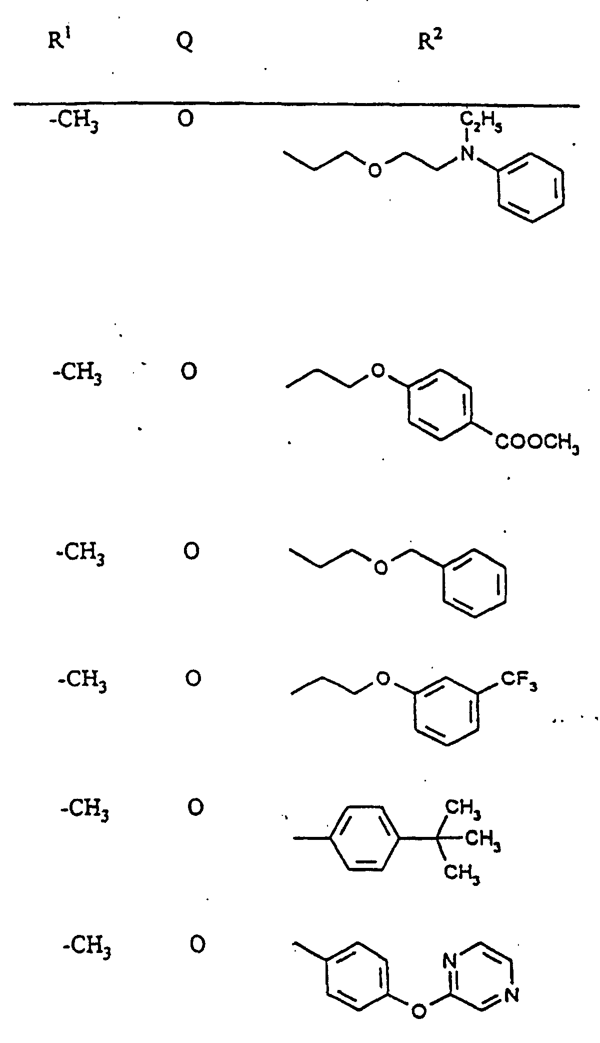 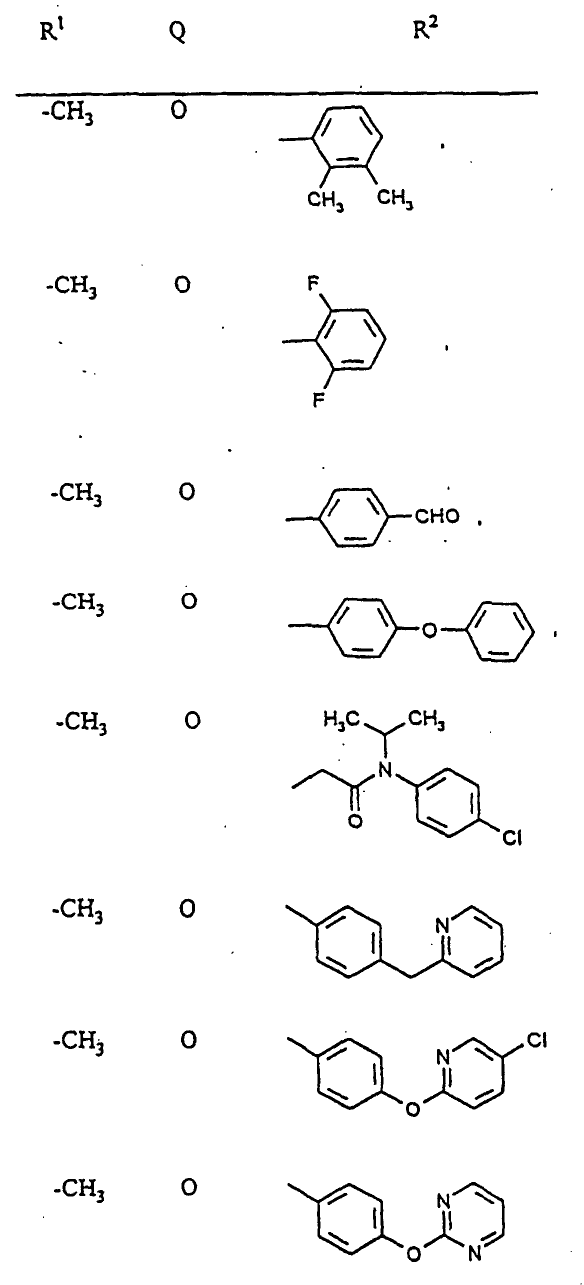 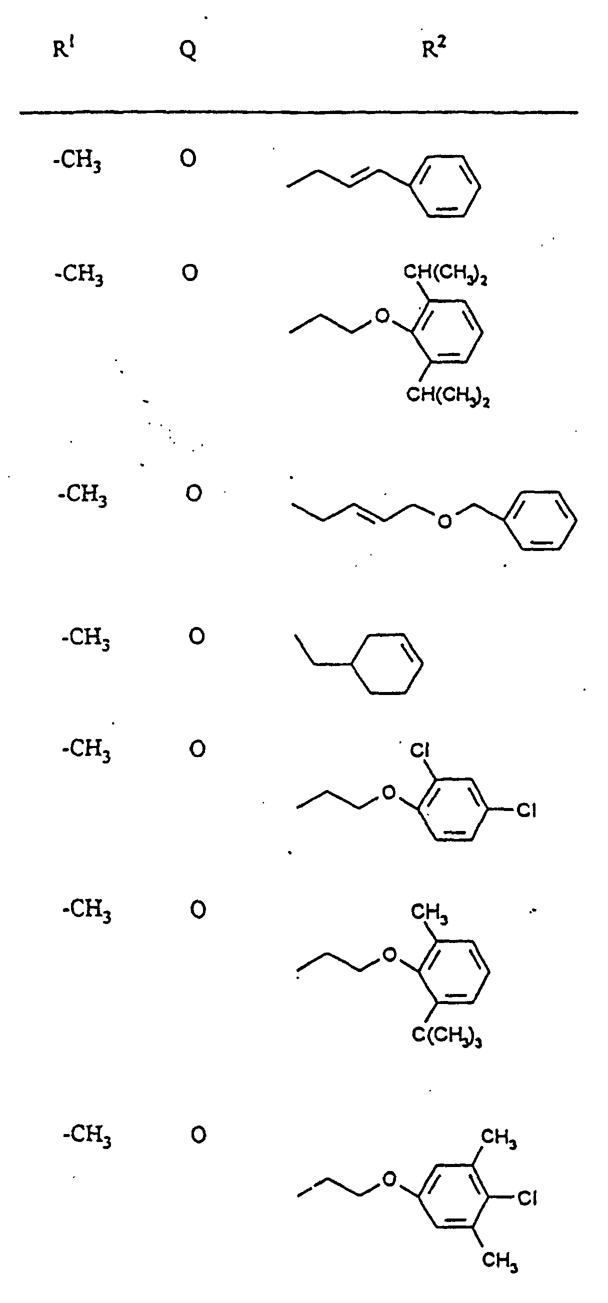 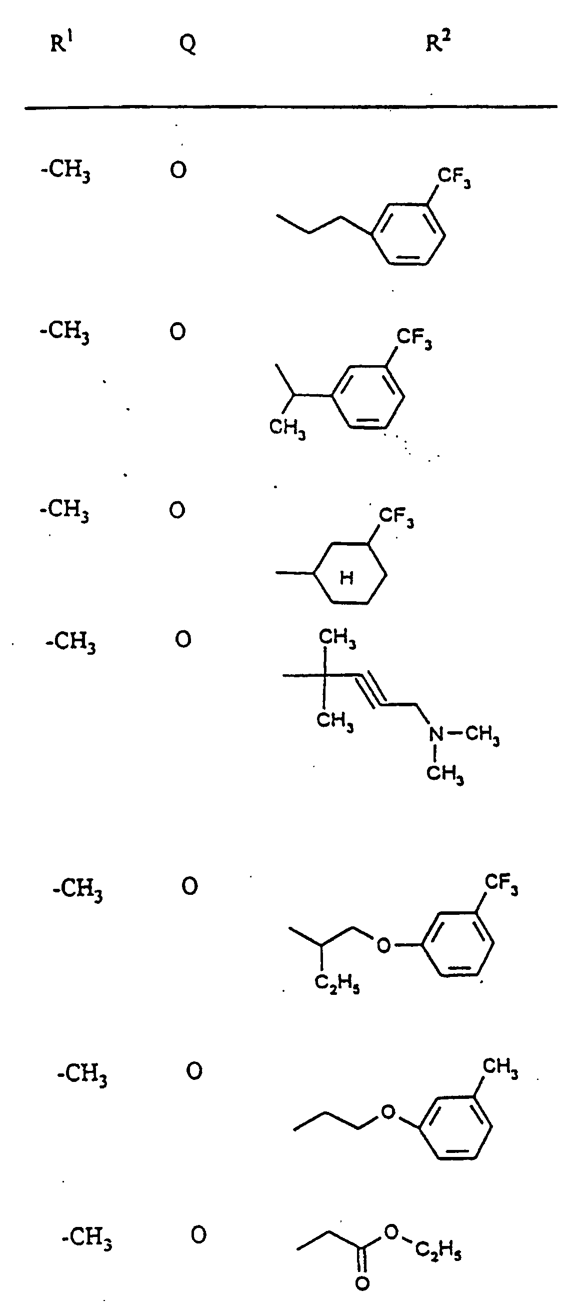 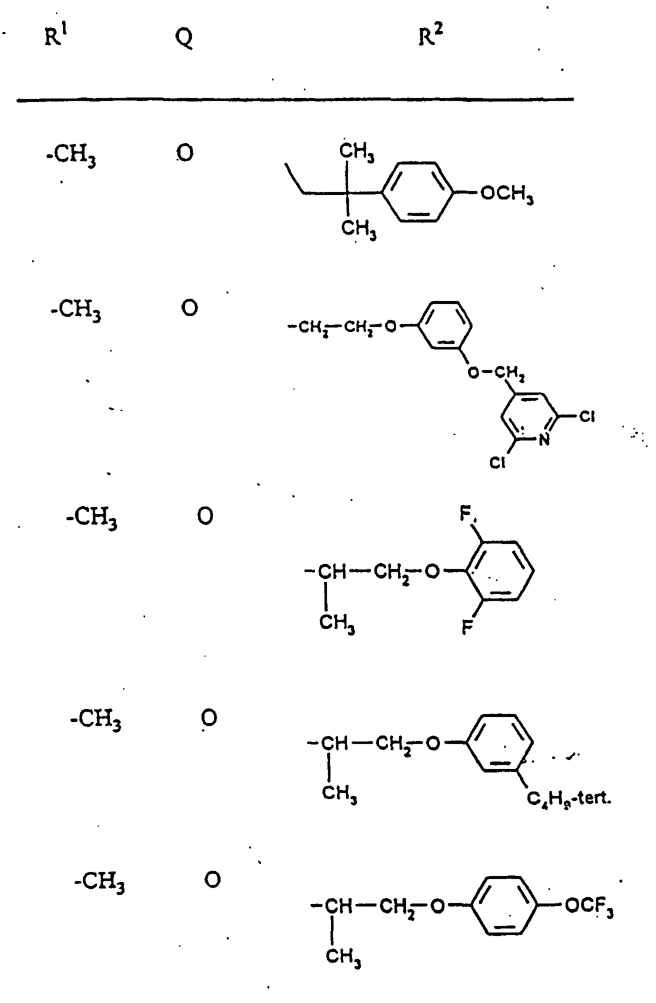 pour la protection de plantes contre l'attaque par des micro-organismes et par des parasites animaux non désirés.

2. Procédé pour la protection de plantes contre l'attaque par des micro-organismes et par des parasites animaux non désirés, **caractérisé en ce qu'**on applique des (thio)esters 1,2,3-thiadiazole-carboxyliques répondant à la formule (I) sur les micro-organismes ou sur les parasites animaux et/ou sur leur biotope.

3. (Thio)esters 1,2,3-thiadiazole-carboxyliques répondant à la formule (I) selon la revendication 1.

4. Procédé pour la préparation de (thio)esters 1,2,3-thiadiazole-carboxyliques répondant à la formule (I) selon la revendication 1, **caractérisé en ce qu'**on fait réagir des dérivés de l'acide 1,2,3-thiadiazole-carboxylique répondant à la formule 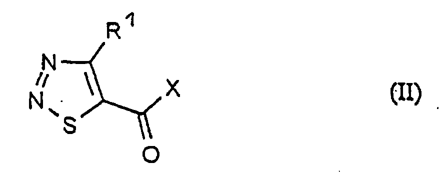 dans laquelle
R¹ a la signification indiquée ci-dessus, et
X représente un atome d'halogène
avec des (thio)alcools répondant à la formule
H-Q-R²
dans laquelle
Q et R² ont les significations indiquées ci-dessus
le cas échéant en présence d'un agent neutralisant les acides et le cas échéant en présence d'un diluant.

5. Agent pour la protection des plantes contre l'attaque par des micro-organismes et par des parasites animaux non désirés, **caractérisé par** une teneur en au moins un (thio)ester 1,2,3-thiadiazole-carboxylique répondant à la formule (I) selon la revendication 1.

6. Procédé pour la préparation d'agents pour la protection des plantes contre l'attaque par des micro-organismes et par des parasites animaux non désirés, **caractérisé en ce qu'**on mélange des (thio)esters 1,2,3-thiadiazole-carboxyliques répondant à la formule (I) selon la revendication 1, avec des diluants et/ou des substances tensioactives.
